(19) Europäisches Patentamt
European Patent Office
Office européen des brevets

(11) EP 3 790 875 B1

# (12) EUROPEAN PATENT SPECIFICATION

(45) Date of publication and mention of the grant of the patent:
**03.07.2024 Bulletin 2024/27**

(21) Application number: **19722614.5**

(22) Date of filing: **07.05.2019**

(51) International Patent Classification (IPC):
***C07D 409/14*** (2006.01) ***C07D 409/04*** (2006.01)
***H10K 50/10*** (2023.01) ***H10K 85/60*** (2023.01)

(52) Cooperative Patent Classification (CPC):
**C07D 409/04; C07D 409/14; H10K 85/615; H10K 85/654; H10K 85/6576;** H10K 50/166; Y02E 10/549

(86) International application number:
**PCT/EP2019/061722**

(87) International publication number:
**WO 2019/215179 (14.11.2019 Gazette 2019/46)**

## (54) N-HETEROARYLENE COMPOUNDS WITH LOW LUMO ENERGIES

N-HETEROARYLEN-VERBINDUNGEN MIT NIEDRIGEN LUMO-ENERGIEN

COMPOSÉS N-HÉTÉROARYLÈNE AYANT DE FAIBLES ÉNERGIES LUMO

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.05.2018 EP 18171304**

(43) Date of publication of application:
**17.03.2021 Bulletin 2021/11**

(73) Proprietor: **Novaled GmbH**
**01099 Dresden (DE)**

(72) Inventors:
- **SCHULZE, Benjamin**
  **01099 Dresden (DE)**
- **WERNER, Ansgar**
  **01099 Dresden (DE)**

(74) Representative: **Michalski Hüttermann & Partner Patentanwälte mbB**
**Kaistraße 16A**
**40221 Düsseldorf (DE)**

(56) References cited:
**EP-A1- 1 962 354**
**WO-A1-2014/208755**
**WO-A1-2017/135510**
**KR-A- 20170 116 944**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

The present invention relates to N-heteroarylene compounds with low LUMO energies, in particular to N-heteroarylene compounds with benzofuranyl or benzothiophenly group, suitable for use as a layer material for electronic devices, and it relates to an organic semiconductor layer comprising at least one compound thereof, as well as to an organic electronic device comprising at least one organic semiconductor layer, and a method of manufacturing the same.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

WO 2014/208755 A1 refers to a cyclic azine compound which is used for organic electroluminescent elements that are capable of achieving improved luminous efficiency and longer service life at the same time.

EP 1 962 354 A1 refers to an organic electroluminescence element wherein an organic thin film layer composed of one or a plurality of layers having at least a light emitting layer is sandwiched between a cathode and an anode.

WO 2017/135510 A1 refers to a compound for an organic optoelectronic device, an organic optoelectronic device employing the same and a display apparatus.

KR 2017 0116944 A refers to a compound for an organic electronic device and an organic electronic device employing.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic material being capable of increasing electron mobility and simultaneously increasing electrochemical stability is needed so that the organic electronic device, such as an organic light emitting diode, may be applied to a large-size flat panel display.

Further, development of an organic material being capable to have an extended life span at higher current density and thereby at higher brightness is needed.

There remains a need to improve performance of organic semiconductor layers, organic semiconductor materials, as well as organic electronic devices thereof, in particular to achieve increased lifetime at higher current density and have a higher efficiency through improving the characteristics of the compounds comprised therein.

There is a need for alternative organic semiconductor materials and organic semiconductor layers as well as organic electronic devices having increased lifetime at higher current density, and/or improved efficiency at low operating voltage.

In particular there is a need for alternative compounds having increased lifetime at higher current density as well as improved efficiency, and at the same time keeping the operating voltage and thereby the power consumption low to deliver long battery life for example mobile electronic devices.

In addition there is a need for alternative compounds having low LUMO energies, and high glass transition temperature ($T_g$) at relatively low evaporation temperatures ($T_{ev}$).

### DISCLOSURE

An aspect of the present invention provides a compound of formula 1:

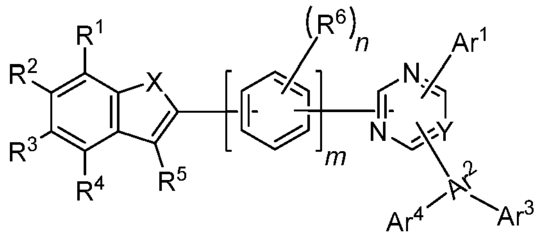 (1),

wherein

alkyl group — is defined as a linear, cyclic or branched alkyl group;

X — is O or S;

$R^1$, $R^2$, $R^3$, $R^4$, $R^5$, $R^6$ — are independently selected from H, substituted or unsubstituted $C_6$ to $C_{38}$ aryl, substituted or unsubstituted $C_3$ to $C_{36}$ heteroaryl or $C_1$ to $C_{16}$ alkyl group, wherein the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl,partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

m — is an integer selected from 0 or 1;

n — is an integer selected from 0;

Y — is CR" or N, wherein R" is H, substituted or unsubstituted $C_6$ to $C_{38}$ aryl, substituted or unsubstituted $C_3$ to $C_{36}$ heteroaryl, or $C_1$ to $C_{16}$ alkyl group, wherein the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

$Ar^1$ — is selected from substituted or unsubstituted $C_6$ to $C_{38}$ aryl, or substituted or unsubstituted $C_3$ to $C_{36}$ heteroaryl, wherein the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

$Ar^2$ — is selected from substituted or unsubstituted $C_6$ to $C_{38}$ arylene or formula 2,

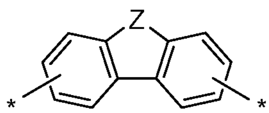 (2) ~~(1)~~,

wherein the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

Z — is selected from O, S, Se, CR''' or NR''' with R''' being selected from $C_1$ to $C_{16}$ alkyl or $C_6$ to $C_{18}$ aryl;

$Ar^3$ — is selected from H, substituted or unsubstituted aryl, substituted or unsubstituted heteroaryl selected from a group comprising of pyridine, pyrimidine, triazine, quinoline, quinoxaline, benzo acridine, dibenzo acridine, phenanthroline, carbazole, dibenzofurane, dibenzothiophene or

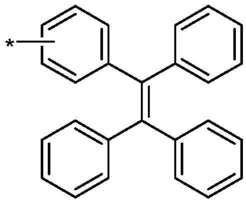,

wherein the substituents are selected from $C_6$ to $C_{18}$ aryl, $C_3$ to $C_{25}$ heteroaryl,-PO(R')$_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

$Ar^4$ is selected from H, substituted or unsubstituted aryl, substituted or unsubstituted heteroaryl selected from a group comprising of pyridine, pyrimidine, triazine, quinoline, quinoxaline, benzo acridine, dibenzo acridine, phenanthroline, carbazole, dibenzofurane, dibenzothiophene, wherein the substituents are selected from $C_6$ to $C_{18}$ aryl, $C_3$ to $C_{25}$ heteroaryl,-PO(R')$_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

and wherein $Ar^4$ is H when $Ar^2$ is selected formula 2.

If not otherwise stated H can represent hydrogen or deuterium.

If not otherwise stated substituents may be selected from $C_1$ to $C_{12}$ alkyl, $C_1$ to $C_{12}$ alkoxy, $C_6$ to $C_{18}$ aryl, $C_3$ to $C_{25}$ heteroaryl, -PO(R')$_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy.

According to one embodiment of the compound of formula 1, wherein R' may be independently selected from alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy.

According to one embodiment of the compound of formula 1, wherein R" may be independently selected from H, substituted or unsubstituted $C_6$ to $C_{38}$ aryl, substituted or unsubstituted $C_3$ to $C_{36}$ heteroaryl, or $C_1$ to $C_{16}$ alkyl group

According to one embodiment of the compound of formula 1, wherein R‴ may be independently selected from $C_1$ to $C_{16}$ alkyl or $C_6$ to $C_{18}$ aryl.

According to one embodiment of the compound of formula 1, wherein the compound of formula 1 may comprises at least one 6 member heteroaryl ring comprises 2 N-atoms to 3 N-atoms, preferably 3 N-atoms.

According to one embodiment of the compound of formula 1, wherein the compound of formula 1 may comprises at least two 6 member heteroaryl rings comprises 2 N-atoms to 3 N-atoms, preferably 3 N-atoms.

According to one embodiment of the compound of formula 1, wherein the compound of formula 1 may comprises at least three 6 member heteroaryl rings comprises 2 N-atoms to 3 N-atoms, preferably 3 N-atoms.

According to one embodiment of the compound of formula 1, wherein the compound of formula 1 may comprises at least four 6 member heteroaryl rings comprises 2 N-atoms to 3 N-atoms, preferably 3 N-atoms.

According to one embodiment of the compound of formula 1, wherein the compound of formula 1 may comprises at least one 5 member heteroaryl ring comprises O as the hetero atom.

According to one embodiment of the compound of formula 1, wherein the compound of formula 1 may comprises at least one 5 member heteroaryl ring comprises S as the hetero atom.

According to one embodiment of the compound of formula 1, wherein the compound of formula 1 may comprises at least one 5 member heteroaryl ring comprises Se as the hetero atom.

According to one embodiment of the compound of formula 1, wherein the compound of formula 1 may comprises at least two 5 member heteroaryl rings comprises O as the hetero atom.

According to one embodiment of the compound of formula 1, wherein the compound of formula 1 may comprises at least two 5 member heteroaryl rings comprises S as the hetero atom.

According to one embodiment of the compound of formula 1, wherein the compound of formula 1 may comprises at least two 5 member heteroaryl rings comprises Se as the hetero atom.

According to one embodiment of the compound of formula 1, wherein the compound of formula 1 may comprises at least 2 to 12 of 6 member non-hetero aryl/ene rings, preferably at least 3 to 10 of 6 member non-hetero aryl/ene rings, further preferred 4 to 8 of 6 member non-hetero aryl/ene rings and in addition preferred 5 to 6 of 6 member non-hetero aryl rings.

According to one embodiment of the compound of formula 1:

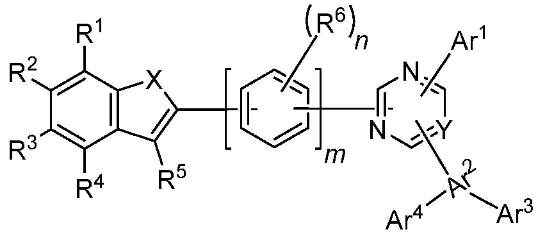 (1),

wherein

| | |
|---|---|
| X | is O; |
| $R^1$, $R^2$, $R^3$, $R^4$, $R^5$, $R^6$ | are independently selected from H, substituted or unsubstituted $C_6$ to $C_{38}$ aryl, substituted or unsubstituted $C_3$ to $C_{36}$ heteroaryl or $C_1$ to $C_{16}$ alkyl group, wherein the substituents may be selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy; |
| m | is an integer selected from 0 or 1; |
| n | is an integer selected from 0; |
| Y | is N or CH; |
| $Ar^1$ | may be selected from substituted or unsubstituted $C_6$ to $C_{38}$ aryl, or substituted or unsubstituted $C_3$ to $C_{36}$ heteroaryl, wherein the substituents may be selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy; |
| $Ar^2$ | may be selected from substituted or unsubstituted $C_6$ to $C_{38}$ arylene or formula 2, |

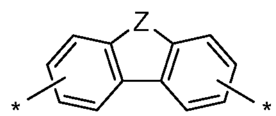 (2),

wherein the substituents may be selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

| | |
|---|---|
| Z | may be selected from O, S, Se, CR''' or NR''' with R''' being selected from $C_1$ to $C_{16}$ alkyl or $C_6$ to $C_{18}$ aryl; |
| Ar' | may be selected from H, substituted or unsubstituted aryl, substituted or unsubstituted heteroaryl selected from a group comprising of pyridine, pyrimidine, triazine, quinoline, quinoxaline, benzo acridine, dibenzo acridine, phenanthroline, carbazole, dibenzofurane, dibenzothiophene, wherein the substituents may be selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy; |
| $Ar^4$ | may be selected from H, substituted or unsubstituted aryl, substituted or unsubstituted heteroaryl selected from a group comprising of pyridine, pyrimidine, triazine, quinoline, quinoxaline, |

benzo acridine, dibenzo acridine, phenanthroline, carbazole, dibenzofurane, dibenzothiophene, wherein the substituents may be selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

and wherein $Ar^4$ is H when $Ar^2$ may be selected formula 2.

According to one embodiment of the compound of formula 1:

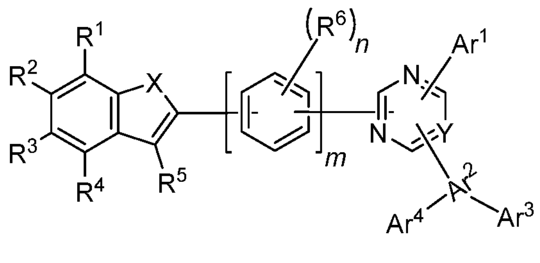
(1),

wherein
X is O; and wherein $Ar^4$ is H when $Ar^2$ may be selected formula 2.

According to one embodiment of the compound of formula 1:

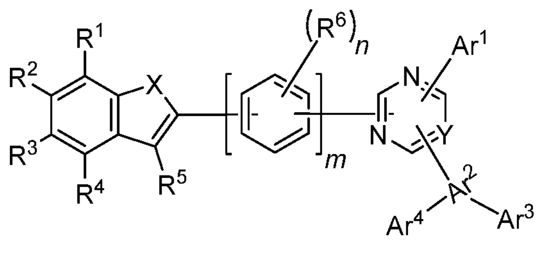
(1),

wherein
X is S; and wherein $Ar^4$ is H when $Ar^2$ may be selected formula 2.

According to one embodiment of the compound of formula 1:

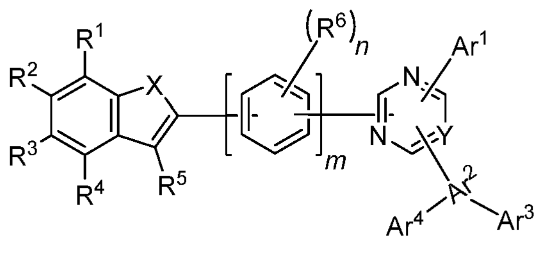
(1),

wherein

$R^1$, $R^2$, $R^3$, $R^4$, $R^5$, $R^6$ are independently selected from H, substituted or unsubstituted $C_6$ to $C_{12}$ aryl, substituted or unsubstituted $C_3$ to $C_{11}$ heteroaryl or $C_1$ to $C_3$ alkyl group, wherein the substituents may be selected from $C_6$ to $C_{12}$ aryl, $C_3$ to $C_{11}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy.

According to one embodiment of the compound of formula 1:

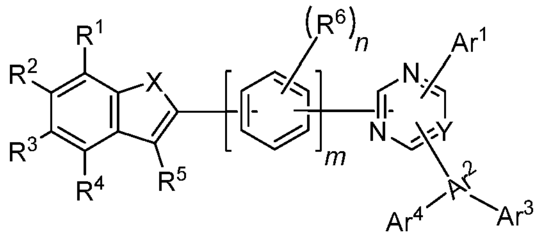

(1),

wherein

$R^1$, $R^2$, $R^3$, $R^4$, $R^5$, $R^6$ are independently selected from H, unsubstituted $C_6$ to $C_{12}$ aryl, unsubstituted $C_3$ to $C_{11}$ heteroaryl or $C_1$ to $C_3$ alkyl group.

According to one embodiment of the compound of formula 1:

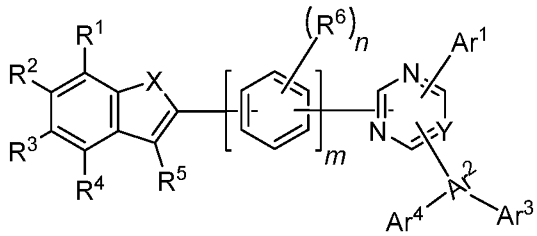

(1),

wherein

$R^1$, $R^2$, $R^3$, $R^4$, $R^5$, $R^6$ are independently selected from Hand unsubstituted $C_6$ aryl, preferably H.

According to one embodiment of the compound of formula 1, wherein m is 0.

According to one embodiment of the compound of formula 1, wherein the compound of formula 1 is represented by formula 1a and/or 1b:

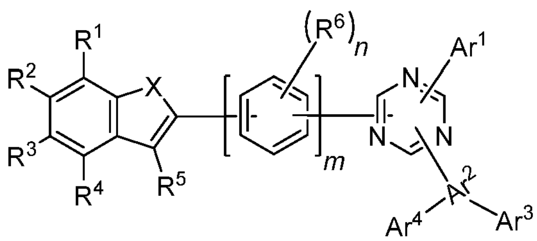

(1a);

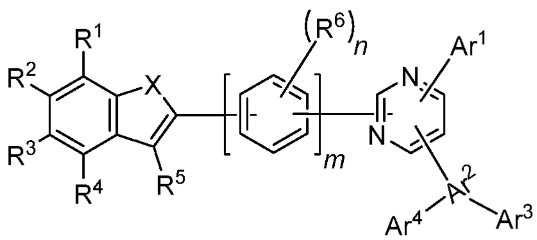

(1b).

According to one embodiment of the compound of formula 1, wherein the compound of formula 1 is represented by formula 1a:

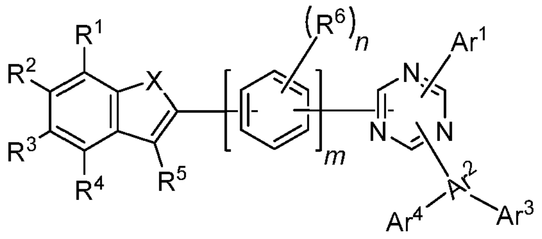

(1a).

According to one embodiment of the compound of formula 1, wherein the compound of formula 1 is represented by formula 1b:

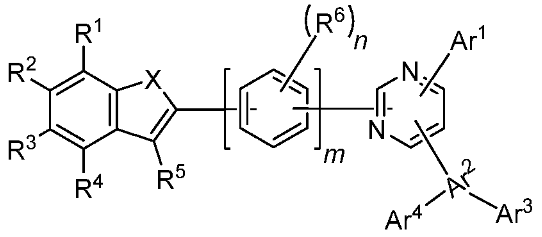

(1b).

According to one embodiment of the compound of formula 1, wherein

| | |
|---|---|
| X | is O or S; |
| $R^1$, $R^2$, $R^3$, $R^4$, $R^5$, $R^6$ | are independently selected from H, phenyl, naphthyl, or biphenyl; |
| m | is 0; |
| n | is 0; |
| Y | is N or CH, preferably N; |
| $Ar^1$ | may be selected from phenyl, naphthyl, biphenyl, phenanthryl triphenyl, or anthryl group; |
| $Ar^2$ | may be selected from divalent phenylene, naphthylene, biphenylene, phenanthrylene, triphenylene, or anthrylene group; |
| Z | may be selected from O, S, Se, CR‴ or NR‴ with R‴ being selected from $C_1$ to $C_{16}$ alkyl or $C_6$ to $C_{18}$ aryl; |
| Ar’ | may be selected from H, substituted or unsubstituted aryl, substituted or unsubstituted heteroaryl selected from a group comprising of pyridine, pyrimidine, triazine, quinoline, quinoxaline, benzo acridine, dibenzo acridine, phenanthroline, carbazole, dibenzofurane, dibenzothiophene; |
| $Ar^4$ | may be selected from H, substituted or unsubstituted aryl, substituted or unsubstituted heteroaryl selected from a group comprising of pyridine, pyrimidine, triazine, quinoline, quinoxaline, phenanthroline, carbazole, dibenzofurane, dibenzothiophene. |

According to one embodiment of the compound of formula 1, wherein

$Ar^2$ is selected from formula 2; and
Z is selected from O, S, Se, CR‴ or NR‴ with R‴ being selected from $C_1$ to $C_{16}$ alkyl or $C_6$ to $C_{18}$ aryl, and more preferred CR‴ with R‴ being selected from $C_1$ to $C_{16}$ alkyl or $C_6$ to $C_{18}$ aryl. According to one embodiment of the compound of formula 1, wherein
$Ar^2$ is selected from formula 2; and
Z is selected from O, S, Se and CR‴ with R‴ being selected from $C_1$ to $C_{16}$ alkyl or $C_6$ to $C_{18}$ aryl. According to one

embodiment of the compound of formula 1, wherein

| | |
|---|---|
| $Ar^2$ | is selected from formula 2; and |
| Z | is selected from O, S and Se |

According to one embodiment of the compound of formula 1, wherein

| | |
|---|---|
| X | is O or S; |
| $R^1$, $R^2$, $R^3$, $R^4$, $R^5$, $R^6$ | are independently selected from H, phenyl, naphthyl, or biphenyl; |
| m | is 0; |
| n | is 0; |
| Y | is N or CH, preferably N; |
| $Ar^1$ | may be selected from phenyl, naphthyl, biphenyl, phenanthryl triphenyl, or anthryl group; |
| $Ar^2$ | may be selected from divalent phenylene, naphthylene, biphenylene, phenanthrylene, triphenylene, or anthrylene group; |
| Z | may be selected from O, S, Se, CR''' or NR''' with R''' being selected from $C_1$ to $C_{16}$ alkyl or $C_6$ to $C_{18}$ aryl; |
| Ar' | may be selected from H, unsubstituted aryl, unsubstituted heteroaryl selected from a group comprising of pyridine, pyrimidine, triazine, quinoline, quinoxaline, benzo acridine, dibenzo acridine, phenanthroline, carbazole, dibenzofurane, dibenzothiophene; |
| $Ar^4$ | may be selected from H, unsubstituted aryl, unsubstituted heteroaryl selected from a group comprising of pyridine, pyrimidine, triazine, quinoline, quinoxaline, benzo acridine, dibenzo acridine, phenanthroline, carbazole, dibenzofurane, dibenzothiophene. |

According to one embodiment of the compound of formula 1, wherein the compound of formula 1 comprises:

- at least one non-hetero phenyl rings to twelve non-hetero phenyl rings, preferably of at least two phenyl rings to seven phenyl rings; and/or
- at least one to four, preferably one to three, further preferred one or two and more preferred one hetero aromatic 6-member ring, and at least one to two, preferably one hetero aromatic 5-member ring.

According to one embodiment of the compound of formula 1, wherein $Ar^1$ and, $Ar^3$ and/or $Ar^4$ are independently selected from substituted or unsubstituted $C_6$ to $C_{18}$ aryl, or substituted or unsubstituted $C_5$ to $C_{18}$ heteroaryl, wherein the substituents of the substituted $C_6$ to $C_{18}$ aryl or substituted $C_5$ to $C_{18}$ heteroaryl are independently selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl.

According to one embodiment of the compound of formula 1, wherein $Ar^2$ is selected from substituted or unsubstituted $C_6$ to $C_{18}$ arylene, or substituted or unsubstituted $C_5$ to $C_{18}$ heteroarylene, wherein the substituents of the substituted $C_6$ to $C_{18}$ aryl or substituted $C_5$ to $C_{18}$ heteroaryl are independently selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl.

According to another embodiment of the compound of formula 1, wherein $R^1$, $R^2$, $R^3$, $R^4$, $R^5$, $R^6$ are independently selected from:

- substituted or unsubstituted aromatic groups comprising at least three phenyl rings to twenty phenyl rings, or
- substituted or unsubstituted aromatic groups comprising at least two phenyl rings to twenty phenyl rings, and at least one heteroaryl ring to four heteroaryl rings.

According to another embodiment of the compound of formula 1, wherein $R^1$, $R^2$, $R^3$, $R^4$, $R^5$, $R^6$ are independently selected from:

- substituted or unsubstituted aromatic groups comprising at least three phenyl rings to twenty phenyl rings, or
- substituted or unsubstituted aromatic groups comprising at least two phenyl rings to twenty phenyl rings, and at least one heteroaryl ring to four heteroaryl rings, wherein the heteroaryl ring having 2 to 6 carbon atoms, preferably 4 to 5 carbon atoms, and the heteroaryl ring is a 5 or 7 member ring, preferably a 6 member ring.

According to another embodiment of the compound of formula 1, wherein $R^1$, $R^2$, $R^3$, $R^4$, $R^5$, $R^6$ are independently selected from:

- substituted or unsubstituted aromatic groups comprising at least four phenyl rings to seven phenyl rings, or
- substituted or unsubstituted aromatic groups comprising at least four phenyl rings to seven phenyl rings, and at least two heteroaryl rings to three heteroaryl rings, wherein the heteroaryl ring having 2 to 6 carbon atoms, preferably 4 to 5 carbon atoms, and the heteroaryl ring is a 5 or 7 member ring, preferably a 6 member ring.

According to another embodiment of the compound of formula 1, wherein $R^1$, $R^2$, $R^3$, $R^4$, $R^5$, $R^6$ are independently selected from:

- substituted or unsubstituted aromatic groups comprising at least 3 substituted or unsubstituted phenyl rings to 12 substituted or unsubstituted phenyl rings, preferably of at least 4 substituted or unsubstituted phenyl rings to 10 substituted or unsubstituted phenyl rings, further preferred of at least 4 substituted or unsubstituted phenyl rings to 6 substituted or unsubstituted phenyl rings, wherein the substituents of the substituted phenyl rings are independently selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl; or
- substituted or unsubstituted aromatic groups comprising at least 2 substituted or unsubstituted phenyl rings to 12 substituted or unsubstituted phenyl rings, preferably at least 4 substituted or unsubstituted phenyl rings to 7 substituted or unsubstituted phenyl rings; and at least 1 substituted or unsubstituted heteroaryl ring to 4 substituted or unsubstituted heteroaryl rings, preferably at least 2 substituted or unsubstituted heteroaryl rings to 3 substituted or unsubstituted heteroaryl rings, wherein the heteroaryl ring having 2 to 6 carbon atoms, preferably 4 to 5 carbon atoms, and the heteroaryl ring is a 5 or 7 member ring, preferably a 6 member ring, and wherein the substituents of the substituted phenyl and substituted heteroaryl ring are independently selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl.

According to another embodiment of the compound of formula 1, wherein $R^1$, $R^2$, $R^3$, $R^4$, $R^5$, $R^6$ are independently selected from:

- substituted or unsubstituted aromatic groups comprising at least 2 substituted or unsubstituted phenyl rings to 12 substituted or unsubstituted phenyl rings, preferably at least 4 substituted or unsubstituted phenyl rings to 7 substituted or unsubstituted phenyl rings, wherein the substituents of the substituted phenyl are independently selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl.

According to another embodiment of the compound of formula 1, wherein $R^1$, $R^2$, $R^3$, $R^4$, $R^5$, $R^6$ are independently selected from:

- substituted or unsubstituted aromatic groups comprising at least 1 substituted or unsubstituted heteroaryl ring to 4 substituted or unsubstituted heteroaryl rings, preferably at least 2 substituted or unsubstituted heteroaryl rings to 3 substituted or unsubstituted heteroaryl rings, wherein the heteroaryl ring having 2 to 6 carbon atoms, preferably 4 to 5 carbon atoms, and the heteroaryl ring is a 5 or 7 member ring, preferably a 6 member ring, and wherein the substituted of the substituents of the heteroaryl are independently selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl.

According to another embodiment of the compound of formula 1, wherein $R^1$, $R^2$, $R^3$, $R^4$, $R^5$, $R^6$ are independently selected from:

- substituted or unsubstituted aromatic groups comprising at least 2 substituted or unsubstituted phenyl rings to 12 substituted or unsubstituted phenyl rings, preferably at least 4 substituted or unsubstituted phenyl rings to 7 substituted or unsubstituted phenyl rings, wherein the substituents of the substituted phenyl are independently selected from $C_6$ to $C_{18}$ aryl.

According to the present invention, the compound of formula 1 comprises:

- the at least three substituted or unsubstituted phenyl rings that are connected via one or more single bonds, via an ethylene group and/or are part of a fused ring system; or
- the at least two or at least three or at least four substituted or unsubstituted phenyl rings are connected via one or more single bonds, via an ethylene group and/or are part of a fused ring system.

According to the present invention, the compound of formula 1 may comprises a first phenyl ring and/or a second phenyl ring of at least three substituted or unsubstituted phenyl rings that are connected via a single bond to the third phenyl ring.

According to the present invention, the compound of formula 1 may comprises at least two or three of a first phenyl ring, second phenyl ring and third phenyl ring of at least three substituted or unsubstituted phenyl rings that are connected via an ethylene group.

According to the present invention, the compound of formula 1 may comprises at least two or three of a first phenyl ring, second phenyl ring and third phenyl ring of at least three substituted or unsubstituted phenyl rings that are part of a fused ring system.

According to the present invention, the compound of formula 1 may comprises a first phenyl ring and/or a second phenyl ring of at least three substituted or unsubstituted aromatic groups that are connected via a single bond to a third heteroaryl ring.

According to the present invention, the compound of formula 1 may comprises at least two or three of a first phenyl ring, second phenyl ring and third heteroaryl ring of at least three substituted or unsubstituted aromatic groups that are connected via an ethylene group.

According to the present invention, the compound of formula 1 may comprises at least two or three of a first phenyl ring, second phenyl ring and third heteroary ring of at least three substituted or unsubstituted aromatic groups that are part of a fused ring system.

If the at least three phenyl rings, or the at least two phenyl rings and the at least one heteroaryl ring of the aromatic groups, are part of a fused ring system, two or more phenyl rings, or two or more phenyl rings and a heteroaryl ring may share at least two carbon atoms and/or phenyl rings and/or heteroaryl rings may be fused with 5-membered rings.

If the at least three phenyl rings a part of a fused ring system, at least two or three phenyl rings may share at least two carbon atoms.

If the at least two phenyl rings and at least one heteroaryl ring of the aromatic group a part of a fused ring system, at least two rings or three rings may share at least two carbon atoms.

If the at least two phenyl rings and a heteroaryl ring are part of a fused ring system, two or more phenyl rings and/or heteroraryl ring may share at least two carbon atoms and/or may be fused with 5-membered rings.

According to one embodiment the compound of formula 1 may comprises:

- substituted or unsubstituted aromatic groups comprising at least three substituted or unsubstituted phenyl rings, wherein at least three phenyl rings are connected via a single bond or are part of a fused ring system, or
- substituted or unsubstituted aromatic groups comprising at least two substituted or unsubstituted phenyl rings and one substituted or unsubstituted heteroaryl ring, wherein at least two phenyl rings and one heteroaryl ring are connected via a single bond or are part of a fused ring system.

According to another embodiment of the compound of formula 1, wherein $Ar^1$ may be selected from phenyl, biphenyl, terphenyl, naphthyl, phenanthryl, triphenyl, anthracenyl.

According to another embodiment of the compound of formula 1, wherein

- $Ar^1$ may be selected from phenyl, biphenyl, terphenyl, naphthyl, phenanthryl, triphenyl, anthracenyl, and preferably from C1 to C8:

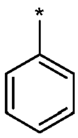 (C1), 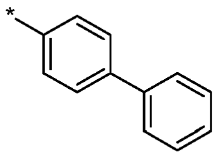 (C2), 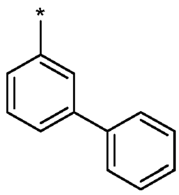 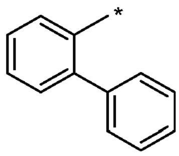 (C3), 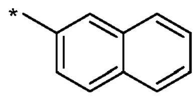 (C4),

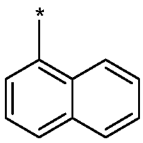 (C5), 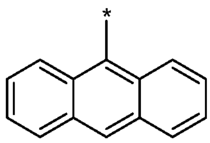 (C6), 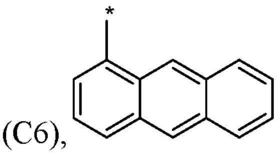 (C7), 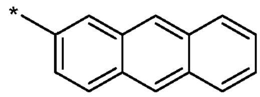 (C8);

and/or

- Ar2 is be selected from phenylene, biphenylene, terphenylene, naphthylene, phenanthrylene, triphenylene, anthracenylene, and preferably from D1 to D13:

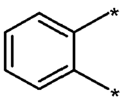 (D1), 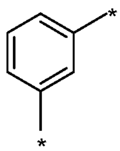 (D2), 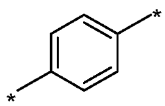 (D3), 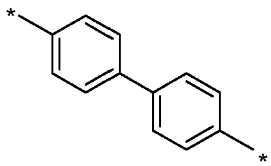 (D4),

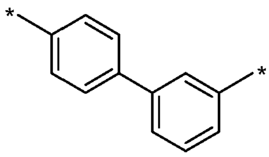 (D5), 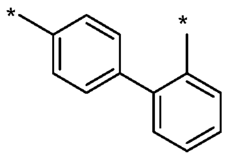 (D6), 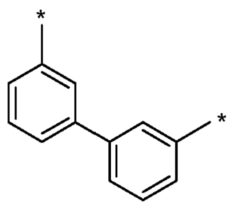 (D7),

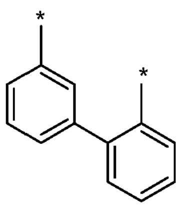 (D8), 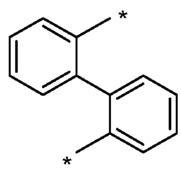 (D9), 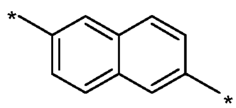 (D10), 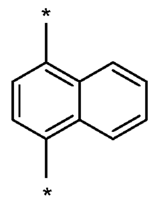

(D11), 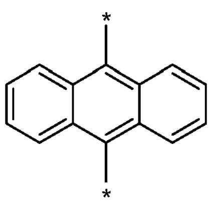 (D12), 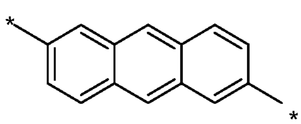 (D13);

- Ar3 may be independently selected from E1 to E28:

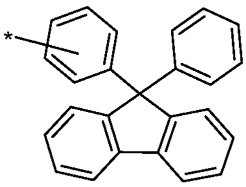 (E1), 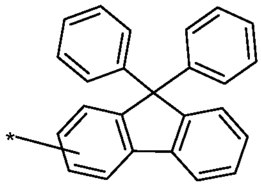 (E2), 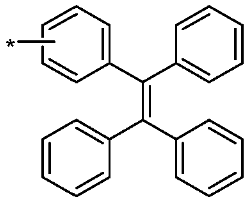 (E3),

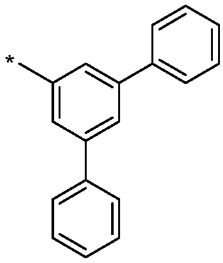 (E4), 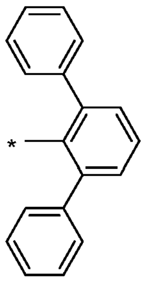 (E5), 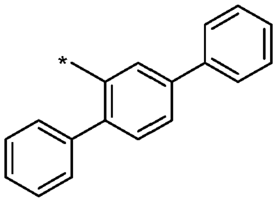 (E6),

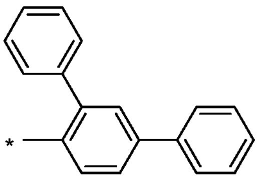 (E7), 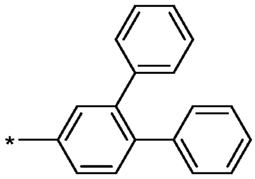 (E8), 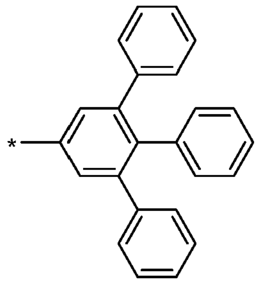 (E9),

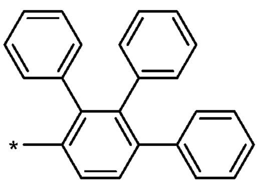 (E10), 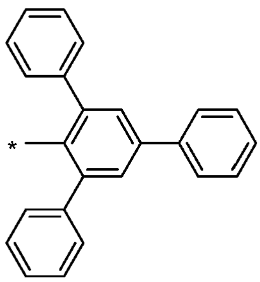 (E11),

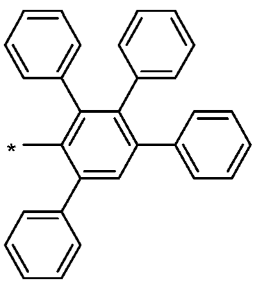 (E12), 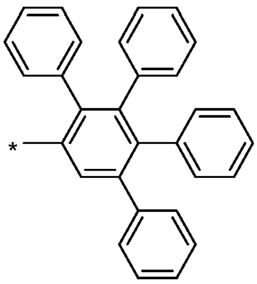 (E13), 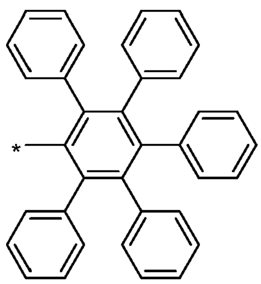 (E14),

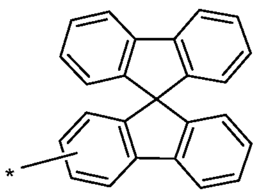 (E15), 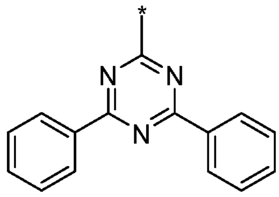 (E16), 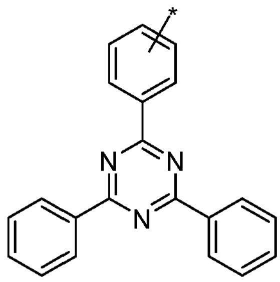 (E17),

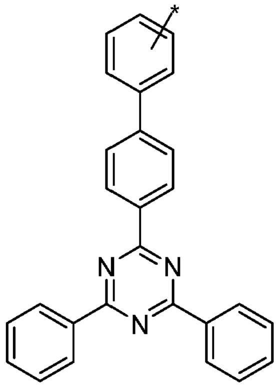 (E18), 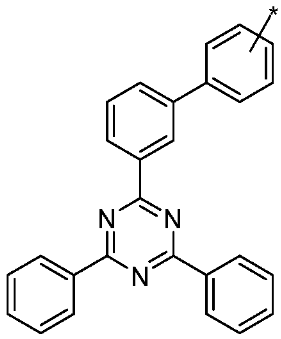 (E19), 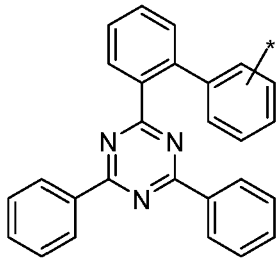 (E20),

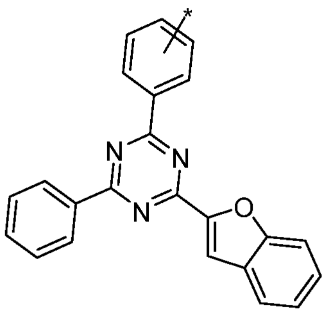 (E21),

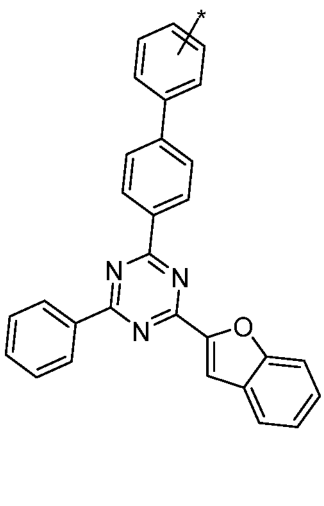 (E22), 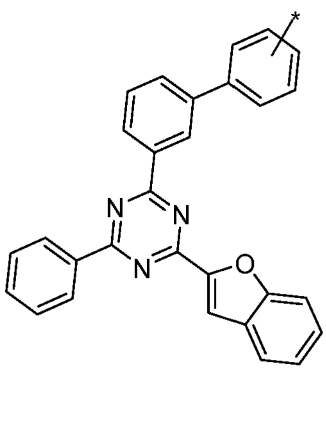 (E23),

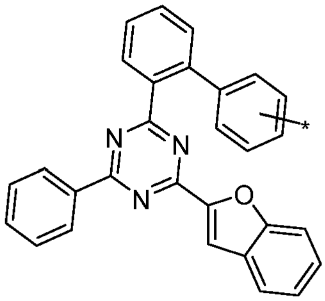 (E24), 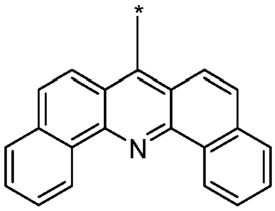 (E25); 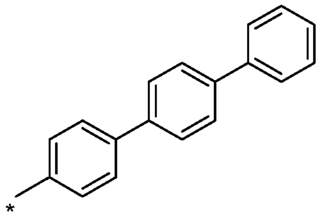 (E26),

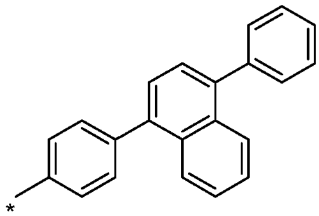 (E27), 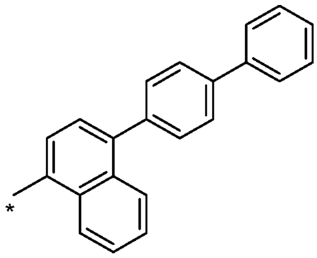 (E28),

and/or

- $Ar^4$ may be independently selected from F1 to F9:

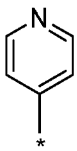 (F1), 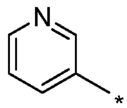 (F2), 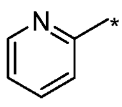 (F3), 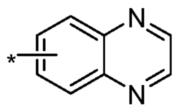 (F4), 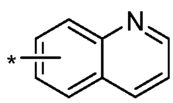 (F5),

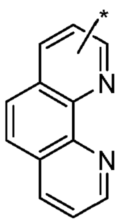 (F6), 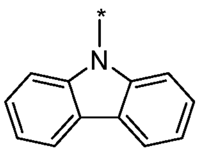 (F7), 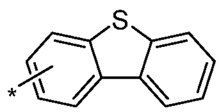 (F8), 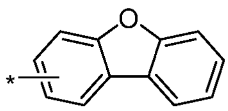 (F9).

The asterix "*"denoting the bonding position at which the substituents of $Ar^1$, $Ar^2$, $Ar^3$ and $Ar^4$ bonds in the compound according to formula 1.

According to another embodiment of the compound of formula 1, wherein the compound of formula 1 may be selected from G1 to G70:

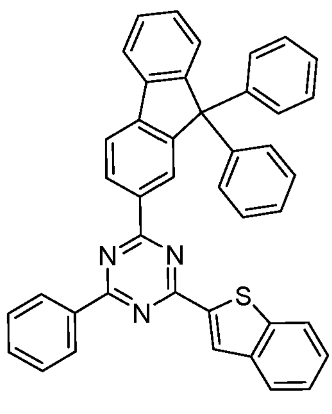 (G1), 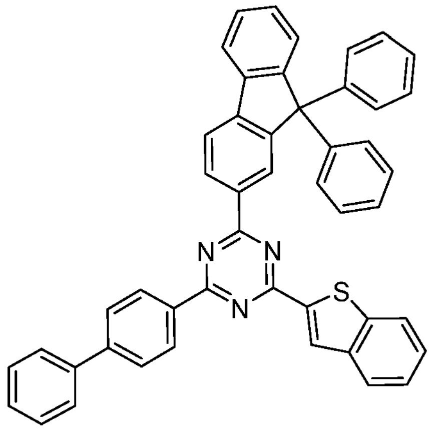 (G2),

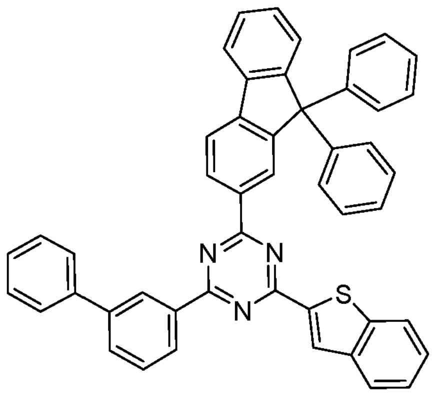 (G3), 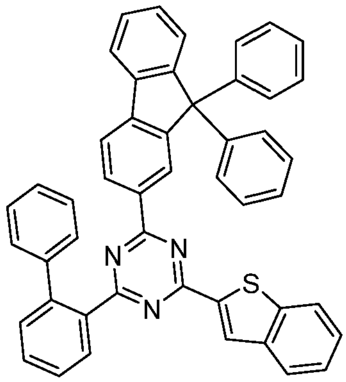 (G4),

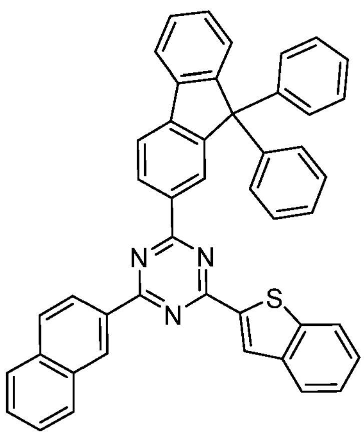 (G5), 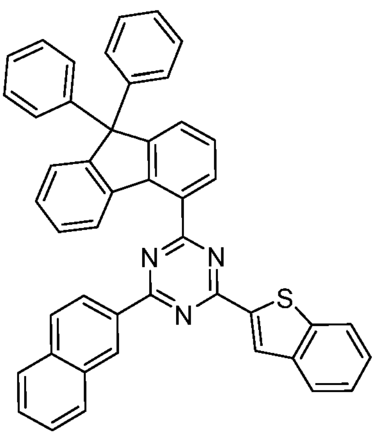 (G6),

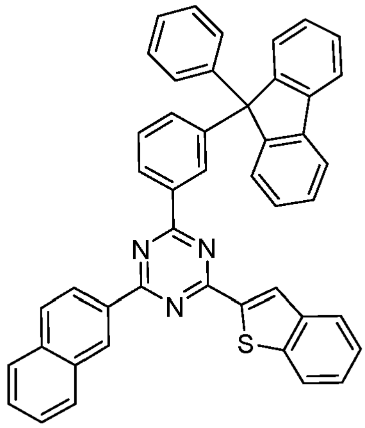

(G7),

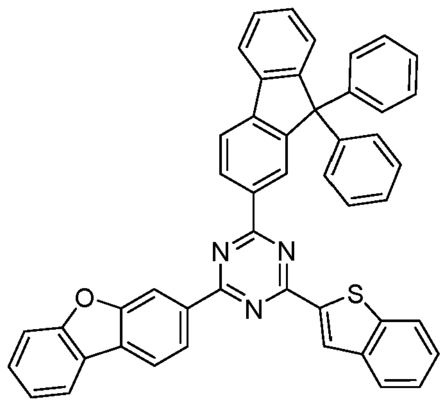

(G8),

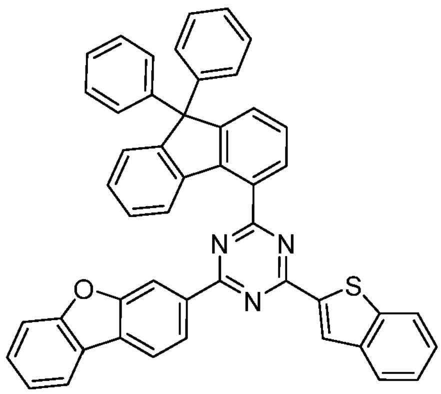

(G9),

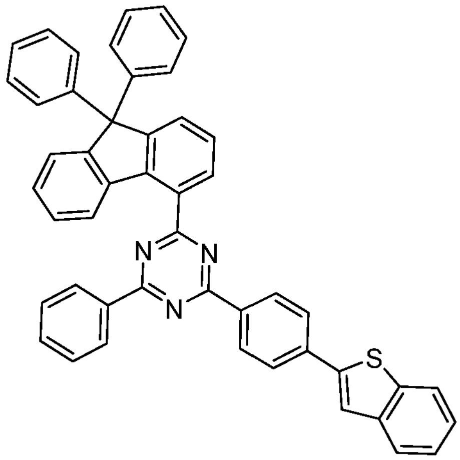

(G10),

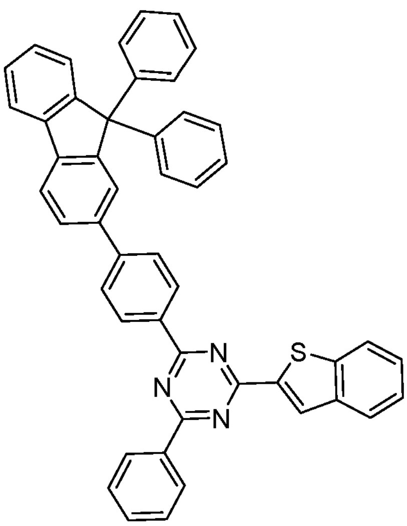

(G11),

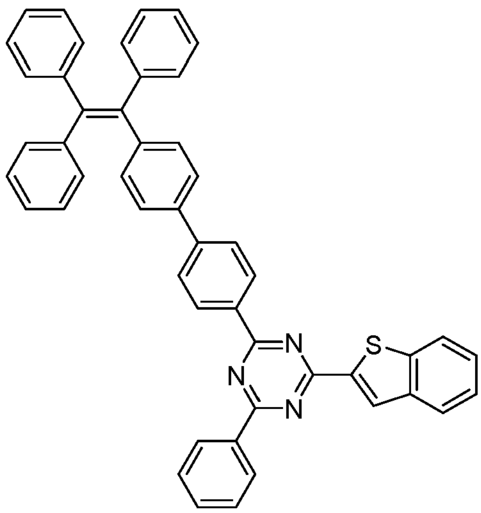

(G12),

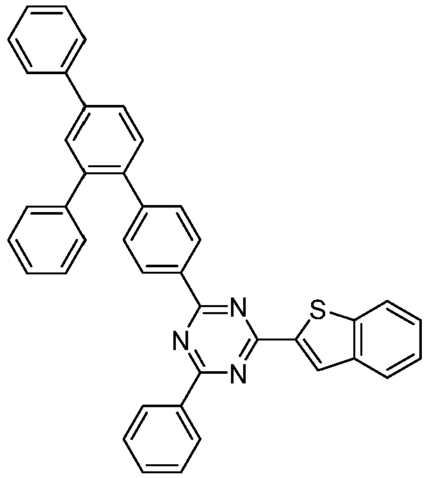

(G13),

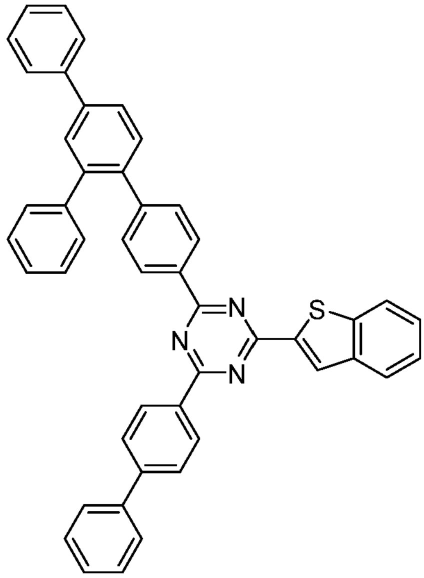

(G14),

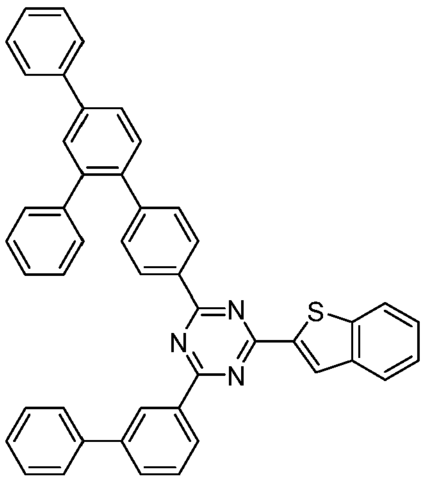

(G15),

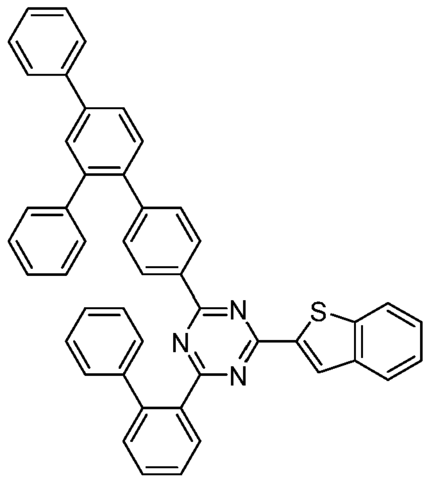

(G16),

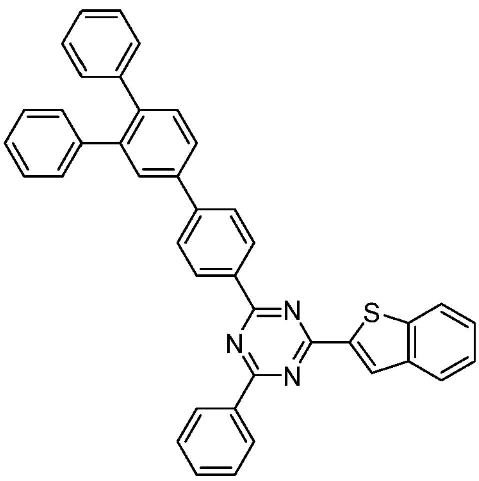

(G17),

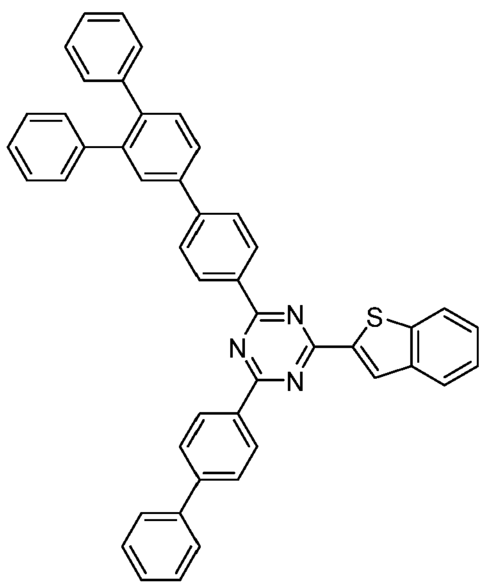

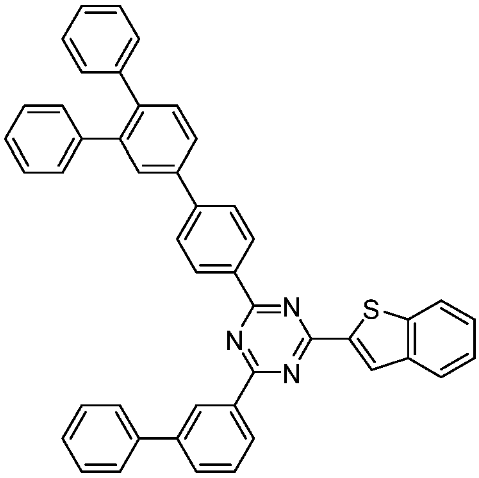

(G18), (G19),

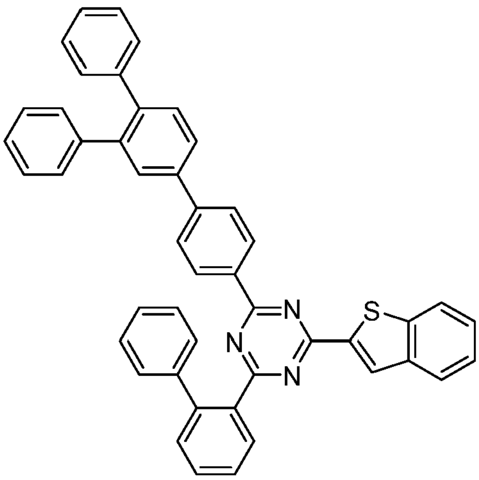

(G20),

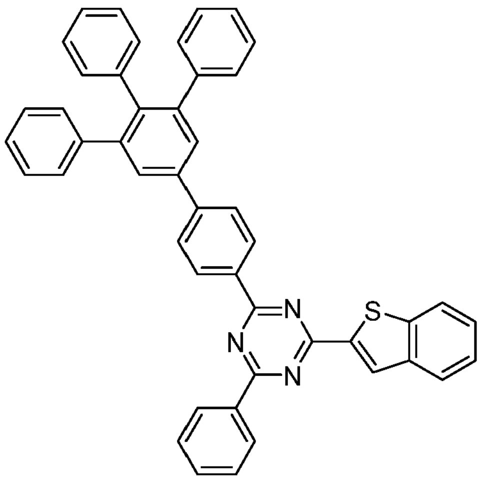

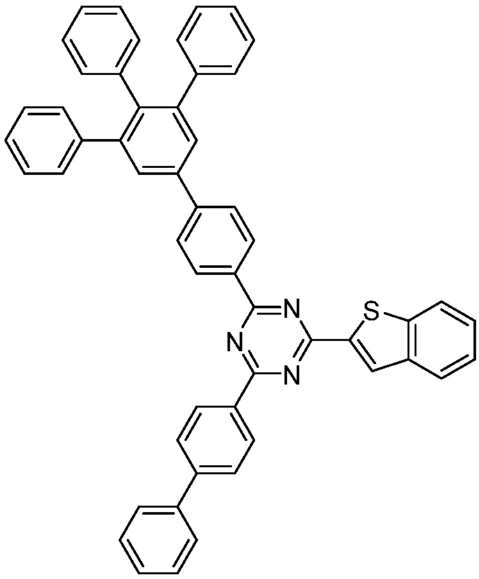

(G21), (G22),

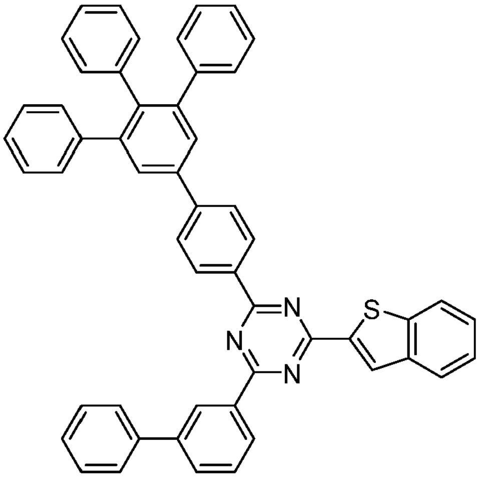

(G23),

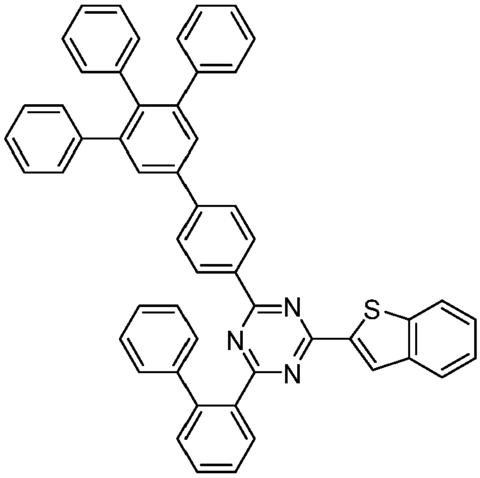

(G24),

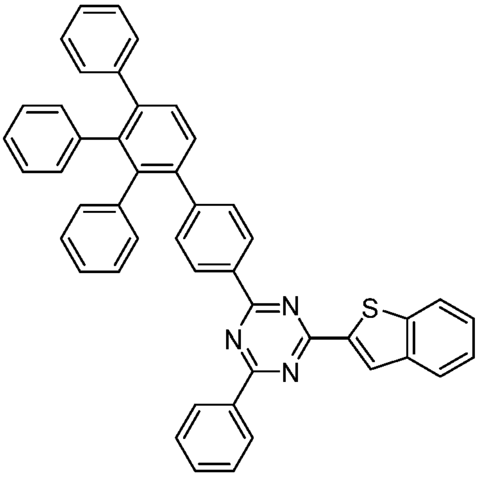

(G25),

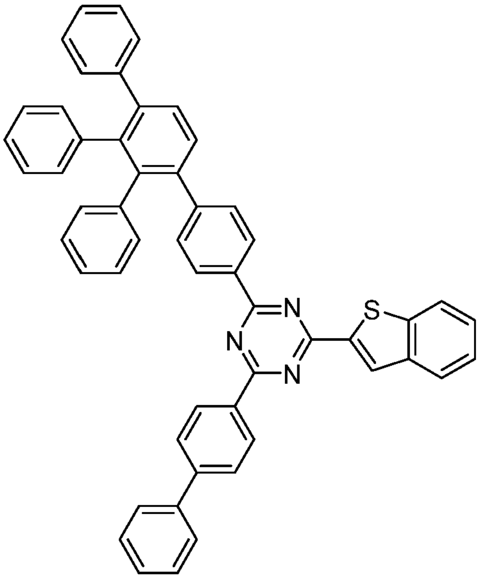

(G26),

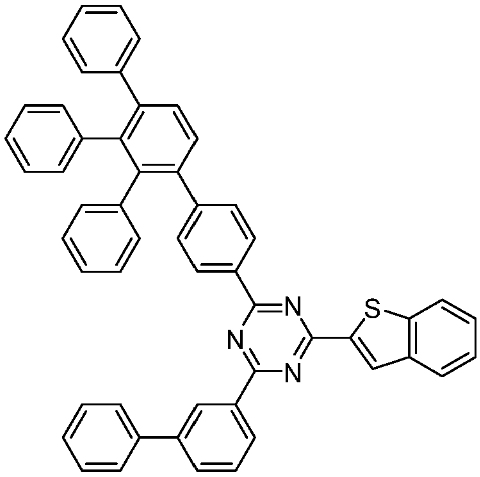

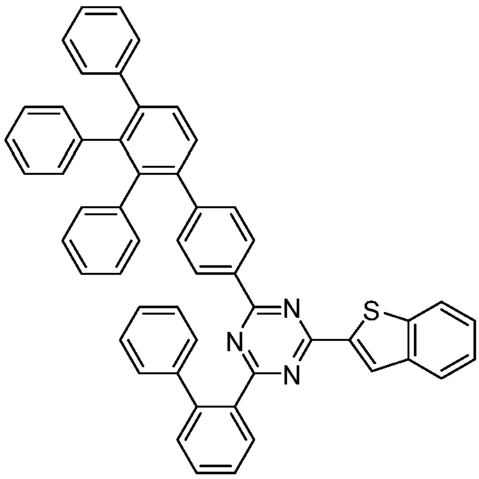

(G27), (G28),

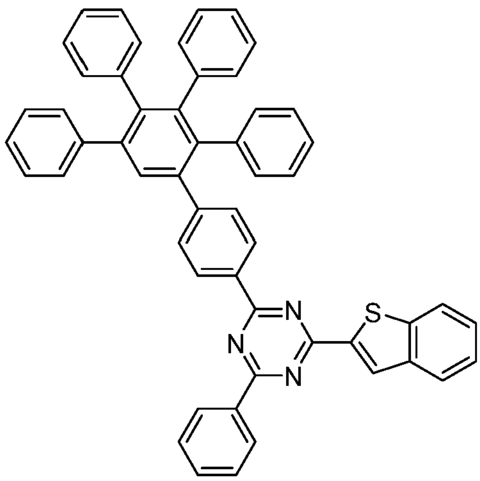

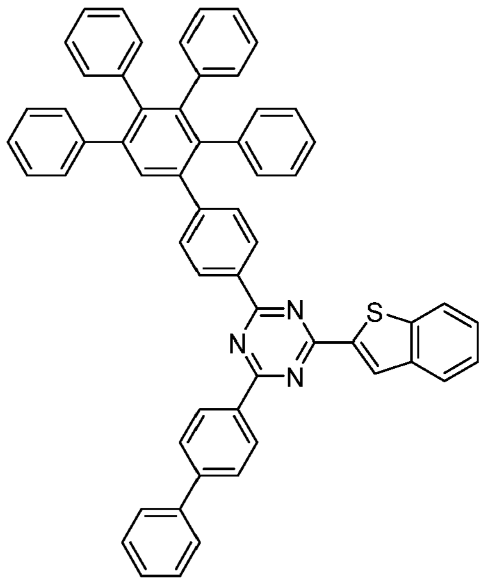

(G29),

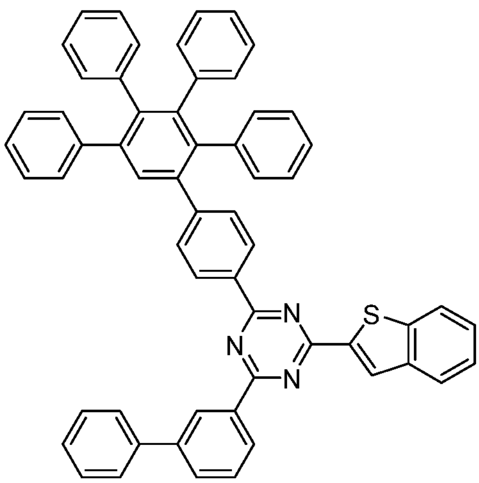

(G30), (G31),

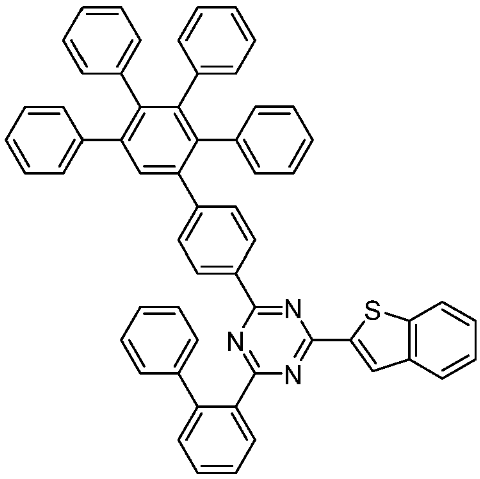

(G32),

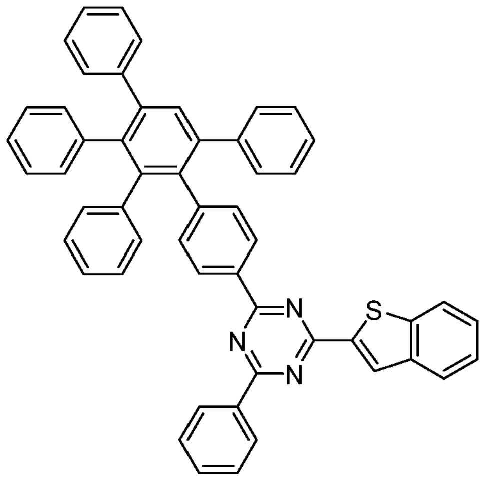

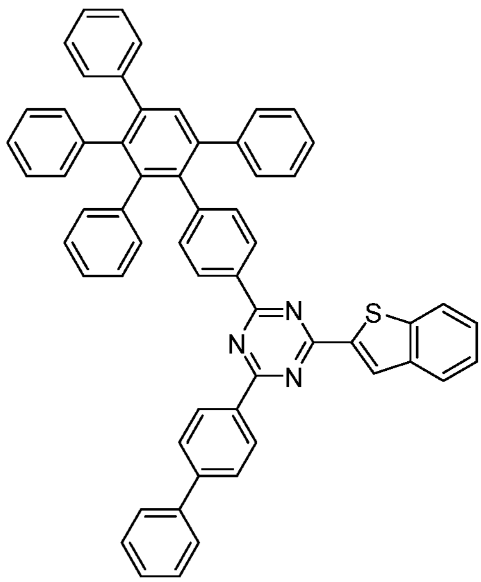

(G33), (G34),

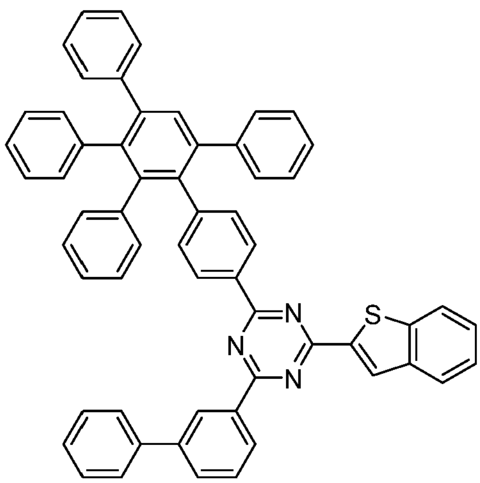

(G35),

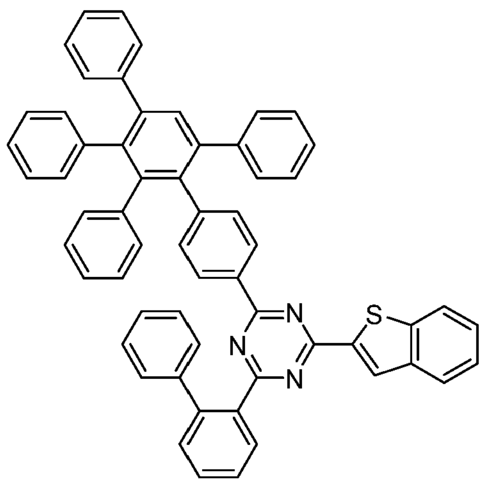

(G36),

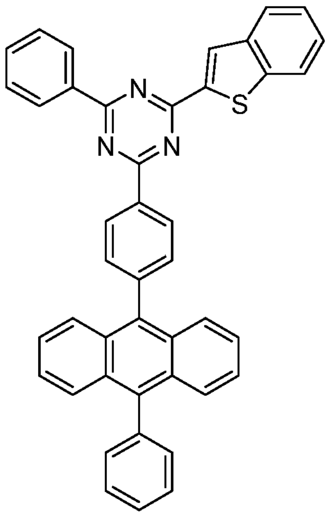

(G37),

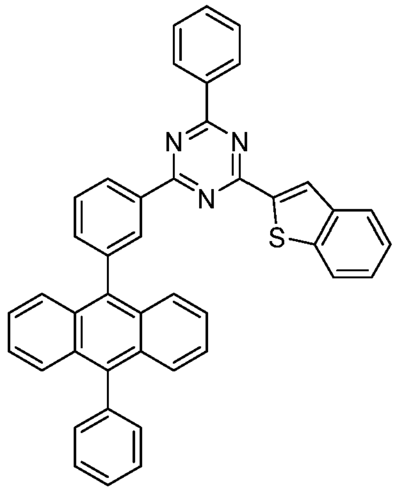

(G38),

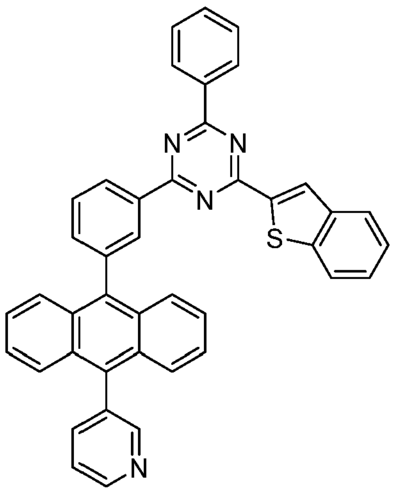

(G39),

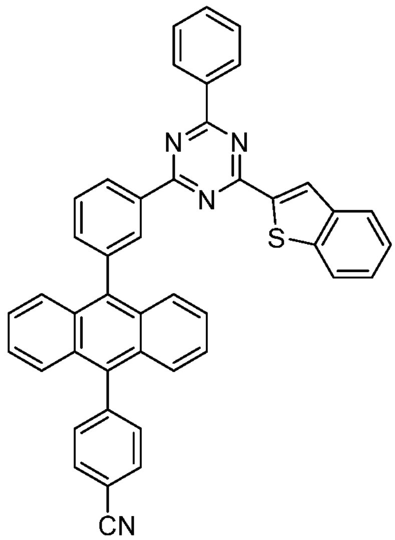

(G40),

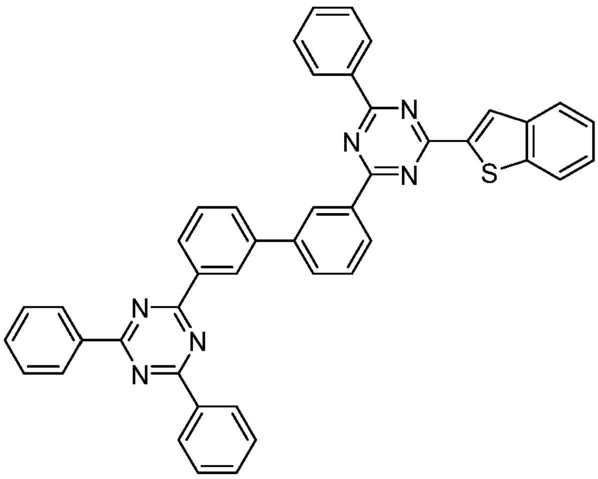

(G41),

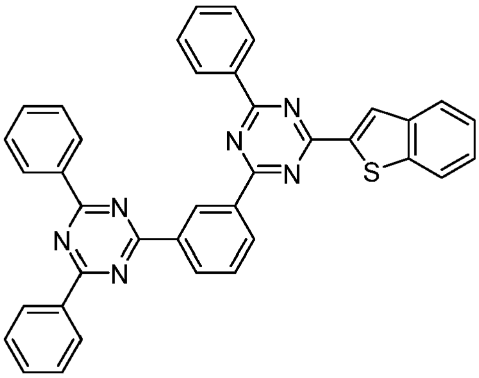

(G42),

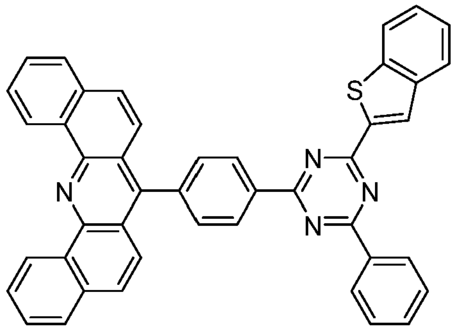

(G43),

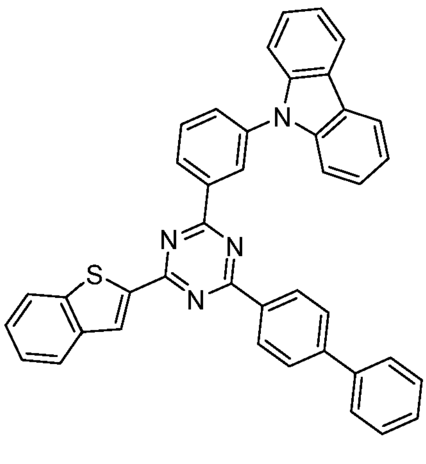

(G44),

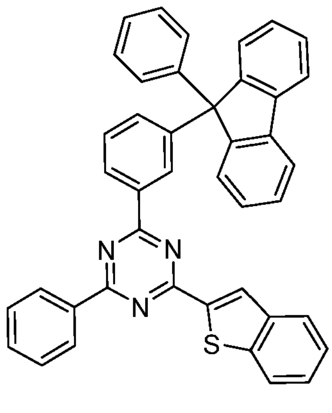

(G45),

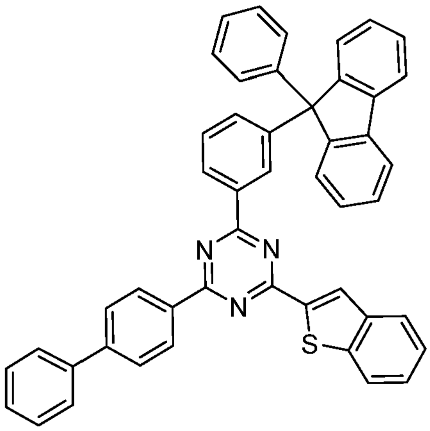

(G46),

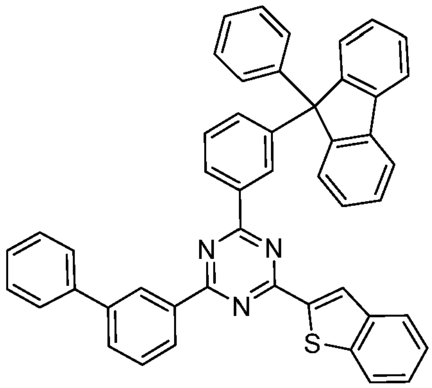

(G47),

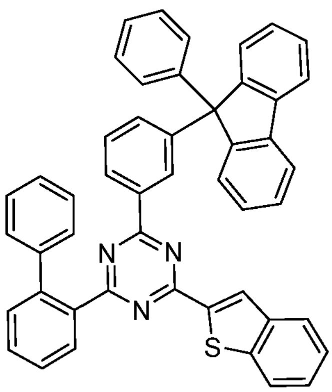

(G48),

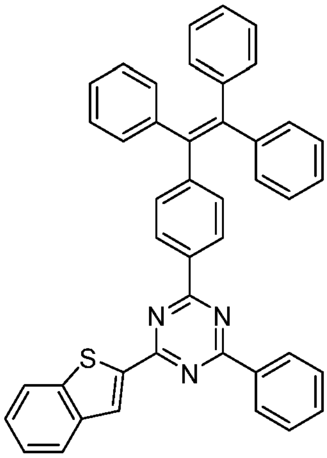

(G49),

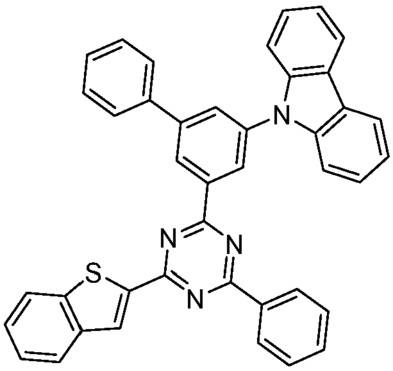

(G50),

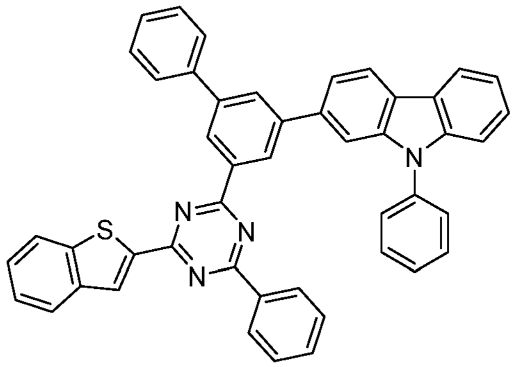

(G51),

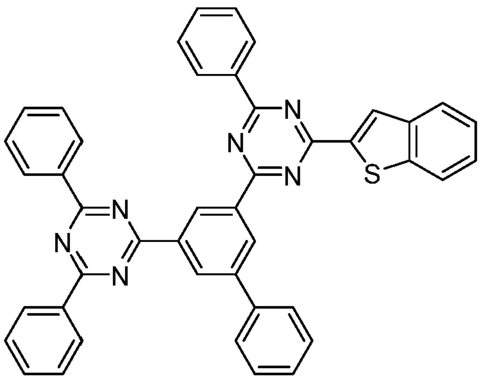

(G52),

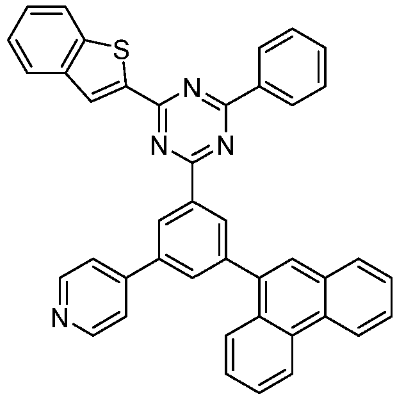

(G53),

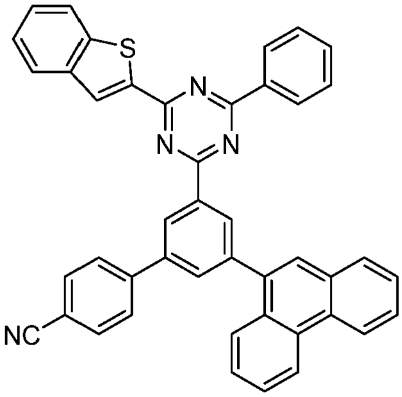

(G54),

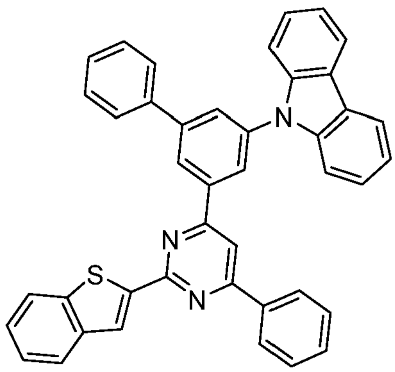

(G55),

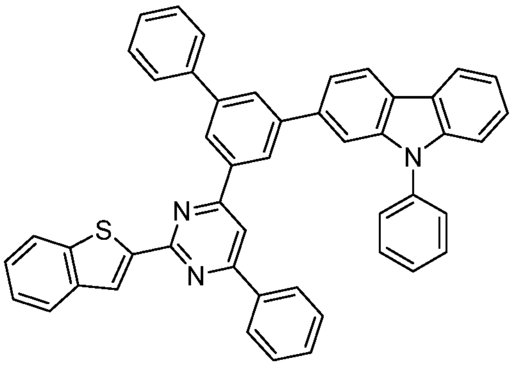

(G57),

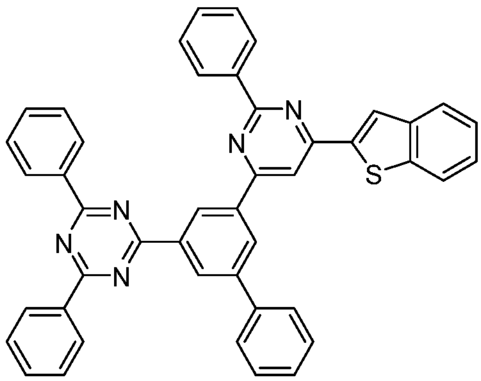

(G58),

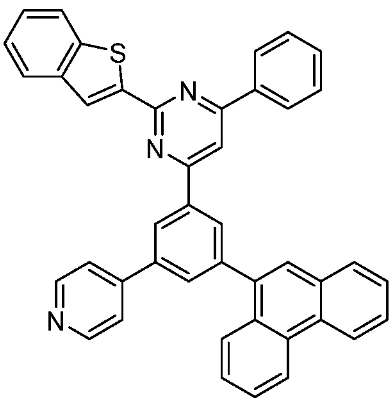

(G59),

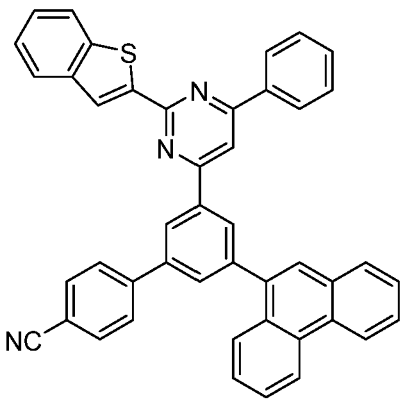

(G60),

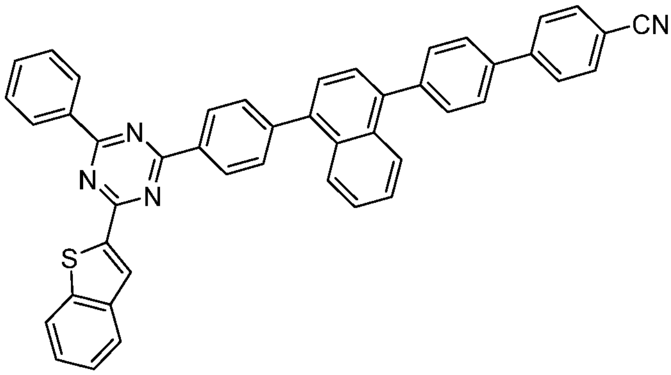

(G61) ,

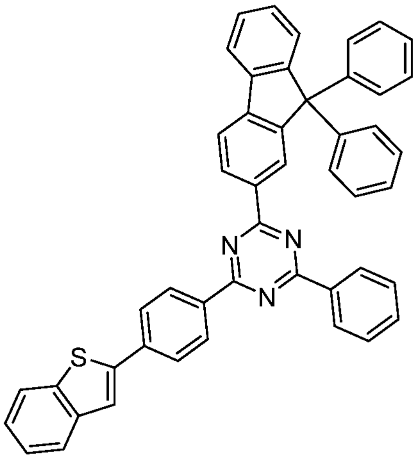

(G62),

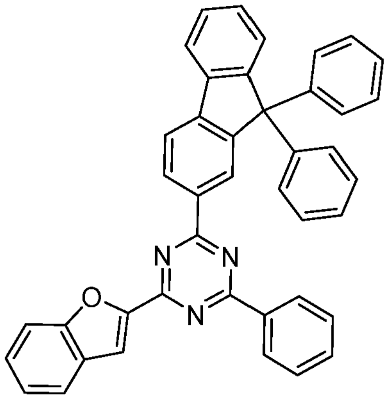

(G63),

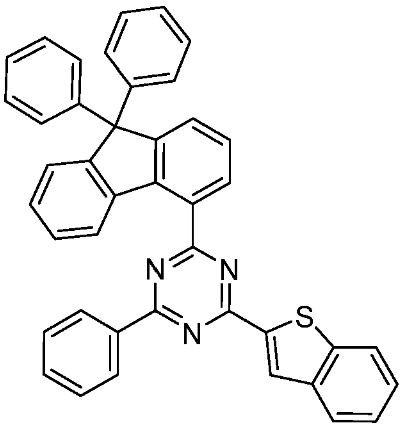

(G64),

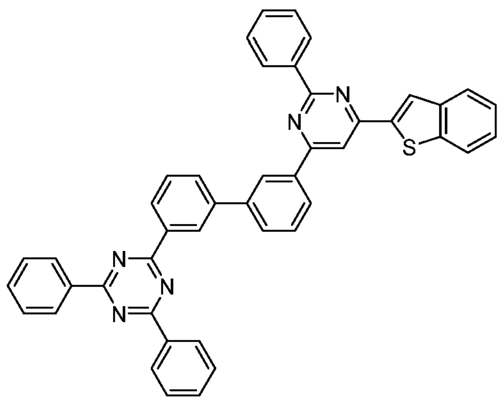

(G65).

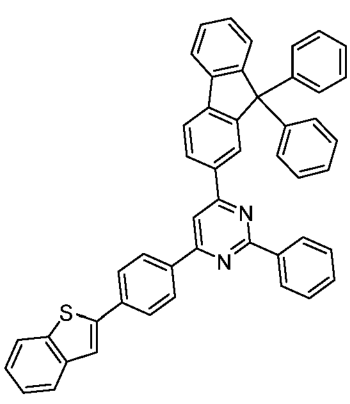

(G66),

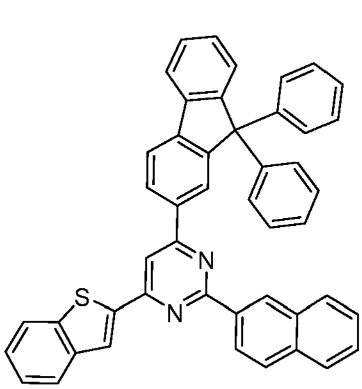

(G67),

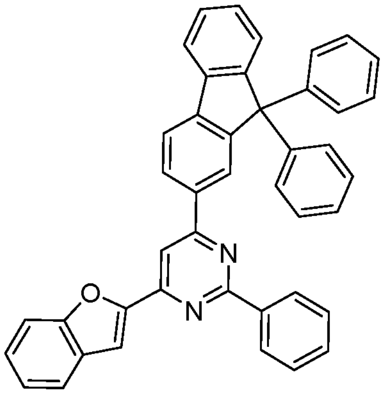

(G68),

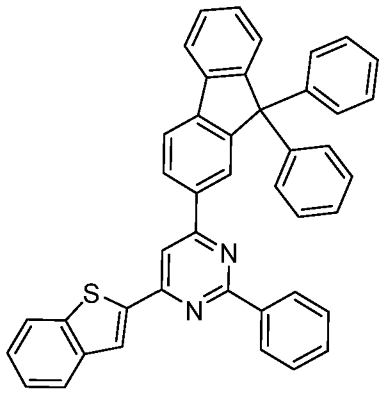

(G69),

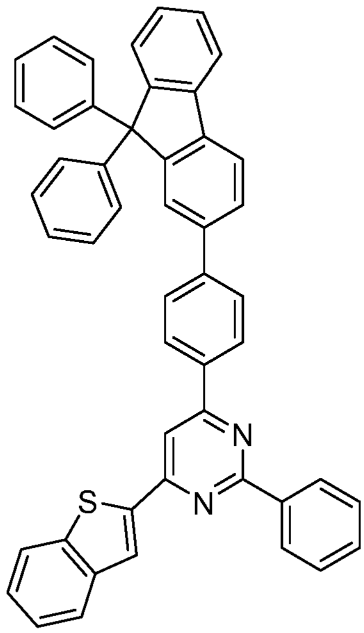

(G70).

According to an aspect the compound of formula 1 can be used as a matrix material for a dopant material.

According to one embodiment the organic semiconductor layer may comprises at least one compound of formula 1.

According to an aspect the layer material can be an organic semiconductor layer, which is used for an organic electronic device. For example, the organic electronic device can be an OLED or there like.

The compounds represented by formula 1 have strong electron transport characteristics to increase charge mobility and/or stability and thereby to improve luminance efficiency, voltage characteristics, and/or lifetime characteristics.

The compounds represented by formula 1 have high electron mobility and a low operating voltage.

In the description the expression "emission layer", "auxiliary ETL" and "first ETL" are synonymously used. ETL means electron transport layer.

The compounds represented by formula 1 and an organic semiconductor layer comprising or comprising of compound of formula 1 may be non-emissive.

In the context of the present specification the term "essentially non-emissive" or "non-emitting" means that the contribution of the compound or layer to the visible emission spectrum from the device is less than 10 %, preferably less than 5 % relative to the visible emission spectrum. The visible emission spectrum is an emission spectrum with a wavelength of ≥ 380 nm to ≤ 780 nm.

Preferably, the organic semiconductor layer comprising the compound of formula 1 is essentially non-emissive or non-emitting.

The term "free of', "does not contain", "does not comprise" does not exclude impurities which may be present in the compounds prior to deposition. Impurities have no technical effect with respect to the object achieved by the present invention.

The operating voltage, also named U, is measured in Volt (V) at 10 milliAmpere per square be centimeter (mA/cm2).

The candela per Ampere efficiency, also named cd/A efficiency, is measured in candela per ampere at 10 milliAmpere per square centimeter (mA/cm2).

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The rate onset temperature is measured in °C and describes the VTE source temperature at which measurable evaporation of a compound commences at a pressure of less than $10^{-5}$ mbar.

The term "OLED", "organic light emitting diode", "organic light emitting device", "organic optoelectronic device" and "organic light-emitting diode" are simultaneously used and have the same meaning.

The term "transition metal" means and comprises any element in the d-block of the periodic table, which comprises groups 3 to 12 elements on the periodic table.

The term "group III to VI metal" means and comprises any metal in groups III to VI of the periodic table.

As used herein, "weight percent", "wt.-%", "percent by weight", "% by weight", and variations thereof refer to a composition, component, substance or agent as the weight of that composition, component, substance or agent of the respective electron transport layer divided by the total weight of the composition thereof and multiplied by 100. It is understood that the total weight percent amount of all components, substances or agents of the respective electron transport layer may be selected such that it does not exceed 100 wt.-%.

As used herein, "volume percent", "vol.-%", "percent by volume", "% by volume", and variations thereof refer to an elemental metal, a composition, component, substance or agent as the volume of that elemental metal, component, substance or agent of the respective electron transport layer divided by the total volume of the respective electron transport layer thereof and multiplied by 100. It is understood that the total volume percent amount of all elemental metal, components, substances or agents of the respective cathode electrode layer may be selected such that it does not exceed 100 vol.-%.

All numeric values are herein assumed to be modified by the term "about", whether or not explicitly indicated. As used herein, the term "about" refers to variation in the numerical quantity that can occur.

Whether or not modified by the term "about", the claims include equivalents to the quantities.

It should be noted that, as used in this specification and the appended claims, the singular forms "a", "an", and "the" include plural referents unless the content clearly dictates otherwise.

It should be noted that, as used in this specification and the appended claims, "*" if not otherwise defined indicates the chemical bonding position.

The anode electrode and cathode electrode may be described as anode electrode / cathode electrode or anode electrode / cathode electrode or anode electrode layer / cathode electrode layer.

According to another aspect, an organic optoelectronic device comprises an anode layer and a cathode layer facing each other and at least one organic semiconductor layer between the anode layer and the cathode layer, wherein the organic semiconductor layer comprises or consists of the compound of formula 1.

According to yet another aspect, a display device comprising the organic electronic device, which can be an organic optoelectronic device, is provided.

In the present specification, when a definition is not otherwise provided, an "alkyl group" may refer to an aliphatic hydrocarbon group. The alkyl group may refer to "a saturated alkyl group" without any double bond or triple bond. The alkyl group may be a linear, cyclic or branched alkyl group.

The term "alkyl group" includes $C_1$ to $C_{16}$ alkyl, $C_3$ to $C_{16}$ branched alkyl, and $C_3$ to $C_{16}$ cyclic alkyl.

The alkyl group may be a $C_1$ to $C_{16}$ alkyl group, or preferably a $C_1$ to $C_{12}$ alkyl group. More specifically, the alkyl group may be a $C_1$ to $C_{14}$ alkyl group, or preferably a $C_1$ to $C_{10}$ alkyl group or a $C_1$ to $C_6$ alkyl group. For example, a $C_1$ to $C_4$ alkyl group comprises 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a tert-butyl group, a pentyl group, a hexyl group, a cyclopropyl group, a cyclobutyl group, a cyclopentyl group, a cyclohexyl group, and the like.

In the present specification "arylene group" may refer to a group comprising at least one hydrocarbon aromatic moiety, and all the elements of the hydrocarbon aromatic moiety may have p-orbitals which form conjugation, for example a phenyl group, a naphthyl group, an anthracenyl group, a phenanthrenyl group, a pyrenyl group, a fluorenyl group and the like, if not otherwise defined.

The term "heteroarylene", also named "heteroaryl ring" may refer to aromatic heterocycles with at least one heteroatom, and all the elements of the hydrocarbon heteroaromatic moiety may have p-orbitals which form conjugation, if not otherwise defined. The heteroatom may be selected from N, O, S, B, Si, P, Se, preferably from N, O and S. The

heteroatom may be preferably selected from N, if not otherwise defined.

A heteroarylene ring may comprise at least 1 to 3 heteroatoms. Preferably a heteroarylene ring may comprise at least 1 to 3 heteroatoms individually selected from N, S and/or O. More preferred a heteroarylene ring may comprise at least 1 to 3 heteroatoms that can be N.

Further preferred in addition to the pyrimidine or triazine group of formula 1 at least one additional heteroaryl/ene ring may comprise at least 1 to 3 N-atoms, or at least 1 to 2-N atoms or at least one N-atom.

According to another preferred embodiment the compound according to formula 1 may comprise:

- at least 6 to 25 aromatic rings, preferably at least 7 to 22 aromatic rings, further preferred at least 8 to 20 aromatic rings, in addition preferred at least 9 to 15 aromatic rings and more preferred at least 10 to 14 aromatic rings; wherein
- at least 1 to 4, preferably 1 to 4 or 2 to 3, are heteroaromatic rings.

According to one embodiment the compound according to formula 1:

- comprises at least 6 to 20 aromatic rings, preferably at least 7 to 18 aromatic rings, further preferred at least 9 to 16 aromatic rings, in addition preferred at least 10 to 15 aromatic rings and more preferred at least 11 to 14 aromatic rings; and/or
- the compound of formula 1 may comprises at least 2 to 6, preferably 3 to 5 or 2 to 4, hetero aromatic rings, wherein the hetero atoms can be selected from N, O, S; and/or
- comprises at least one fluorene ring and at least one hetero-fluorene ring, wherein the hetero atoms can be selected from N, O, S.

According to a further preferred embodiment the compound of formula 1 may comprises at least 2 to 7, preferably 2 to 5, or 2 to 3 hetero aromatic rings.

According to a further preferred embodiment the compound of formula 1 may comprises at least 2 to 7, preferably 2 to 5, or 2 to 3 hetero aromatic rings, wherein at least one of the aromatic rings is a five member hetero aromatic ring.

According to a further preferred embodiment the compound of formula 1 may comprises at least 3 to 7, preferably 3 to 6, or 3 to 5 hetero aromatic rings, wherein at least two of the hetero aromatic rings are five member hetero-aromatic-rings.

According to one embodiment the compound according to formula 1 may comprise at least 6 to 12 non-hetero aromatic rings and 2 to 3 hetero aromatic rings.

According to one preferred embodiment the compound according to formula 1 may comprise at least 7 to 12 non-hetero aromatic rings and 2 to 5 hetero aromatic rings.

According to one preferred embodiment the compound according to formula 1 may comprise at least 7 to 11 non-hetero aromatic rings and 2 to 3 hetero aromatic rings.

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

According to another embodiment the compound of formula 1 may have a melting point of ≥ 250° C and ≤ 380° C, preferably ≥ 260° C and ≤ 370° C, further preferred ≥ 270° C and ≤ 360° C.

Glass transition temperature

The glass transition temperature is measured under nitrogen and using a heating rate of 10 K per min in a Mettler Toledo DSC 822e differential scanning calorimeter as described in DIN EN ISO 11357, published in March 2010.

According to another embodiment the compound of formula 1 may have a glass transition temperature Tg of ≥ 115° C and ≤ 380° C, preferably ≥ 130° C and ≤ 350° C, further preferred ≥ 145° C and ≤ 320° C, in addition preferred ≥ 160° C and ≤ 220° C.

Rate onset temperature

The rate onset temperature is determined by loading 100 mg compound into a VTE source. As VTE source a point source for organic materials is used as supplied by Kurt J. Lesker Company (www.lesker.com) or CreaPhys GmbH (http://www.creaphys.com). The VTE source is heated at a constant rate of 15 K/min at a pressure of less than $10^{-5}$

mbar and the temperature inside the source measured with a thermocouple. Evaporation of the compound is detected with a QCM detector which detects deposition of the compound on the quartz crystal of the detector. The deposition rate on the quartz crystal is measured in Ångstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200° C to 260° C. If the rate onset temperature is below 200° C the evaporation may be too rapid and therefore difficult to control. If the rate onset temperature is above 260° C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

According to another embodiment the compound of formula 1 may have a rate onset temperature $T_{RO}$ of ≥ 200° C and ≤ 350° C, preferably ≥ 220° C and ≤ 350° C, further preferred ≥ 240° C and ≤ 320° C.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy may be selected to determine the bond lengths of the molecules.

According to one embodiment the compounds according to formula 1 may have a dipole moment (Debye) in the range from ≥ 0.1 to ≤ 1.50, preferably from ≥ 0.15 to ≤ 1.

Calculated HOMO and LUMO

The HOMO and LUMO are calculated with the program package TURBOMOLE V6.5. The optimized geometries and the HOMO and LUMO energy levels of the molecular structures are determined by applying the hybrid functional B3LYP with a 6-31G* basis set in the gas phase. If more than one conformation is viable, the conformation with the lowest total energy may be selected.

According to one embodiment the compounds according to formula 1 may have a LUMO energy level (eV) in the range from about- 2.30 eV to - 1.70 eV, preferably from - 1.8 eV to - 2.2 eV.

Molar mass

The molar mass of a compound in g/mol is given by the sum of the standard atomic weight (namely, the standard relative atomic mass) of the atoms which form the compound multiplied by the molar mass constant, $M_u$. $M_u$ is $1 \times 10^{-3}$ kg/mol = 1 g/mol.

According to one embodiment the compounds according to formula 1 may have a molar mass (g/mol) in the range from 450 g/mol, to 1800 g/mol, preferably from 500 to 1600 g/mol, further preferred from 600 g/mol, to 1400 g/mol.

Technical effect

Surprisingly, it was found that the compounds of formula 1 and the inventive organic electronic devices solve the problem underlying the present invention by being superior over the organic electroluminescent devices and compounds known in the art, in particular with respect to cd/A efficiency, also referred to as current efficiency and to lifetime. At the same time the operating voltage is kept at a similar or even improved level which is important for reducing power consumption and increasing battery life, for example of a mobile display device. High cd/A efficiency is important for high efficiency and thereby increased battery life of a mobile device, for example a mobile display device. Long lifetime at high current density is important for the longevity of a device which is run at high brightness.

A high glass transition temperature may offer a technical benefit in high temperature applications, as the morphology of the organic semiconductor layer is less likely to deteriorate. Surprisingly, it was found that the rate onset temperature is still within a range suitable for mass production.

Additionally it was found that despite high glass transition temperature, the compounds of formula 1 offer low evaporation temperature, which may offer a technical advantage over prior art in mass production applications.

The inventors have surprisingly found that particular good performance can be achieved when using the organic electroluminescent device as a fluorescent blue device.

The specific arrangements mentioned herein as preferred were found to be particularly advantageous.

Likewise, some compounds falling within the scope of the broadest definition of the present invention have surprisingly be found to be particularly well performing with respect to the mentioned property of cd/A efficiency and/or lifetime. These compounds are discussed herein to be particularly preferred.

Further an organic optoelectronic device having high efficiency and/or long lifetime may be realized.

Anode

A material for the anode may be a metal or a metal oxide, or an organic material, preferably a material with work function above 4.8 eV, more preferably above 5.1 eV, most preferably above 5.3 eV. Preferred metals are noble metals like Pt, Au or Ag, preferred metal oxides are transparent metal oxides like ITO or IZO which may be advantageously used in bottom-emitting OLEDs having a reflective cathode.

In devices comprising a transparent metal oxide anode or a reflective metal anode, the anode may have a thickness from 50 nm to 100 nm, whereas semitransparent metal anodes may be as thin as from 5 nm to 15 nm, and non-transparent metal anodes may have a thickness from 15 nm to 150nm.

Hole injection layer (HIL)

The hole injection layer may improve interface properties between the anode and an organic material used for the hole transport layer, and is applied on a non-planarized anode and thus may planarize the surface of the anode. For example, the hole injection layer may include a material having a median value of the energy level of its highest occupied molecular orbital (HOMO) between the work function of the anode material and the energy level of the HOMO of the hole transport layer, in order to adjust a difference between the work function of the anode and the energy level of the HOMO of the hole transport layer.

When the hole transport region comprises a hole injection layer 36, the hole injection layer may be formed on the anode by any of a variety of methods, for example, vacuum deposition, spin coating, casting, Langmuir-Blodgett (LB) method, or the like.

When hole injection layer is formed using vacuum deposition, vacuum deposition conditions may vary depending on the material that is used to form the hole injection layer, and the desired structure and thermal properties of the hole injection layer to be formed and for example, vacuum deposition may be performed at a temperature of 100 °C to 500 °C, a pressure of $10^{-6}$ Pa to $10^{-1}$ Pa, and a deposition rate of 0.1 to 10 nm/sec, but the deposition conditions are not limited thereto.

When the hole injection layer is formed using spin coating, the coating conditions may vary depending on the material that is used to form the hole injection layer, and the desired structure and thermal properties of the hole injection layer to be formed. For example, the coating rate may be in the range of 2000 rpm to 5000 rpm, and a temperature at which heat treatment is performed to remove a solvent after coating may be in a range of 80 °C to 200 °C, but the coating conditions are not limited thereto.

The hole injection layer may further comprise a p-dopant to improve conductivity and/or hole injection from the anode.

p-dopant

In another aspect, the p-dopant may be homogeneously dispersed in the hole injection layer.

In another aspect, the p-dopant may be present in the hole injection layer in a higher concentration closer to the anode and in a lower concentration closer to the cathode.

The p-dopant may be one of a quinone derivative or a radialene compound but not limited thereto. Non-limiting examples of the p-dopant are quinone derivatives such as tetracyanoquinonedimethane (TCNQ), 2,3,5,6-tetrafluoro-tetracyano-1,4-benzoquinonedimethane (F4-TCNQ), 4,4',4"-((1E,1'E,1"E)-cyclopropane-1,2,3-triylidenetris(cyanomethanylylidene))-tris(2,3,5,6-tetrafluorobenzonitrile).

According to another embodiment, the device comprising a compound of formula 1 may further comprise a layer comprising a radialene compound and/or a quinodimethane compound.

In another embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have formula (XX) and/or the quinodimethane compound may have formula (XXIa) or (XXIb):

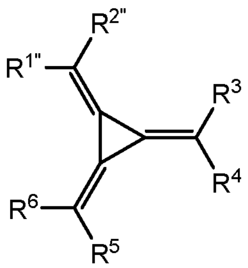
(XX)

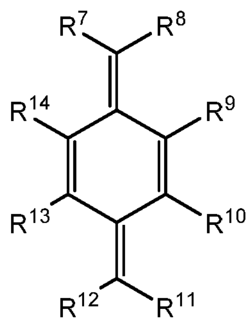
(XXIa)

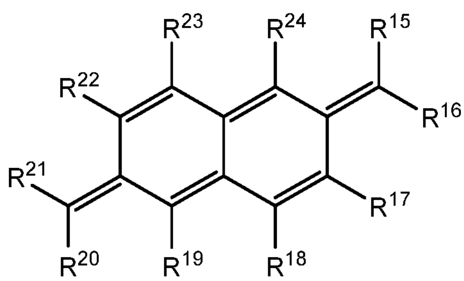
(XXIb),

wherein $R^{1"}$, $R^{2"}$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from an electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and electron withdrawing groups. Electron withdrawing group that can be suitable used are above mentioned.

Hole transport layer (HTL)

Conditions for forming the hole transport layer and the electron blocking layer may be defined based on the above-described formation conditions for the hole injection layer.

A thickness of the hole transport part of the charge transport region may be from 10 nm to 1000 nm, for example, 10 nm to 100 nm. When the hole transport part of the charge transport region comprises the hole injection layer and the hole transport layer, a thickness of the hole injection layer may be from 10 nm to 1000 nm, for example 10 nm to 100 nm and a thickness of the hole transport layer may be from 5 nm to 200 nm, for example 10 nm to 150 nm. When the thicknesses of the hole transport part of the charge transport region, the HIL, and the HTL are within these ranges, satisfactory hole transport characteristics may be obtained without a substantial increase in operating voltage.

Hole transport matrix materials used in the hole transport region are not particularly limited. Preferred are covalent compounds comprising a conjugated system of at least 6 delocalized electrons, preferably organic compounds comprising at least one aromatic ring, more preferably organic compounds comprising at least two aromatic rings, even more preferably organic compounds comprising at least three aromatic rings, most preferably organic compounds comprising at least four aromatic rings. Typical examples of hole transport matrix materials which are widely used in hole transport layers are polycyclic aromatic hydrocarbons, triarylene amine compounds and heterocyclic aromatic compounds. Suitable ranges of frontier orbital energy levels of hole transport matrices useful in various layer of the hole transport region are well-known. In terms of the redox potential of the redox couple HTL matrix/ cation radical of the HTL matrix, the preferred values (if measured for example by cyclic voltammetry against ferrocene/ferrocenium redox couple as reference) may be in the range 0.0 - 1.0 V, more preferably in the range 0.2 - 0.7 V, even more preferably in the range 0.3 - 0.5 V.

Buffer layer

The hole transport part of the charge transport region may further include a buffer layer.

Buffer layer that can be suitable used are disclosed in US 6 140 763, US 6 614 176 and in US2016/248022.

The buffer layer may compensate for an optical resonance distance of light according to a wavelength of the light emitted from the EML, and thus may increase efficiency.

Emission layer (EML)

The emission layer may be formed on the hole transport region by using vacuum deposition, spin coating, casting, LB method, or the like. When the emission layer is formed using vacuum deposition or spin coating, the conditions for deposition and coating may be similar to those for the formation of the hole injection layer, though the conditions for the deposition and coating may vary depending on the material that is used to form the emission layer. The emission layer may include an emitter host (EML host) and an emitter dopant (further only emitter).

A thickness of the emission layer may be 100Å to 1000Å, for example 200Å to 600Å. When the thickness of the emission layer is within these ranges, the emission layer may have improved emission characteristics without a substantial increase in operating voltage.

Emitter host

According to another embodiment, the emission layer comprises compound of formula 1 as emitter host.

The emitter host compound has at least three aromatic rings, which are independently selected from carbocyclic rings and heterocyclic rings.

Other compounds that can be used as the emitter host is an anthracene matrix compound represented by formula 400 below:

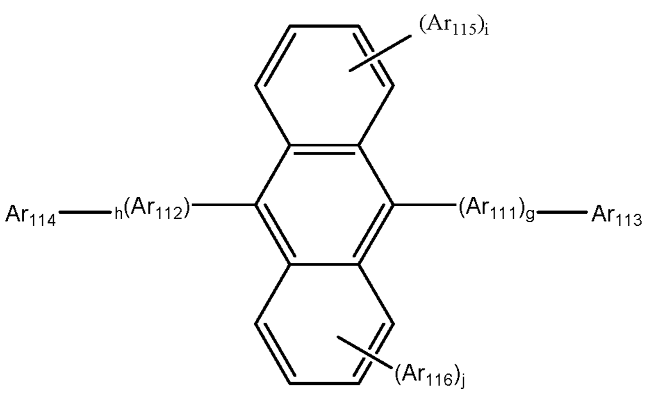

Fortmula 400

In formula 400, $Ar_{111}$ and $Ar_{112}$ may be each independently a substituted or unsubstituted $C_6$-$C_{60}$ arylene group; $Ar_{113}$ to $Ar_{116}$ may be each independently a substituted or unsubstituted $C_1$-$C_{10}$ alkyl group or a substituted or unsubstituted $C_6$-$C_{60}$ arylene group; and g, h, i, and j may be each independently an integer from 0 to 4.

In some embodiments, Arm and $Ar_{112}$ in formula 400 may be each independently one of a phenylene group, a naphthalene group, a phenanthrenylene group, or a pyrenylene group; or

a phenylene group, a naphthalene group, a phenanthrenylene group, a fluorenyl group, or a pyrenylene group, each substituted with at least one of a phenyl group, a naphthyl group, or an anthryl group.

In formula 400, g, h, i, and j may be each independently an integer of 0, 1, or 2.

In formula 400, $Ar_{113}$ to $Ar_{116}$ may be each independently one of

- a $C_1$-$C_{10}$ alkyl group substituted with at least one of a phenyl group, a naphthyl group, or an anthryl group;
- a phenyl group, a naphthyl group, an anthryl group, a pyrenyl group, a phenanthrenyl group, or a fluorenyl group;
- a phenyl group, a naphthyl group, an anthryl group, a pyrenyl group, a phenanthrenyl group, or a fluorenyl group, each substituted with at least one of a deuterium atom, a halogen atom, a hydroxyl group, a cyano group, a nitro group, an amino group, an amidino group, a hydrazine group, a hydrazone group, a carboxyl group or a salt thereof,
- a sulfonic acid group or a salt thereof, a phosphoric acid group or a salt thereof,
- a $C_1$-$C_{60}$ alkyl group, a $C_2$-$C_{60}$ alkenyl group, a $C_2$-$C_{60}$ alkynyl group, a $C_1$-$C_{60}$ alkoxy group, a phenyl group, a naphthyl group, an anthryl group, a pyrenyl group, a phenanthrenyl group, or
- a fluorenyl group

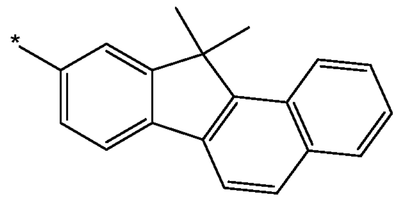

;

or

- formulas 7 or 8

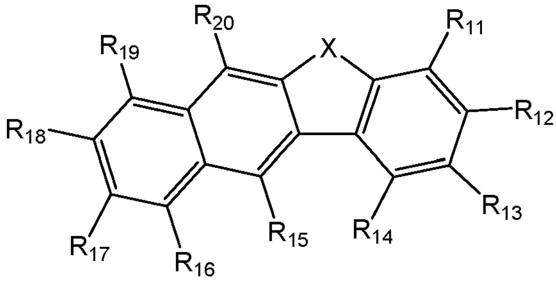

(7),

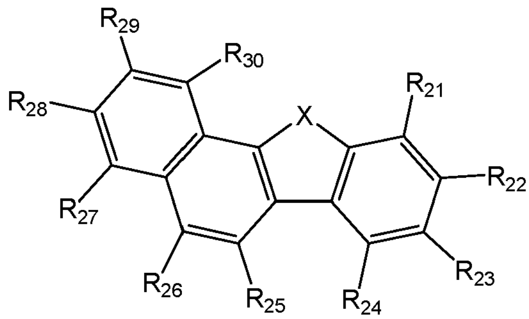

(8).

Wherein in the formulas 7 and 8, X may be selected form an oxygen atom and a sulfur atom, but embodiments of the invention are not limited thereto.

In the formula 7, any one of $R_{11}$ to $R_{14}$ is used for bonding to $Ar_{111}$. $R_{11}$ to $R_{14}$ that are not used for bonding to Arm and $R_{15}$ to $R_{20}$ are the same as $R_1$ to $R_8$.

In the formula 8, any one of $R_{21}$ to $R_{24}$ is used for bonding to Arm. $R_{21}$ to $R_{24}$ that are not used for bonding to $Ar_{111}$ and $R_{25}$ to $R_{30}$ are the same as $R_1$ to $R_8$.

Preferably, the EML host comprises between one and three heteroatoms selected from the group comprising of N, O or S. More preferred the EML host comprises one heteroatom selected from S or O.

<u>Emitter dopant</u>

The dopant is mixed in a small amount to cause light emission, and may be generally a material such as a metal complex that emits light by multiple excitation into a triplet or more. The dopant may be, for example an inorganic,

organic, or organic/inorganic compound, and one or more kinds thereof may be used.

The emitter may be a red, green, or blue emitter.

The dopant may be a fluorescent dopant, for example ter-fluorene, the structures are shown below. 4.4'-bis(4-diphenyl amiostyryl)biphenyl (DPAVBI, 2,5,8,11-tetra-tert-butyl perylene (TBPe), and Compound 8 below are examples of fluorescent blue dopants.

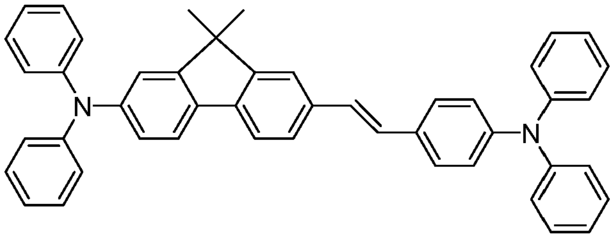

Compound 8

The dopant may be a phosphorescent dopant, and examples of the phosphorescent dopant may be an organic metal compound comprising Ir, Pt, Os, Ti, Zr, Hf, Eu, Tb, Tm, Fe, Co, Ni, Ru, Rh, Pd, or a combination thereof. The phosphorescent dopant may be, for example a compound represented by formula Z, but is not limited thereto:

$J_2MX$ (Z).

In formula Z, M is a metal, and J and X are the same or different, and are a ligand to form a complex compound with M.

The M may be, for example Ir, Pt, Os, Ti, Zr, Hf, Eu, Tb, Tm, Fe, Co, Ni, Ru, Rh, Pd or a combination thereof, and the J and X may be, for example a bidendate ligand.

One or more emission layers may be arranged between the anode and the cathode. To increase overall performance, two or more emission layers may be present.

Charge generation layer

A charge generation layer (also named CGL) may be arranged between the first and the second emission layer, and second and third emission layer, if present. Typically, the CGL comprises a n-type charge generation layer (also named n-CGL or electron generation layer) and a p-type charge generation layer (also named p-CGL or hole generation layer). An interlayer may be arranged between the n-type CGL and the p-type CGL.

In one aspect, the n-type CGL may comprise a compound of formula 1. The n-type CGL further comprises a metal, metal salt or organic metal complex, preferably a metal. The metal may be selected from an alkali, alkaline earth or rare earth metal.

The p-type CGL may comprise a dipyrazino[2,3-f:2',3'-h]quinoxaline, a quinone compound or a radialene compound, preferably dipyrazino[2,3-f:2',3'-h]quinoxaline-2,3,6,7,10,11-hexacarbonitrile or a compound or formula (XX) and/or a compound of formula (XXIa) or (XXIb) as mentioned above.

In another aspect, the n-type and p-type CGL are in direct contact.

Electron transport layer (ETL)

According to another embodiment, the organic semiconductor layer that comprises compound of formula 1 is an electron transport layer. In another embodiment the electron transport layer may consist of compound of formula 1.

For example, an organic light emitting diode according to an embodiment of the present invention comprises at least one electron transport layer, and in this case, the electron transport layer comprises at least one compound of formula 1, or preferably of at least one compound of formulae G1 to G70, preferably of G1, G11, G29 or G45.

In another embodiment, the organic electronic device comprises an electron transport region of a stack of organic layers formed by two or more electron transport layers, wherein at least one electron transport layer comprises a compound of formula 1.

The electron transport layer may include one or two or more different electron transport compounds.

According to another embodiment, a first electron transport layer comprises at least one compound of formula 1 according to the invention and a second electron transport layer comprises a matrix compound, which may be selected different to the compound of formula 1 according to the invention, and may be selected from:

- an anthracene based compound or a hetero substituted anthracene based compound, preferably 2-(4-(9,10-di(naphthalen-2-yl)anthracene-2-yl)phenyl)-1-phenyl-1H-benzo[d]imidazole and/or N4,N4"-di(naphthalen-1-yl)-N4,N4"-diphenyl-[1,1':4',1"-terphenyl]-4,4"-diamine and/or
- a triazine based compound, preferably the triazine based compound comprising aryl and/or heteroaryl substituents, preferably aryl substituents, and/or
- a phosphine oxide based compound, preferably (3-(dibenzo[c,h]acridin-7-yl)phenyl)diphenylphosphine oxide and/or phenyl bis(3-(pyren-1-yl)phenyl)phosphine oxide and/or 3-Phenyl-3H-benzo[b]dinaphtho[2,1-d:1',2'-f]phosphepine-3-oxide; or
- a substituted phenanthroline compound, preferably 2,4,7,9-tetraphenyl-1,10-phenanthroline or 2,9-di(biphenyl-4-yl)-4,7-diphenyl-1,10-phenanthroline.

According to another embodiment a first electron transport layer comprises at least one compound of formula 1 according to the invention and a second electron transport layer comprises a matrix compound, which may be selected different to the compound of formula 1 according to the invention, and may be selected from a phosphine oxide based compound, preferably (3-(dibenzo[c,h]acridin-7-yl)phenyl)diphenylphosphine oxide and/or phenyl bis(3-(pyren-1-yl)phenyl)phosphine oxide and/or 3-Phenyl-3H-benzo[b]dinaphtho[2,1-d:1',2'-f]phosphepine-3 -oxide.
The first electron transport layer may also be described as auxiliary electron transport layer or a hole blocking layer.
According to another embodiment, a first and a second electron transport layers comprise compound of formula 1, wherein the compound of formula 1 is not selected the same.
The thickness of the electron transport layer may be from 0.5 nm to 100 nm, for example 2 nm to 40 nm, preferably 2 to 10 nm. The thickness of the first electron transport layer may be from 0.5 nm to 100 nm, for example 2 nm to 40 nm, preferably 2 to 10 nm.
A thickness of an optional second electron transport layer may be 1 nm to 100 nm, for example 2 nm to 50 nm, preferably 10 to 40 nm. When the thickness of the electron transport layer is within these ranges, the electron transport layer may have satisfactory electron transporting ability without a substantial increase in operating voltage.
The electron transport layer may further comprise a monovalent or divalent metal halide or an organic monovalent or divalent metal complex, preferably an alkali halide and/or alkali organic complex.
According to another embodiment, the first and second electron transport layers comprise compound of formula 1, wherein the second electron transport layer further comprises an alkali halide and/or alkali organic complex.

## Alkali halide

Alkali halides, also known as alkali metal halides, are the family of inorganic compounds with the chemical formula MX, where M is an alkali metal and X is a halogen.
M can be selected from Li, Na, Potassium, Rubidium and Cesium.
X can be selected from F, Cl, Br and J.
According to various embodiments of the present invention a lithium halide may be preferred. The lithium halide can be selected from the group comprising LiF, LiCl, LiBr and LiJ. However, most preferred is LiF.
The alkali halide is essentially non-emissive or non-emissive.

## Alkali organic complex

The alkali organic complex comprises an alkali metal and at least one organic ligand. The alkali metal is preferably selected from lithium.
According to various embodiments of the present invention the organic ligand of the lithium organic complex is a quinolate, a borate, a phenolate, a pyridinolate or a Schiff base ligand;

- preferably the lithium quinolate complex has the formula III, IV or V:

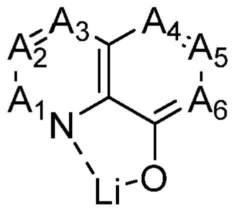 (III), 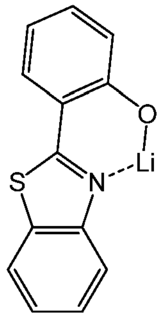 (IV), 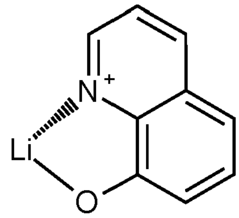 (V),

wherein

$A_1$ to $A_6$ are same or independently selected from CH, CR, N and O;
R is same or independently selected from hydrogen, halogen, alkyl or arylene or heteroarylene with 1 to 20 carbon atoms; and more preferred A1 to A6 are CH;

- preferably the borate based organic ligand is a tetra(1H-pyrazol-1-yl)borate;
- preferably the phenolate is a 2-(pyridin-2-yl)phenolate, a 2-(diphenylphosphoryl)phenolate, an imidazol phenolates, or 2-(pyridin-2-yl)phenolate and more preferred 2-(1-phenyl-1H-benzo[d]imidazol-2-yl)phenolate;
- preferably the pyridinolate is a 2-(diphenylphosphoryl)pyridin-3-olate.

According to various embodiments of the present invention the organic ligand of the alkali organic complex, preferably of a lithium organic complex, can be a quinolate. Quinolates that can be suitable used are disclosed in WO 2013079217 A1.
According to various embodiments of the present invention the organic ligand of the lithium organic complex can be a borate based organic ligand, Preferably the lithium organic complex is a lithium tetra(1H-pyrazol-1-yl)borate. Borate based organic ligands that can be suitable used are disclosed in WO 2013079676 A1.
According to various embodiments of the present invention the organic ligand of the lithium organic complex can be a phenolate ligand, Preferably the lithium organic complex is a lithium 2-(diphenylphosphoryl)phenolate. Phenolate ligands that can be suitable used are disclosed in WO 2013079678 A1.
Further, phenolate ligands can be selected from the group of pyridinolate, preferably 2-(diphenylphosphoryl)pyridin-3-olate. Pyridine phenolate ligands that can be suitable used are disclosed in JP 2008195623.
In addition, phenolate ligands can be selected from the group of imidazol phenolates, preferably 2-(1-phenyl-1H-benzo[d]imidazol-2-yl)phenolate. Imidazol phenolate ligands that can be suitable used are disclosed in JP 2001291593.
Also, phenolate ligands can be selected from the group of oxazol phenolates, preferably 2-(benzo[d]oxazol-2-yl)phenolate. Oxazol phenolate ligands that can be suitable used are disclosed in US 20030165711.
The alkali organic complex may be essentially non-emissive.

Electron injection layer (EIL)

According to another aspect of the invention, the organic electroluminescent device may further comprise an electron injection layer between the electron transport layer (first-ETL) and the cathode.
The electron injection layer (EIL) may facilitate injection of electrons from the cathode.
According to another aspect of the invention, the electron injection layer comprises:

(i) an electropositive metal selected from alkali metals, alkaline earth metals and rare earth metals in substantially elemental form, preferably selected from Li, Na, K, Rb, Cs, Mg, Ca, Sr, Ba, Eu and Yb, more preferably from Li, Na, Mg, Ca, Sr and Yb, even more preferably from Li and Yb, most preferably Yb; and/or
(ii) an alkali metal complex and/or alkali metal salt, preferably the Li complex and/or salt, more preferably a Li quinolinolate, even more preferably a lithium 8-hydroxyquinolinolate, most preferably the alkali metal salt and/or complex of the second electron transport layer (second-ETL) is identical with the alkali metal salt and/or complex of the injection layer.

The electron injection layer may include at least one selected from LiF, NaCl, CsF, $Li_2O$, and BaO.
A thickness of the EIl, may be from 0.1 nm to 10 nm, or 0.3 nm to 9 nm. When the thickness of the electron

injection layer is within these ranges, the electron injection layer may have satisfactory electron injection ability without a substantial increase in operating voltage.

The electron injection layer may comprise a compound of formula 1.

Cathode

A material for the cathode may be a metal, an alloy, or an electrically conductive compound that have a low work function, or a combination thereof. Specific examples of the material for the cathode may be lithium (Li), magnesium (Mg), aluminum (Al), aluminumlithium (Al-Li), calcium (Ca), magnesium-indium (Mg-In), magnesium-silver (Mg-Ag), silver (Ag) etc. In order to manufacture a top-emission light-emitting device having a reflective anode deposited on a substrate, the cathode may be formed as a light-transmissive electrode from, for example, indium tin oxide (ITO), indium zinc oxide (IZO) or silver (Ag).

In devices comprising a transparent metal oxide cathode or a reflective metal cathode, the cathode may have a thickness from 50 nm to 100 nm, whereas semitransparent metal cathodes may be as thin as from 5 nm to 15 nm.

Substrate

A substrate may be further disposed under the anode or on the cathode. The substrate may be a substrate that is used in a general organic light emitting diode and may be a glass substrate or a transparent plastic substrate with strong mechanical strength, thermal stability, transparency, surface smoothness, ease of handling, and water resistance.

Hereinafter, the embodiments are illustrated in more detail with reference to examples. However, the present disclosure is not limited to the following examples.

**Description of the Drawings**

These and/or other aspects and advantages of the present invention will become apparent and more readily appreciated from the following description of the exemplary embodiments, taken in conjunction with the accompanying drawings, of which:

FIG. 1 is a schematic sectional view of an organic light-emitting diode (OLED), according to an exemplary embodiment of the present invention with an emission layer, one electron transport layer and an electron injection layer;

FIG. 2 is a schematic sectional view of an organic light-emitting diode (OLED), according to an exemplary embodiment of the present invention with an emission layer and two electron transport layers;

FIG. 3 is a schematic sectional view of an OLED, according to an exemplary embodiment of the present invention with an emission layer and three electron transport layers;

FIG. 4 is a schematic sectional view of an organic light-emitting diode (OLED), according to an exemplary embodiment of the present invention with an emission layer and one electron transport layer;

FIG. 5 is a schematic sectional view of an organic light-emitting diode (OLED), according to an exemplary embodiment of the present invention with an emission layer and two electron transport layers;

FIG. 6 is a schematic sectional view of an OLED, according to an exemplary embodiment of the present invention with an emission layer and three electron transport layers.

Reference will now be made in detail to the exemplary aspects, examples of which are illustrated in the accompanying drawings, wherein like reference numerals refer to the like elements throughout. The exemplary embodiments are described below, in order to explain the aspects, by referring to the figures.

Herein, when a first element is referred to as being formed or disposed "on" a second element, the first element can be disposed directly on the second element, or one or more other elements may be disposed there between. When a first element is referred to as being formed or disposed "directly on" a second element, no other elements are disposed there between.

The term "contacting sandwiched" refers to an arrangement of three layers whereby the layer in the middle is in direct contact with the two adjacent layers.

The organic light emitting diodes according to an embodiment of the present invention may include a hole transport region; an emission layer; and a first electron transport layer comprising a compound according to formula 1.

FIG. 1 is a schematic sectional view of an organic light-emitting diode 100, according to an exemplary embodiment of the present invention. The OLED 100 comprises an emission layer 150, an electron transport layer (ETL) 161 comprising compound of formula 1 and an electron injection layer 180, whereby the first electron transport layer 161 is disposed directly on the emission layer 150 and the electron injection layer 180 is disposed directly on the first electron transport layer 161.

FIG. 2 is a schematic sectional view of an organic light-emitting diode 100, according to an exemplary embodiment of the present invention. The OLED 100 comprises an emission layer 150 and an electron transport layer stack (ETL) 160 comprising a first electron transport layer (ETL1) 161 comprising compound of formula 1 and a second electron transport layer (ETL2) 162, whereby the second electron transport layer 162 is disposed directly on the first electron transport layer 161. Alternatively, the electron transport layer stack (ETL) 160 comprises a first electron transport layer 161 and a second electron transport layer 162 comprising a compound of formula 1, whereby the second electron transport layer 162 is disposed directly on the first electron transport layer 161.

FIG. 3 is a schematic sectional view of an organic light-emitting diode 100, according to an exemplary embodiment of the present invention. The OLED 100 comprises an emission layer 150 and an electron transport layer stack (ETL) 160 comprising a first electron transport layer (ETL1) 161 that comprises compound of formula 1, a second electron transport layer (ETL2) 162 that comprises compound of formula 1 but different to the compound of the first electron transport layer, and a third electron transport layer (ETL3) 163, whereby the second electron transport layer 162 is disposed directly on the first electron transport layer 161 and the third electron transport layer 163 is disposed directly on the first electron transport layer 162.

FIG. 4 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, one first electron transport layer (ETL1) 161, an electron injection layer (EIL) 180, and a cathode electrode 190. The first electron transport layer (ETL1) 161 comprises compound of formula 1 and optionally an alkali halide or alkali organic complex. The electron transport layer (ETL1) 161 is formed directly on the EML 150.

FIG. 5 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, an electron transport layer stack (ETL) 160, an electron injection layer (EIL) 180, and a cathode electrode 190. The electron transport layer (ETL) 160 comprises a first electron transport layer 161 and a second electron transport layer 162, wherein the first electron transport layer is arranged near to the anode (120) and the second electron transport layer is arranged near to the cathode (190). The first and/or the second electron transport layer comprise compound of formula 1 and optionally an alkali halide or alkali organic complex.

FIG. 6 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, an electron transport layer stack (ETL) 160, an electron injection layer (EIL) 180, and a second cathode electrode 190. The electron transport layer stack (ETL) 160 comprises a first electron transport layer (ETL1) 161, a second electron transport layer (ETL2) 162 and a third electron transport layer (ETL3) 163. The first electron transport layer 161 is formed directly on the emission layer (EML) 150. The first, second and/or third electron transport layer comprise compound of formula 1 that is different for each layer, and optionally an alkali halide or alkali organic complex.

## Organic semiconductor layer

According to another aspect an organic semiconductor layer may comprise at least one compound of formula 1 and/or formula 1a or formula 1b.

According to one embodiment the organic semiconductor layer may comprises at least one compound of formula 1 and further comprises a metal, metal salt or organic alkali metal complex, preferably alkali metal complex, more preferably LiQ or alkali borate.

According to one embodiment the organic semiconductor layer may comprises at least one compound of formula 1 and further comprises a metal, metal salt or organic metal complex, preferably an organic monovalent or divalent metal complex, more preferably LiQ or alkali borate.

According to one embodiment the organic semiconductor layer may comprises at least one compound of formula 1 and LiQ.

According to one embodiment the organic semiconductor layer may comprises at least one compound of formula 1 and alkali borate.

According to one embodiment, wherein at least one organic semiconductor layer is arranged between the emission layer and the cathode, preferably between the electron injection layer and the cathode.

In another embodiment, the organic semiconductor layer is a first electron transport layer and it is arranged between the emission layer and the second electron transport layer.

According to one embodiment, the organic semiconductor layer is arranged between the first and second emission layer. The organic semiconductor layer can be an electron transport layer, an emission layer, a hole blocking layer, a charge generation layer and/or an electron injection layer, preferably an electron transport layer or a charge

generation layer, and more preferred an electron transport layer.

According to one embodiment, the organic semiconductor layer can be arranged between a photoactive layer and a cathode layer, preferably between an emission layer or light-absorbing layer and the cathode layer, preferably the organic semiconductor layer is an electron transport layer.

According to one embodiment, the organic semiconductor layer may comprise at least one alkali halide or alkali organic complex.

An organic semiconductor layer comprises a compound according to formula 1, 1a or 1b is essentially non-emissive or non-emitting.

Organic electronic device

An organic electronic device according to the invention comprises at least one organic semiconductor layer, wherein at least one organic semiconductor layer comprises a compound according to formula 1.

An organic electronic device according to one embodiment, which comprises at least one organic semiconductor layer that comprises a compound according to formula 1, wherein this layer is essentially non-emissive or non-emitting.

According to one embodiment, the organic electronic device may comprises at least one organic semiconductor layer comprising compound of formula 1 that is an electron transport layer, an emission layer, a hole blocking layer, a charge generation layer and/or an electron injection layer, preferably an electron transport layer or a charge generation layer, more preferred an electron transport layer.

An organic electronic device according to one embodiment may include a substrate, an anode layer, an organic semiconductor layer comprising compound of formula 1, and a cathode layer.

The organic electronic device according to according to one embodiment may comprises at least one organic semiconductor layer, wherein the organic semiconductor layer comprising compound of formula 1 is arranged between a photoactive layer and a cathode layer, preferably between an emission layer or light-absorbing layer and the cathode layer, preferably the organic semiconductor layer is an electron transport layer

The organic electronic device according to one embodiment may comprises at least one organic semiconductor layer comprising compound of formula 1, wherein the at least one organic semiconductor layer further comprises at least one alkali halide or alkali organic complex.

An organic electronic device according to one embodiment comprises at least one organic semiconductor layer comprising at least one compound of formula 1, at least one anode layer, at least one cathode layer and at least one emission layer, wherein the organic semiconductor layer comprising at least one compound of formula 1 is preferably arranged between the emission layer and the cathode layer.

An organic electronic device according to one embodiment comprises at least one organic semiconductor layer comprising at least one compound of formula 1 and further comprises at least one alkali halide or alkali organic complex.

An organic electronic device according to one embodiment comprises at least one organic semiconductor layer, at least one anode layer, at least one cathode layer and at least one emission layer, wherein the organic semiconductor layer comprising at least one compound of formula 1 is preferably arranged between the emission layer and the cathode layer. Preferably the at least one organic semiconductor layer is an electron transport layer.

An organic light-emitting diode (OLED) according to the invention may include an anode, a hole transport layer (HTL), an emission layer (EML), an electron transport layer (ETL) comprising at least one compound of formula 1, and a cathode, which are sequentially stacked on a substrate. In this regard, the HTL, the EML, and the ETL are thin films formed from organic compounds.

An organic electronic device according to one embodiment can be a light emitting device, thin film transistor, a battery, a display device or a photovoltaic cell, and preferably a light emitting device. A light emitting device can be an OLED.

According to one embodiment the OLED may have the following layer structure, wherein the layers having the following order:

an anode layer, a hole injection layer, optional a first hole transport layer, optional a second hole transport layer, an emission layer, an electron transport layer comprising compound of formula 1 according to the invention, an electron injection layer, and a cathode layer.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing may be selected from spincoating, printing, casting; and/or
- slot-die coating.

According to various embodiments of the present invention, there is provided a method using:

- a first deposition source to release the compound of formula 1 according to the invention, and
- a second deposition source to release the alkali halide or alkali organic complex, preferably a lithium halide or lithium organic complex;

the method comprising the steps of forming the electron transport layer stack; whereby for an organic light-emitting diode (OLED):

- the first electron transport layer is formed by releasing the compound of formula 1 according to the invention from the first deposition source and the alkali halide or alkali organic complex, preferably a lithium halide or lithium organic complex from the second deposition source.

According to various embodiments of the present invention, the method may further include forming on the anode electrode an emission layer and at least one layer selected from the group comprising of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, preferably a first electron transport layer is formed on the emission layer and a second electron transport layer is formed on the first electron transport layer and the second electron transport layer comprises a compound of formula 1,
- and finally a cathode electrode is formed,
- optional a hole injection layer, a hole transport layer, and a hole blocking layer, formed in that order between the first anode electrode and the emission layer,
- optional an electron injection layer is formed between the electron transport layer stack and the cathode electrode.

According to various embodiments of the present invention, the method may further include forming an electron injection layer on a first electron transport layer. However, according to various embodiments of the OLED of the present invention, the OLED may not comprise an electron injection layer.

According to various embodiments, the OLED may have the following layer structure, wherein the layers having the following order:
an anode, first hole transport layer, second hole transport layer, emission layer, optional second electron transport layer, first electron transport layer comprising compound of formula 1 according to the invention, optional a second electron transport layer, optional an electron injection layer, and a cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

Preparation of compounds of formula 1

Compounds of formula 1 may be prepared as described in WO2016171358, WO2017135510 and below.

Compounds of formula (1), wherein m is 1, 2 or 3, may be synthesized using the following compound as starting material:

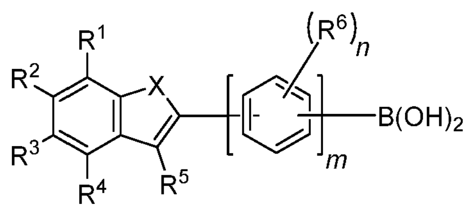

Preparation of 2-(benzo[b]thiophen-2-yl)-4-chloro-6-phenyl-1,3,5-triazine

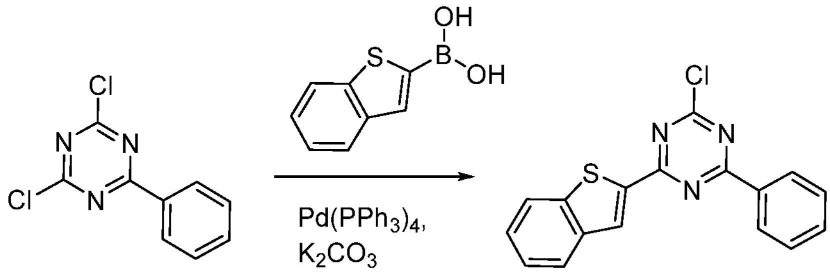


A flask was flushed with nitrogen and charged with 2,4-dichloro-6-phenyl-1,3,5-triazine (79 g, 351 mmol), benzo[b]thiophen-2-ylboronic acid (50 g, 281 mmol), $Pd(PPh_3)_4$ (20 g, 17.6 mmol), and $K_2CO_3$ (121 g, 877 mmol). A mixture of deaerated toluene/THF/water (1:1:1, 960 mL) was added and the reaction mixture was heated to 40 °C under a nitrogen atmosphere for 4 h. After cooling down to room temperature, the formed precipitate was collected by suction filtration and washed with water and toluene. The obtained solid was dissolved in hot chloroform and filtered through a pad of Florisil. After rinsing with additional chloroform, the filtrate was concentrated under reduced pressure and n-hexane was added. The obtained precipitate was collected by suction filtration and washed with n-hexane. After drying, 64 g (70%) of compound was obtained.

Compounds of formula (1), wherein m is 1, 2 or 3, may be synthesized as described above, but using the following compound as starting material:

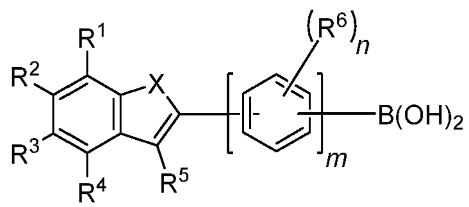

General procedure 1 for the preparation of compound of formula 1

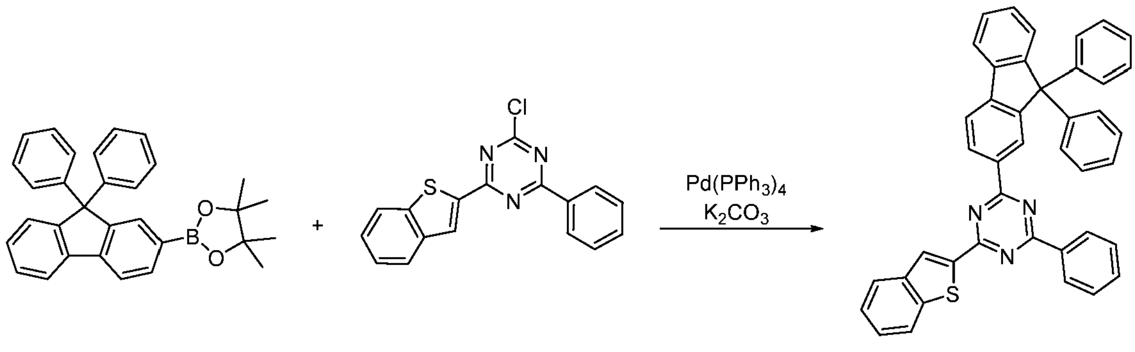


G1

A flask was flushed with nitrogen and charged with 2-(benzo[b]thiophen-2-yl)-4-chloro-6-phenyl-1,3,5-triazine (15.5 g, 48.1 mmol), 2-(9,9-diphenyl-9H-fluoren-2-yl)-4,4,5,5-tetramethyl-1,3,2-dioxaborolane (20.5 g, 80.6 mmol), Pd(dppf)$Cl_2$ (1 g, 1.4 mmol), and $K_2CO_3$ (16.6 g, 120 mmol). A mixture of deaerated THF/water (4:1, 250 mL) was added and the reaction mixture was heated to reflux under a nitrogen atmosphere for 24 h. After cooling down to room temperature, the formed precipitate was collected by suction filtration and washed with THF and water. The obtained solid was dissolved in dichloromethane and filtered through a pad of Florisil. After rinsing with additional dichloromethane, the filtrate was evaporated to dryness and toluene was added. The obtained suspension was diluted with n-hexane and the precipitate was collected by suction filtration and washed with n-hexane. After drying, 17.9 g (60%) of compound were obtained. HPLC/ESI-MS: 100%, m/z = 606 ($[M+H]^+$).

Compound 2-(benzo[b]thiophen-2-yl)-4-phenyl-6-(3-(9-phenyl-9H-fluoren-9-yl)phenyl)-1,3,5-triazine (G45) may be prepared as shown in scheme above by the reaction of 2,4-dichloro-6-phenyl-1,3,5-triazine with 2-(9,9-diphenyl-9H-fluoren-2-yl)-4,4,5,5-tetramethyl-1,3,2-dioxaborolane.

Preparation of 2-chloro-4-phenyl-6-(3',4',5'-triphenyl-[1,1':2',1"-terphenyl]-4-yl)-1,3,5-triazine

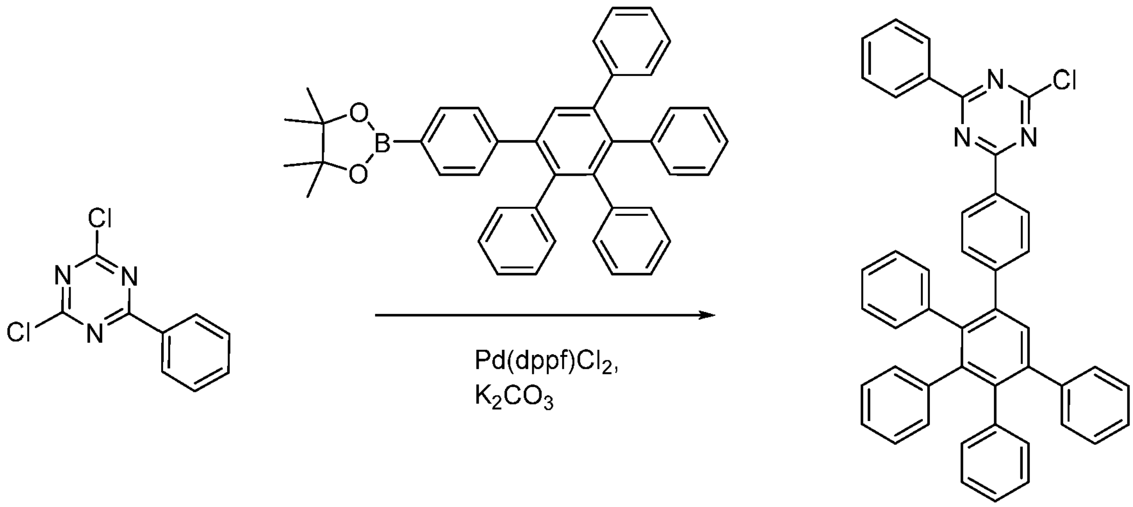


A flask was flushed with nitrogen and charged with 2,4-dichloro-6-phenyl-1,3,5-triazine (29 g, 128 mmol), 4,4,5,5-tetramethyl-2-(3',4',5'-triphenyl-[1,1':2',1"-terphenyl]-4-yl)-1,3,2-dioxaborolane (60 g, 103 mmol), Pd(dppf)$Cl_2$ (4.7 g, 6.4 mmol), and $K_2CO_3$ (44 g, 320 mmol). A mixture of deaerated toluene/THF/water (1:1:1, 1050 mL) was added and the reaction mixture was heated to 65 °C under a nitrogen atmosphere for 22 h. After cooling down to room temperature, the solvent is evaporated and the solid was dissolved in chloroform and water. The organic phase is washed with water four times, dried over $MgSO_4$ and filtered through a pad of silica. The filtrate was concentrated under reduced pressure and n-hexane was added. Further purification was achieved by column chromatography (silica, toluene/n-hexane 3:1) and sublimation to afford 8 g (11%) of compound were obtained.

General procedure 2 for the preparation of compound of formula 1

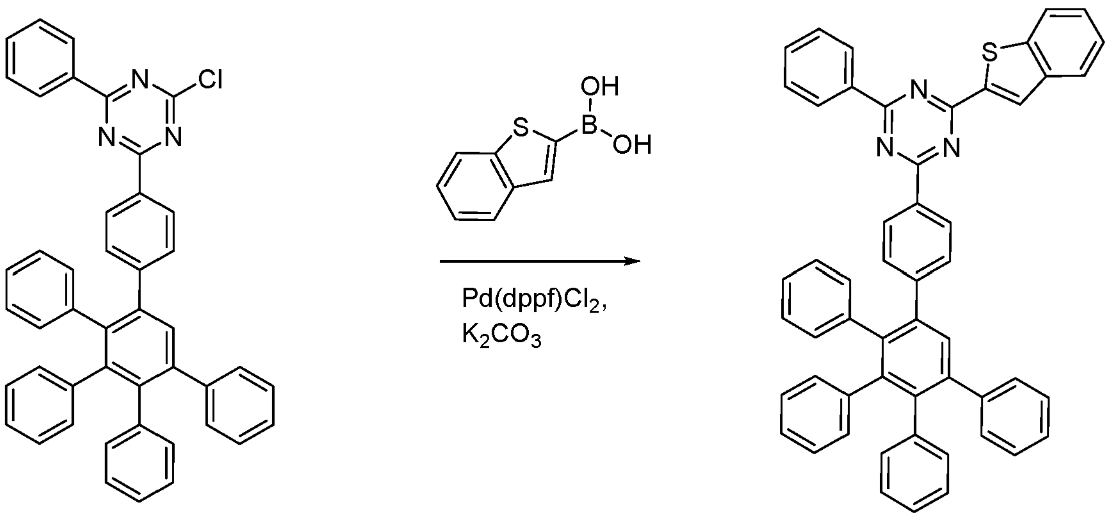


G29

A flask was flushed with nitrogen and charged with 2-chloro-4-phenyl-6-(3',4',5'-triphenyl-[1,1':2',1"-terphenyl]-4-yl)-1,3,5-triazine (8 g, 12.3 mmol), benzo[b]thiophen-2-ylboronic acid (2.6 g, 14.8 mmol), Pd(dppf)$Cl_2$ (0.18 g, 0.25 mmol), and $K_2CO_3$ (3.4 g, 25 mmol). A mixture of deaerated THF/water (4:1, 200 mL) was added and the reaction mixture was heated to reflux under a nitrogen atmosphere for 70 h. After cooling down to room temperature, the formed precipitate was collected by suction filtration, dissolved in chloroform and washed with water three times. After drying over $MgSO_4$, the organic phase was filtered through a pad of silica and the filtrate was evaporated to dryness. The obtained solid was triturated with dichloromethane/n-hexane. Subsequently, the crude product was dissolved in chloroform and filtered through a pad of silica and Florisil. After rinsing with additional chloroform, the filtrate was evaporated to afford 4.2 g (45%) of compound were obtained. HPLC/ESI-MS: 100%, m/z = 746 ($[M+H]^+$).

Preparation of 2-(benzo[b]thiophen-2-yl)-4-(3-chlorophenyl)-6-phenyl-1,3,5-triazine

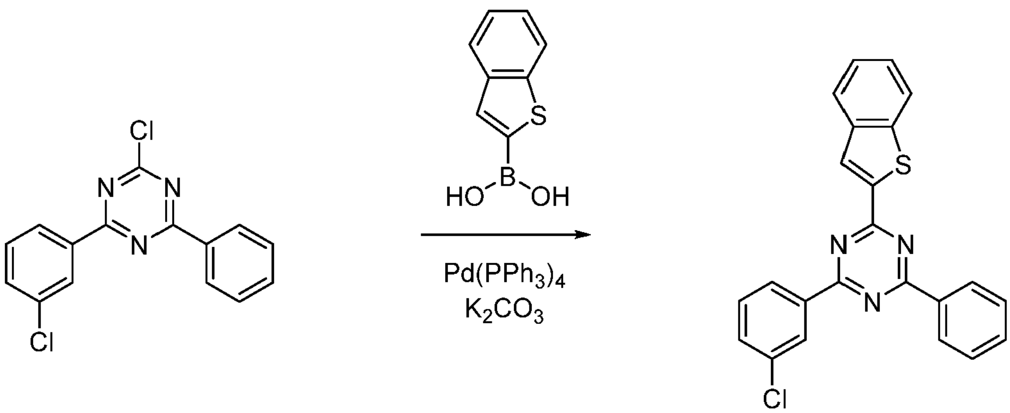


A flask was flushed with nitrogen and charged with 2-chloro-4-(3-chlorophenyl)-6-phenyl-1,3,5-triazine (50 g, 165.5 mmol), benzo[b]thiophen-2-ylboronic acid (30.9 g, 173.8 mmol), Pd$(PPh_3)_4$ (5.7 g, 4.9 mmol), and $K_2CO_3$ (68.5 g, 496 mmol). A mixture of deaerated THF/water (4:1, 1.25 L) was added and the reaction mixture was heated to 70 °C under a nitrogen atmosphere for two days. After cooling down to room temperature, the solvent is evaporated and the solid was washed with water and dried. The crude product was dissolved in hot chlorobenzene and filtered through a plug of silica gel. The organic phase is concentrated and the obtained residue was triturated with methanol. After drying, 59.5 g (90%) of compound were obtained.

General procedure 3 for the preparation of compound of formula 1

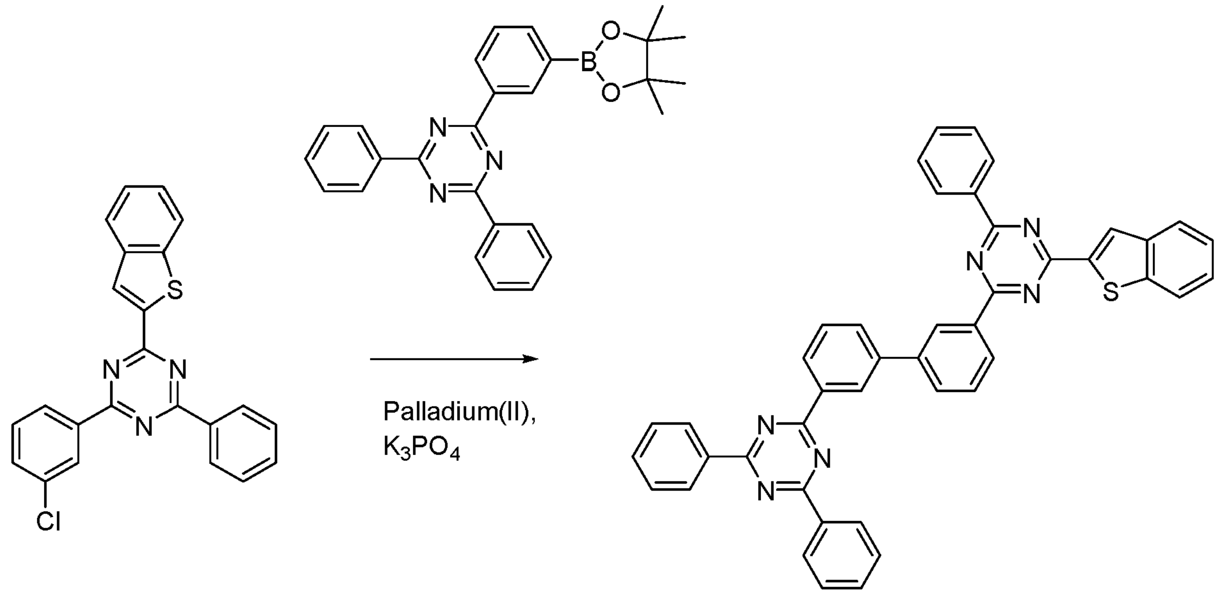


A flask was flushed with nitrogen and charged with 2-(benzo[b]thiophen-2-yl)-4-(3-chlorophenyl)-6-phenyl-1,3,5-triazine (10 g, 25 mmol), 2,4-diphenyl-6-(3-(4,4,5,5-tetramethyl-1,3,2-dioxaborolan-2-yl)phenyl)-1,3,5-triazine (13.1 g, 30 mmol), chloro(crotyl)(2-dicyclohexylphosphino-2',6'-dimethoxybiphenyl)palladium(II) (0.3 g, 0.5 mmol), and $K_3PO_4$ (10.6 g, 50 mmol). A mixture of deaerated THF/water (10:1, 385 mL) was added and the reaction mixture was heated to 45 °C under a nitrogen atmosphere for 18 h. After cooling down to room temperature, the formed precipitate was collected by suction filtration and washed with THF, water and hexane. The crude product was dissolved in hot chlorobenzene and filtered through a pad of silica gel. After rinsing with additional hot chlorobenzene, the combined filtrates were concentrated in vacuo and the obtained precipitate was isolated by suction filtration and washed with n-hexane and ethanol. The filtration step with hot chlorobenzene is repeated one more time. The obtained solid was then recrystallized from THF and dried to afford 6.6 g (39%) of compound were obtained. HPLC/ESI-MS: 100%, m/z = 673 ($[M+H]^+$).

Preparation of 2-(benzo[b]thiophen-2-yl)-4-phenyl-6-(4-(4,4,5,5-tetramethyl-1,3,2-dioxaborolan-2-yl)phenyl)-1,3,5-triazine

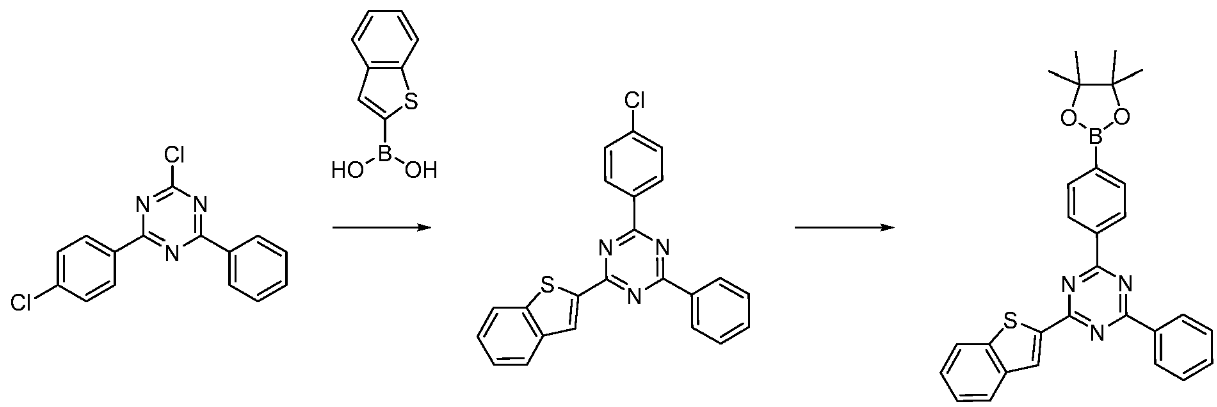

A flask was flushed with nitrogen and charged with 2-(Benzo- [b]thiophen-2-yl)-4-(4-chlorophenyl)-6-phenyl-1,3,5-triazine (20 g, 50 mmol), 4,4,4',4',5,5,5',5'-octamethyl-2,2'-bi(1,3,2-dioxaborolane) (15.2 g, 60 mmol), $Pd_2(dba)_3$ (1.14 g, 1.25 mmol), XPhos (1.2 g, 2.5 mmol), and potassium acetate (14.7 g, 150 mmol). Dry and deaerated 1,4-dioxane

(300 mL) was added and the reaction mixture was heated to reflux under a nitrogen atmosphere for 18 h. Subsequently, all volatiles have been removed under reduced pressure and the residue was dissolved in chloroform and water. The organic phase was washed with water five times, dried over $MgSO_4$ and filtered through a pad of silica. The filtrate was concentrated to a minimal amount under reduced pressure and n-hexane was added. The resulting precipitate was collected by suction filtration and dried to yield 22.3 g (91%) of compound were obtained.

General procedure 4 for the preparation of compound of formula 1

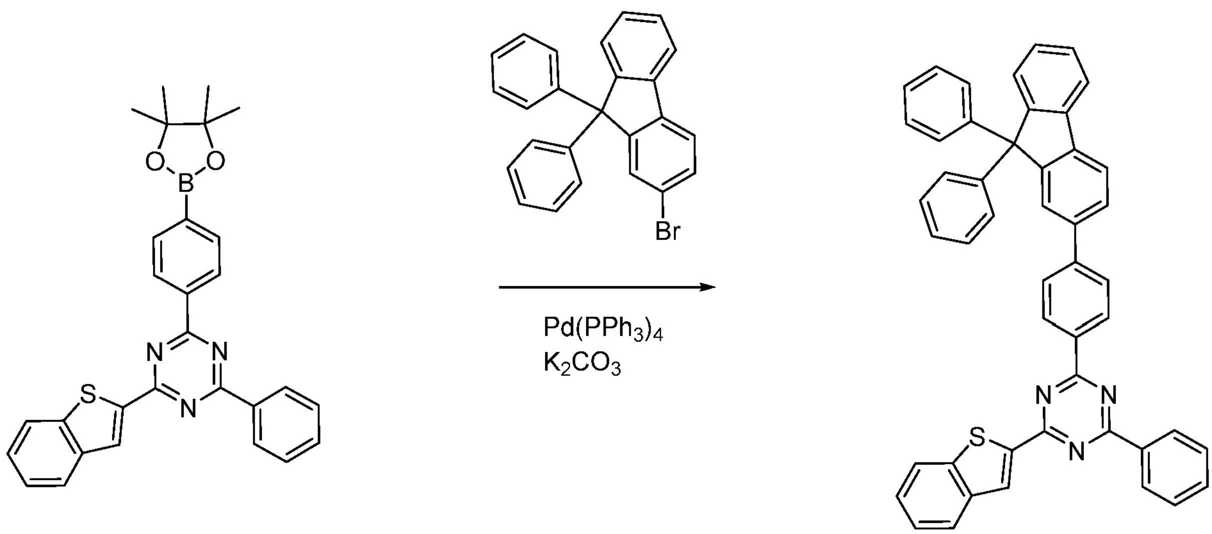


G11

A flask was flushed with nitrogen and charged with 2-(benzo[b]thiophen-2-yl)-4-phenyl-6-(4-(4,4,5,5-tetramethyl-1,3,2-dioxaborolan-2-yl)phenyl)-1,3,5-triazine (11 g, 22.4 mmol), 2-bromo-9,9-diphenyl-9H-fluorene (9.8 g, 24.6 mmol), $Pd(PPh_3)_4$ (0.52 g, 0.45 mmol), and $K_2CO_3$ (6.2 g, 45 mmol). A mixture of deaerated 1,4-dioxane/water (5:1, 155 mL) was added and the reaction mixture was heated to 90 °C under a nitrogen atmosphere for 70 h. After cooling down to room temperature, the formed precipitate was collected by suction filtration and washed with water. The obtained solid was dissolved in chloroform and washed with water four times. After drying over $MgSO_4$, the organic phase was filtered through a pad of silica. The filtrate was concentrated to a minimal amount under reduced pressure and n-hexane was added. The resulting precipitate was collected by suction filtration and recrystallized from toluene to yield 9.9 g (65%) of compound were obtained. HPLC/ESI-MS: 99.9%, m/z = 682 ([M+H]$^+$).

General procedure 5 for the preparation of compound of formula 1

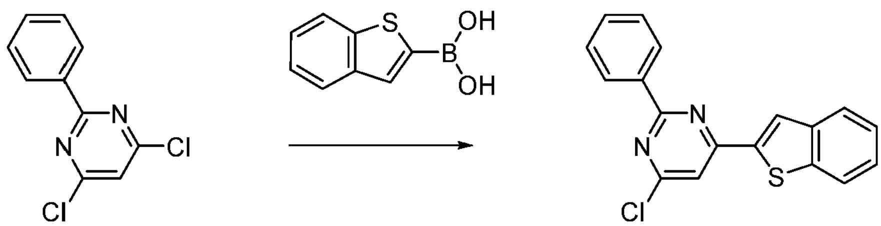

Preparation of 4-(benzo[b]thiophen-2-yl)-6-chloro-2-phenylpyrimidine

A flask was flushed with nitrogen and charged with 4,6-dichloro-2-phenylpyrimidine (30.0 g, 133.3 mmol), benzo[b]thiophen-2-ylboronic acid (19.0 g, 106.6 mmol), potassium carbonate (46.0 g, 333.2 mmol) and tetrakis(triphenylphosphin)palladium(0) (7.7 g, 6.7 mmol). A mixture of deaerated THF/toluene/water (1:1:1, 540 mL) was added and the reaction mixture was heated to 65 °C under a nitrogen atmosphere for 4 hours. After cooling down to room temperature, solvents were removed under reduced pressure. The residue was dissolved in dichloromethane (300 mL) and washed with water (3x100 mL). Aqueous phase was extracted with dichloromethane and the combined organic phases were first dried over MgSO4 and then filtered over a pad of silica gel. After rinsing with additional dichloromethane (1.5 L), the filtrate was concentrated under reduced pressure to a minimal volume and n-hexane (100 mL) was added. The resulting suspension was stirred at room temperature and the precipitate was collected by suction filtration and triturated in cyclohexane (600 mL) to yield 20.2 g (58 %) of 4-(benzo[b]thiophen-2-yl)-6-chloro-2-phenylpyrimidine that was used in the next step without additional purification.

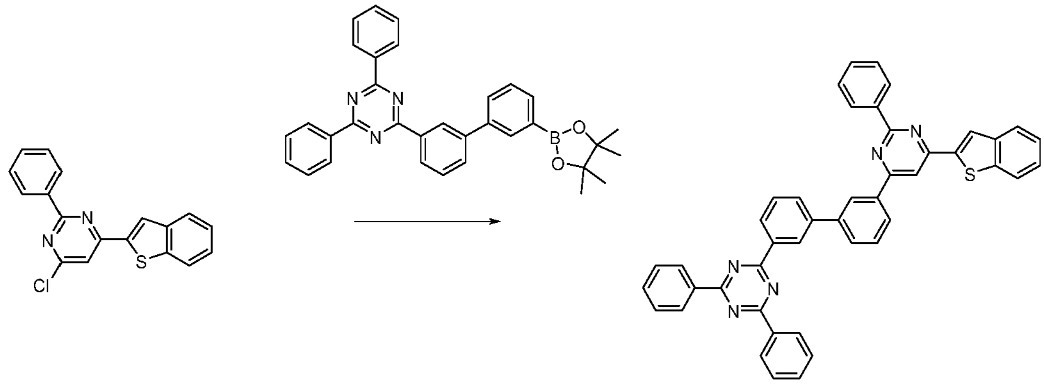

Preparation of 2-(3'-(6-(benzo[b]thiophen-2-yl)-2-phenylpyrimidin-4-yl)-[1,1'-biphenyl]-3-yl)-4,6-diphenyl-1,3,5-triazine

A flask was flushed with nitrogen and charged with 4-(benzo[b]thiophen-2-yl)-6-chloro-2-phenylpyrimidine (10.0 g, 28.3 mmol), 2,4-diphenyl-6-(3'-(4,4,5,5-tetramethyl-1,3,2-dioxaborolan-2-yl)-[1,1'-biphenyl]-3-yl)-1,3,5-triazine (14.6 g, 28.3 mmol), potassium carbonate (7.8 g, 56.6 mmol), and [1,1'-Bis(diphenylphosphino)ferrocene]dichloropalladium(II) (414 mg, 0.57 mmol). A mixture of deaerated THF/water (4:1, 140 mL) was added and the reaction mixture was heated to 65 °C under a nitrogen atmosphere overnight. After cooling down to room temperature, solvents were removed under reduced pressure. The residue was dissolved in dichloromethane (300 mL) and washed with water (3x150 mL). Aqueous phase was extracted with dichloromethane and the combined organic phases were first dried over MgSO4 and then solvents were removed under reduced pressure. Solid residue was dissolved in chlorobenzene (300 mL) and filtered over a pad of silica gel. After rinsing with additional chlorobenzene (2.0 L), the filtrate was concentrated and the suspension was left stirring overnight at room temperature. The precipitate was collected by suction filtration and recrystallized in chlorobenzene (3 x 150-200 mL) to yield 13.0 g (68 %) of 2-(3'-(6-(benzo[b]thiophen-2-yl)-2-phenylpyrimidin-4-yl)-[1,1'-biphenyl]-3-yl)-4,6-diphenyl-1,3,5-triazine. Final purification was achieved by sublimation. HPLC: 99.8%, HPLC/ESI-MS: m/z = 672 ([M+H]+).

The chemical structure, molar mass, calculated HOMO, LUMO, dipole moment, melting point, glass transition temperature and rate onset temperature of compounds of formula 1 of examples 1 to 12 and comparative examples 1 of C1 are shown in Table 1.

Table 1

| Properties of compound of comparative examples 1 and compounds of formula 1 of example 1 to example 12 | | | | | | | |
|---|---|---|---|---|---|---|---|
| Referred to as: | Structure | mp (°C) | Tg (°C) | $T_{RO}$ (°C) | LU MO (eV) | HO MO (eV) | Diplol e mom ent (Deby e) |
| C1 | 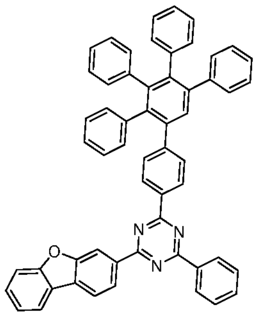 | 300 | 166 | 266 | -1.92 | -5.83 | 1.18 |
| Example 1 G29 | 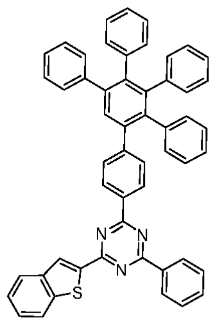 | 301 | 163 | 251 | -2.00 | -5.84 | 0.34 |
| Example 2 G1 | 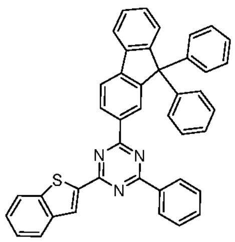 | 266 | 138 | 220 | -2.02 | -5.85 | 0.17 |
| Example 3 G45 | 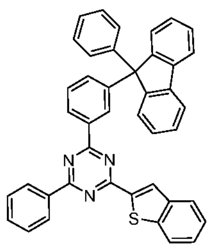 | -- | 117 | 230 | -1.99 | -5.84 | 1.06 |
| Example 4 G11 | 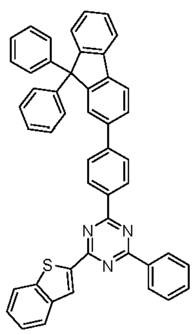 | 305 | 155 | 258 | -2.04 | -5.65 | 0.17 |

(continued)

| Properties of compound of comparative examples 1 and compounds of formula 1 of example 1 to example 12 | | | | | | | |
|---|---|---|---|---|---|---|---|
| Referred to as: | Structure | mp (°C) | Tg (°C) | $T_{RO}$ (°C) | LU MO (eV) | HO MO (eV) | Diplol e mom ent (Deby e) |
| Example 5 G62 | 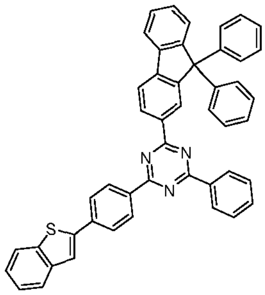 | 289 | 148 | 256 | -2.05 | -5.69 | 0.20 |
| Example 6 G5 | 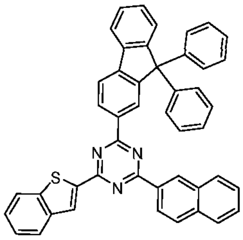 | 325 | 148 | 255 | -2.04 | -5.83 | 0.51 |
| Example 7 G41 | 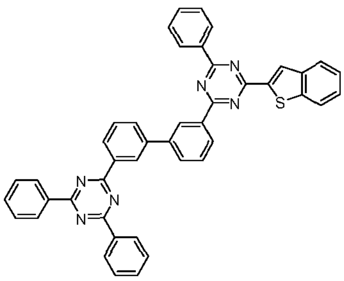 | 252 | 119 | 270 | -2.07 | -6.00 | 0.71 |
| Example 8 G63 | 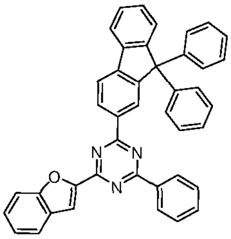 | -- | 135 | 206 | -1.99 | -5.84 | 0.25 |
| Example 9 G49 | 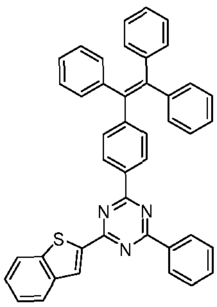 | 321 | 159 | 265 | -2.06 | -5.15 | 0.94 |

(continued)

| Properties of compound of comparative examples 1 and compounds of formula 1 of example 1 to example 12 | | | | | | | |
|---|---|---|---|---|---|---|---|
| Referred to as: | Structure | mp (°C) | Tg (°C) | $T_{RO}$ (°C) | LU MO (eV) | HO MO (eV) | Diplol e mom ent (Deby e) |
| Example 10 G37 | 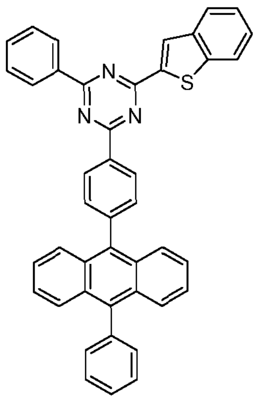 | 320 | 159 | 265 | -2.06 | -5.15 | 0.94 |
| Example 11 G64 | 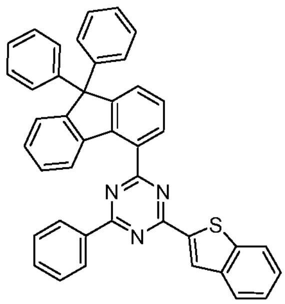 | 275 | 136 | 212 | -2.05 | -5.84 | 0.75 |
| Example 12 G65 | 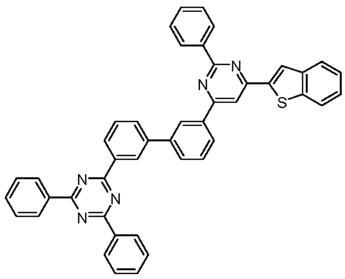 | -- | -- | -- | -1.96 | -5.99 | 1.45 |

General procedure for fabrication of OLEDs

For top emission devices, Example 1 to 4 of compounds G29, G1, G45 and G11 and comparative examples C1, a glass substrate was cut to a size of 50 mm x 50 mm x 0.7 mm, ultrasonically cleaned with isopropyl alcohol for 5 minutes and then with pure water for 5 minutes, and cleaned again with UV ozone for 30 minutes. 100 nm Ag were deposited on the glass substrate as anode at a pressure of $10^{-5}$ to $10^{-7}$ mbar to form the anode.

Then, 92 vol.-% Biphenyl-4-yl(9,9-diphenyl-9H-fluoren-2-yl)-[4-(9-phenyl-9H-carbazol-3-yl)phenyl]-amine (CAS 1242056-42-3) with 8 vol.-% 2,2',2"-(cyclopropane-1,2,3-triylidene)tris(2-(p-cyanotetrafluorophenyl)acetonitrile) was vacuum deposited on the anode, to form a hole injection layer (HIL) having a thickness of 10 nm. Then, Biphenyl-4-yl(9,9-diphenyl-9H-fluoren-2-yl)-[4-(9-phenyl-9H-carbazol-3-yl)phenyl]-amine was vacuum deposited on the HIL, to form a hole transport layer (HTL) having a thickness of 118 nm.

Then, N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then 97 vol.-% ABH113 (Sun Fine Chemicals) as EML host and 3 vol.-% NUBD370 (Sun Fine Chemicals) as fluorescent blue dopant were deposited on the EBL, to form a blue-emitting EML with a thickness of 20 nm.

Then, a first electron transport layer (ETL1) is formed on the EML with a thickness of 5 nm by depositing 2,4-diphenyl-6-(4',5',6'-triphenyl-[1,1':2',1":3",1‴:3‴,1""-quinquephenyl]-3""-yl)-1,3,5-triazine on the emission layer with a thickness of 5 nm, see Table 2.

Then, a second electron transport layer (ETL2) is formed on the first electron transport layer with a thickness of 31 nm by depositing 50 vol.-% of a compound selected from G29, G1, G45 and G11 of formula 1 as matrix compound and 50 vol.-% of alkali organic complex LiQ, see Table 2; as well as for the comparative example a second electron transport layer is formed on the first electron transport layer with a thickness of 31 nm by depositing 50 vol.-% of a compound the comparative examples C1 as matrix compound and 50 vol.-% of alkali organic complex LiQ, see Table 2.

Table 2

Performance of an organic electroluminescent device comprising a first electron transport layer and a second electron transport layer comprising a compound of formula 1 as well as a compound of C1.

| | Matrix compound in ETL2 | Concentration of matrix compound in ETL2 (vol.-%) | Alkali organic complex | Concentration of alkali organic complex (vol.-%) | Thickness electron transport layer (nm) | Operating voltage at 10 $mA/cm^2$ (V) | cd/A efficiency at 10 $mA/cm^2$ (cd/A) | LT97 at 30 $mA/cm^2$ (h) |
|---|---|---|---|---|---|---|---|---|
| Comparative example 1 | C1 | 50 | LiQ | 50 | 31 | 3.1 | 7.7 | 46 |
| Example 1 | G29 | 50 | LiQ | 50 | 31 | 3.7 | 7.4 | 64 |
| Example 2 | G1 | 50 | LiQ | 50 | 31 | 3.6 | 7.1 | 64 |
| Example 3 | G45 | 50 | LiQ | 50 | 31 | 3.8 | 7.5 | 56 |
| Example 4 | G11 | 50 | LiQ | 50 | 31 | 3.6 | 7.5 | 59 |

Then, the electron injection layer is formed on the electron transporting layer by deposing Yb with a thickness of 2 nm.

Ag is evaporated at a rate of 0.01 to 1 A/s at $10^{-7}$ mbar to form a cathode with a thickness of 11 nm.

A cap layer of Biphenyl-4-yl(9,9-diphenyl-9H-fluoren-2-yl)-[4-(9-phenyl-9H-carbazol-3-yl)phenyl]-amine is formed on the cathode with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/$m^2$ using an Instrument Systems CAS-140CT array spectrometer for each of the voltage values.

The cd/A efficiency at 10 mA/$cm^2$ is determined by interpolating the luminance-voltage and current-voltage characteristics, respectively.

Lifetime LT of the device is measured at ambient conditions (20°C) and 30 mA/$cm^2$, using a Keithley 2400 sourcemeter, and recorded in hours.

The brightness of the device is measured using a calibrated photo diode. The lifetime LT is defined as the time till the brightness of the device is reduced to 97 % of its initial value.

Technical Effect of the invention

As can be seen in Table 2, the compounds of formula 1 of inventive example 1 to 4 have low evaporation temperature as compared to the comparative example 1.

The life time of organic electronic devices comprising the compound of formula 1 is improved; see examples 1 to 4, over organic electronic devices comprising the compound C1 known in the art.

While this invention has been described in connection with what is presently considered to be practical exemplary embodiments, it is to be understood that the invention is not limited to the disclosed embodiments. Therefore, the aforementioned embodiments should be understood to be exemplary but not limiting the present invention in any way.

## Claims

1. A compound according to formula 1:

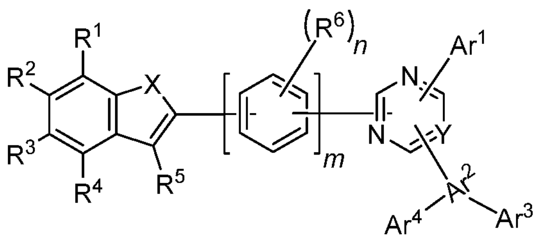 (1),

wherein

alkyl group is defined as a linear, cyclic or branched alkyl group;
X is O or S;
$R^1$, $R^2$, $R^3$, $R^4$, $R^5$, $R^6$ are independently selected from H, substituted or unsubstituted $C_6$ to $C_{38}$ aryl, substituted or unsubstituted $C_3$ to $C_{36}$ heteroaryl or $C_1$ to $C_{16}$ alkyl group, wherein
the substituents are selected from $C_6$ to $C_{18}$ aryl, $C_3$ to $C_{25}$ heteroaryl, -PO(R')$_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;
m is an integer selected from 0 or 1;
n is an integer selected from 0;
Y

is CR" or N, wherein R" is H, substituted or unsubstituted $C_6$ to $C_{38}$ aryl, substituted or unsubstituted $C_3$ to $C_{36}$ heteroaryl, or $C_1$ to $C_{16}$ alkyl group, wherein
the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

$Ar^1$ is selected from substituted or unsubstituted $C_6$ to $C_{38}$ aryl, or substituted or unsubstituted $C_3$ to $C_{36}$ heteroaryl, wherein the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;
$Ar^2$ is selected from substituted or unsubstituted $C_6$ to $C_{38}$ arylene or formula 2,

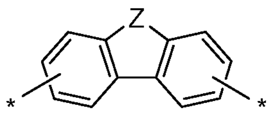

(2),

wherein
the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;
Z is selected from O, S, Se, CR''' or NR''' with R''' being selected from $C_1$ to $C_{16}$ alkyl or $C_6$ to $C_{18}$ aryl;
$Ar^3$ is selected from H, substituted or unsubstituted aryl, substituted or unsubstituted heteroaryl selected from a group comprising of pyridine, pyrimidine, triazine, quinoline, quinoxaline, benzo acridine, dibenzo acridine, phenanthroline, carbazole, dibenzofurane, dibenzothiophene, or

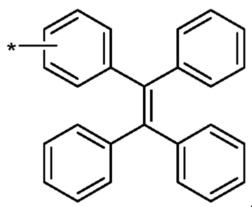

,

wherein
the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;
$Ar^4$ is selected from H, substituted or unsubstituted aryl, substituted or unsubstituted heteroaryl selected from a group consisting of pyridine, pyrimidine, triazine, quinoline, quinoxaline, benzo acridine, dibenzo acridine, phenanthroline, carbazole, dibenzofurane, dibenzothiophene, wherein
the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

and wherein $Ar^4$ is H when $Ar^2$ is selected formula 2.

**2.** The compound of formula 1 according to claim 1, wherein

X is O;
$R^1$, $R^2$, $R^3$, $R^4$, $R^5$, $R^6$ are independently selected from H, substituted or unsubstituted $C_6$ to $C_{38}$ aryl, substituted or unsubstituted $C_3$ to $C_{36}$ heteroaryl or $C_1$ to $C_{16}$ alkyl group, wherein

the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

m is an integer selected from 0 or 1;

n is an integer selected from 0;

Y is N or CH;

$Ar^1$ is selected from substituted or unsubstituted $C_6$ to $C_{38}$ aryl, or substituted or unsubstituted $C_3$ to $C_{36}$ heteroaryl, wherein

the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

$Ar^2$ is selected from substituted or unsubstituted $C_6$ to $C_{38}$ arylene or formula 2,

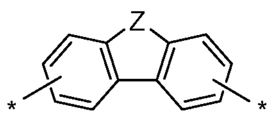

(2)
(1),

wherein

the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

Z is selected from O, S, Se, CR''' or NR''' with R''' being selected from $C_1$ to $C_{16}$ alkyl or $C_6$ to $C_{18}$ aryl;

$Ar^3$ is selected from H, substituted or unsubstituted aryl, substituted or unsubstituted heteroaryl selected from a group comprising of pyridine, pyrimidine, triazine, quinoline, quinoxaline, benzo acridine, dibenzo acridine, phenanthroline, carbazole, dibenzofurane dibenzothiophene, wherein

the substituents are selected from $C_6$ to $C_{18}$ aryl, $C_3$ to $C_{25}$ heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy;

$Ar^4$ is selected from H, substituted or unsubstituted aryl, substituted or unsubstituted heteroaryl selected from a group consisting of pyridine, pyrimidine, triazine, quinoline, quinoxaline, benzo acridine, dibenzo acridine, phenanthroline, carbazole, dibenzofurane, dibenzothiophene, wherein t

he substituents selected from $C_6$ to $C_{18}$ $C_3$ to $C_{25}$ are aryl, heteroaryl, $-PO(R')_2$ with R' being alkyl, aryl or heteroaryl, D, F or CN, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, partially or perfluorinated $C_1$ to $C_{16}$ alkoxy, partially or perdeuterated $C_1$ to $C_{16}$ alkoxy.

**3.** The compound of formula 1 according to claim 1 or 2, wherein the compound of formula 1 is represented by formula 1a and/or 1b:

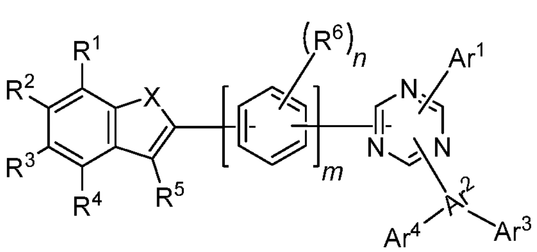

(1a);

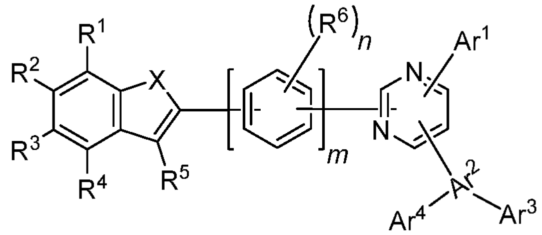

(1b).

4. The compound of formula 1 according to claims 1 to 3, wherein

X is O or S;
$R^1$, $R^2$, $R^3$, $R^4$, $R^5$, $R^6$ are independently selected from H, phenyl, naphthyl, or biphenyl;
m is 0;
n is 0;
Y is N or CH, preferably N;
$Ar^1$ is selected from phenyl, naphthyl, biphenyl, phenanthryl triphenyl, or anthryl group;
$Ar^2$ is selected from divalent phenylene, naphthylene, biphenylene, phenanthrylene, triphenylene, or anthrylene group;
Z is selected from O, S, Se, CR‴ or NR‴ with R‴ being selected from $C_1$ to $C_{16}$ alkyl or $C_6$ to $C_{18}$ aryl;
$Ar^3$ is selected from H, substituted or unsubstituted aryl, substituted or unsubstituted heteroaryl selected from a group consisting of pyridine, pyrimidine, triazine, quinoline, quinoxaline, benzo acridine, dibenzo acridine, phenanthroline, carbazole, dibenzofurane, dibenzothiophene;
$Ar^4$ is selected from H, substituted or unsubstituted aryl, substituted or unsubstituted heteroaryl selected from a group consisting of pyridine, quinoline, quinoxaline, phenanthroline, carbazole, dibenzofurane, dibenzothiophene.

5. The compound of formula 1 according to claims 1 to 4, wherein

$Ar^2$ is selected from formula 2; and
Z is selected from O, S, Se, CR‴ or NR‴ with R‴ being selected from $C_1$ to $C_{16}$ alkyl or $C_6$ to $C_{18}$ aryl, and more preferred CR‴ with R‴ being selected from $C_1$ to $C_{16}$ alkyl or $C_6$ to $C_{18}$ aryl.

6. The compound of formula 1 according to any of claims 1 to 4, wherein the compound of formula 1 comprises at least one non-hetero phenyl rings to twelve non-hetero phenyl rings, preferably of at least two phenyl rings to seven phenyl rings

7. The compound of formula 1 according to any of claims 1 to 6, wherein the compound of formula 1 comprises at least one to four, preferably one to three, further preferred one or two and more preferred one hetero aromatic 6-member ring, and at least one to two, preferably one hetero aromatic 5-member ring.

8. The compound of formula 1 according to any of claims 1 to 7, wherein $Ar^1$ and $Ar^3$ and/or $Ar^4$ are independently selected from substituted or unsubstituted $C_6$ to $C_{18}$ aryl, or substituted or unsubstituted $C_5$ to $C_{18}$ heteroaryl, and $Ar^2$ are independently selected from substituted or unsubstituted $C_6$ to $C_{18}$ arylene, or substituted or unsubstituted $C_5$ to $C_{18}$ heteroarylene, wherein
the substituents of the substituted $C_6$ to $C_{18}$ aryl, substituted $C_5$ to $C_{18}$ heteroaryl, substituted $C_6$ to $C_{18}$ arylene or substituted $C_5$ to Cis heteroarylene are independently selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl.

9. The compound of formula 1 according to any of claims 1 to 8, wherein

- $Ar^1$ is selected from phenyl, biphenyl, terphenyl, naphthyl, phenanthryl, triphenyl, anthracenyl, and preferably from C 1 to C9:

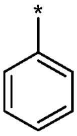 (C1), 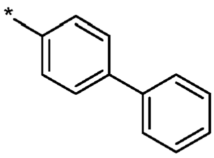 (C2), 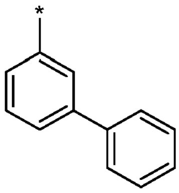 (C3), 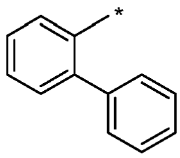 (C4),

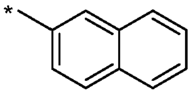 (C5), 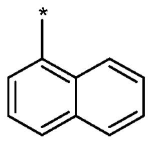 (C6), 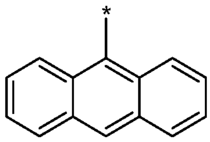 (C7), 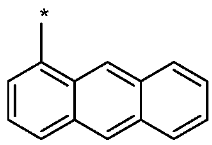 (C8),

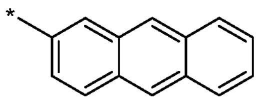 (C9);

and/or

- $Ar^2$ is be selected from phenylene, biphenylene, terphenylene, naphthylene, phenanthrylene, triphenylene, anthracenylene, and preferably from D1 to D13:

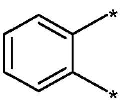 (D1), 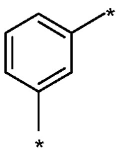 (D2), 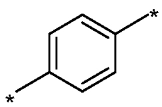 (D3), 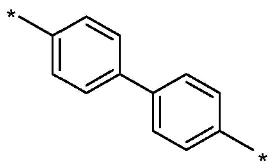 (D4),

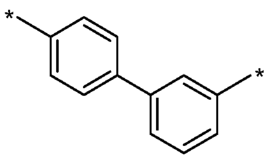 (D5), 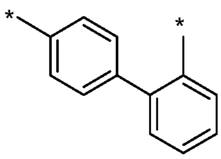 (D6), 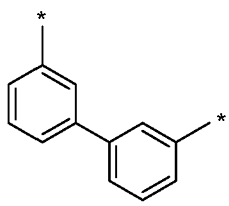 (D7),

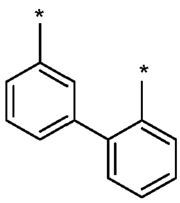 (D8), 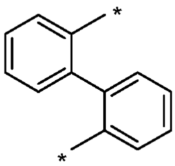 (D9), 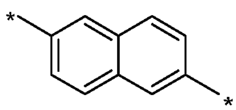 (D10), 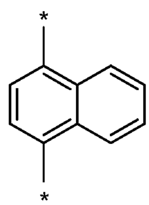 (D11),

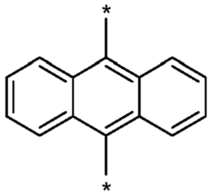 (D12), 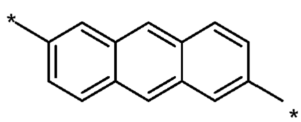 (D13);

- $Ar^3$ is independently selected from E1 to E28

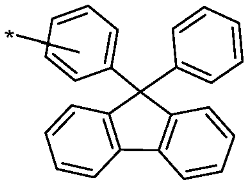 (E1), 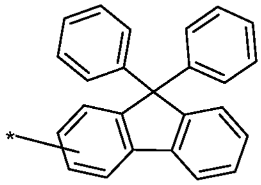 (E2), 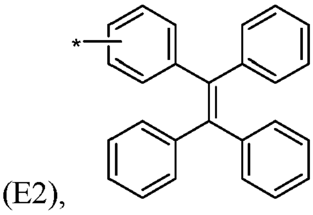 (E3),

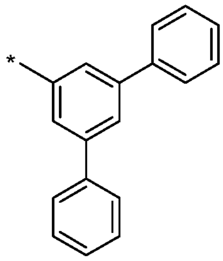 (E4), 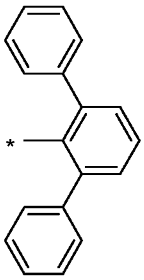 (E5), 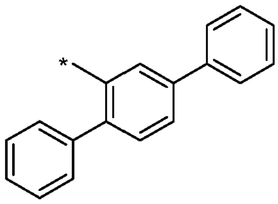 (E6),

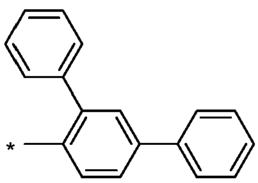 (E7), 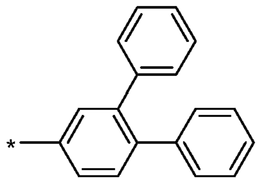 (E8), 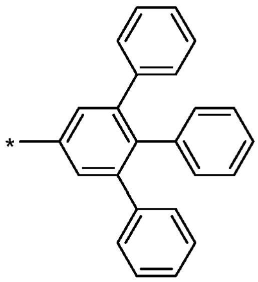 (E9),

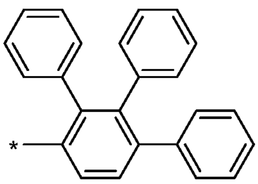 (E10), 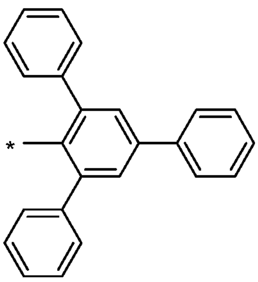 (E11),

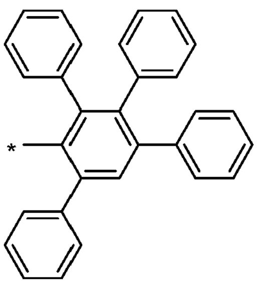 (E12), 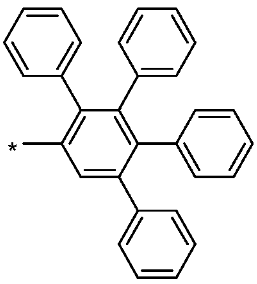 (E13), 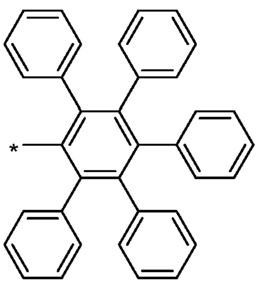 (E14),

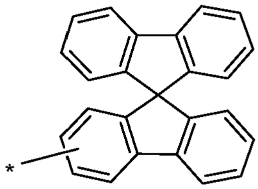 (E15), 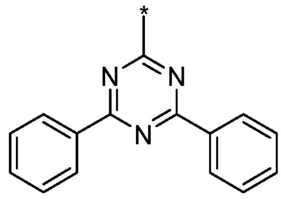 (E16), 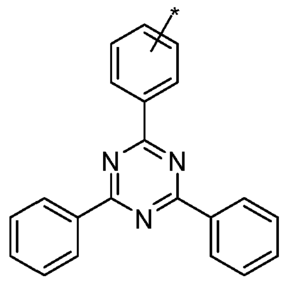 (E17),

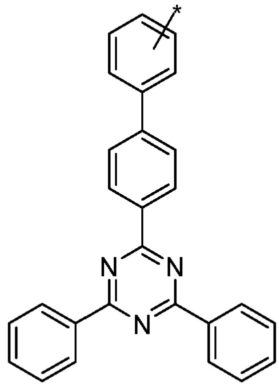 (E18), 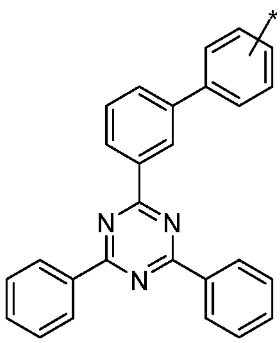 (E19), 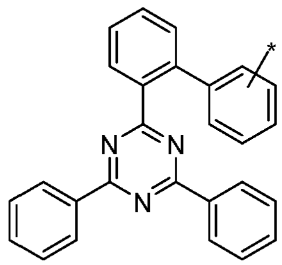 (E20),

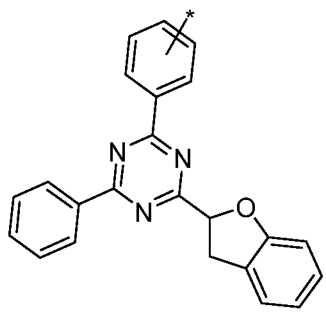 (E21),

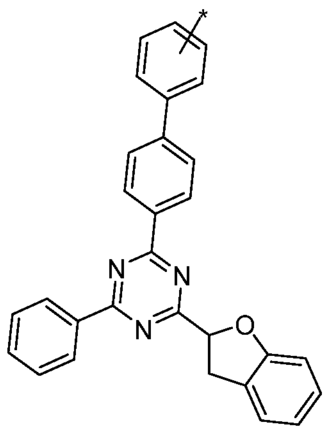 (E22), 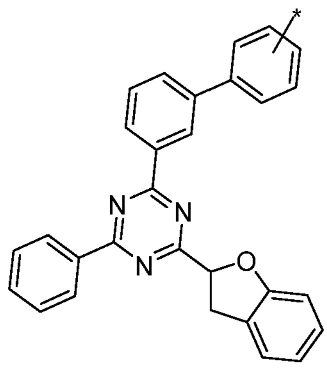 (E23),

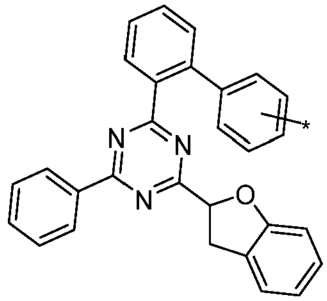 (E24), 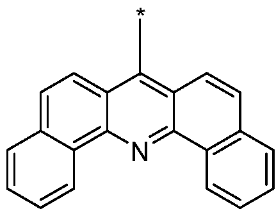 (E25); 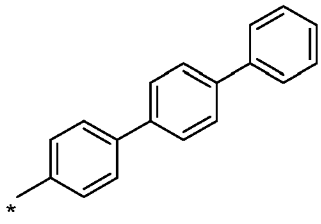 (E26),

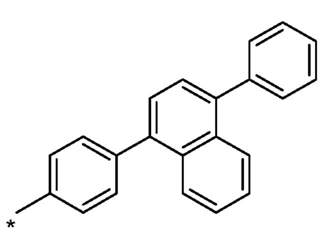 (E27), 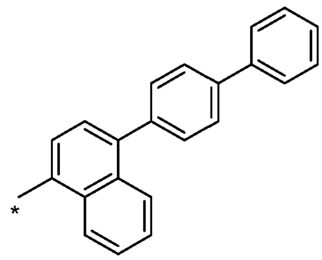 (E28),

- Ar$^4$ is independently selected from F1 to F9:

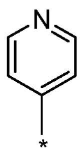 (F1), 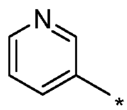 (F2), 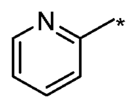 (F3), 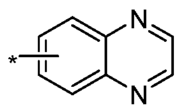 (F4), 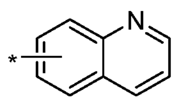 (F5), 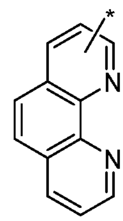 (F6),

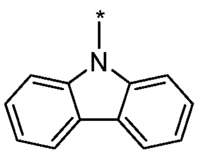 (F7), 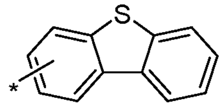 (F8), 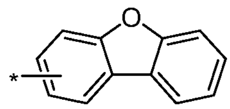 (F9).

**10.** The compound of formula 1 according to any of claims 1 to 9, wherein the compound of formula 1 is selected from G1 to G70:

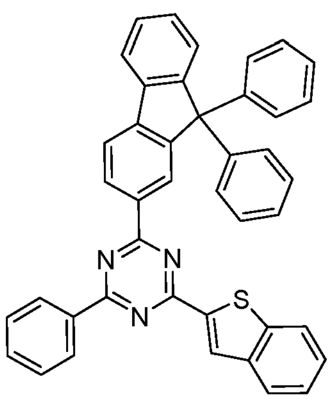 (G1), 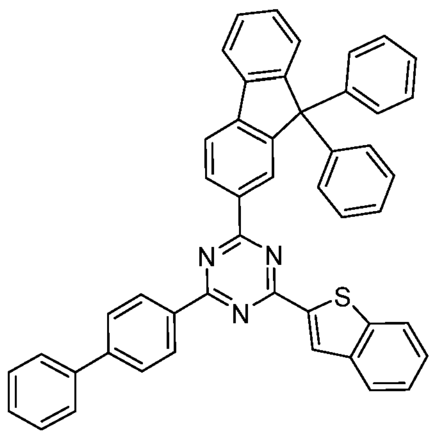 (G2),

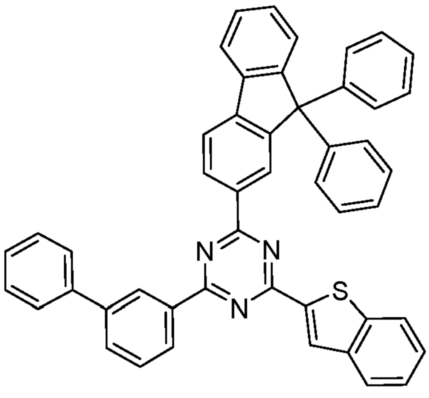 (G3), 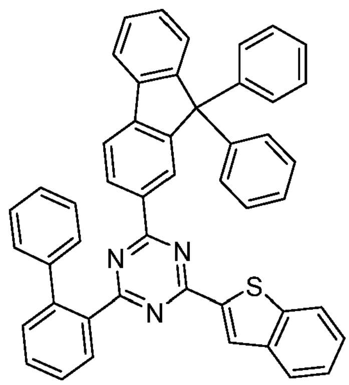 (G4),

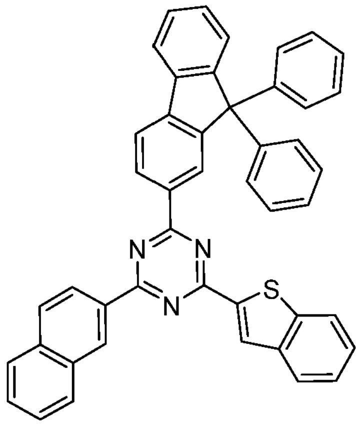 (G5), 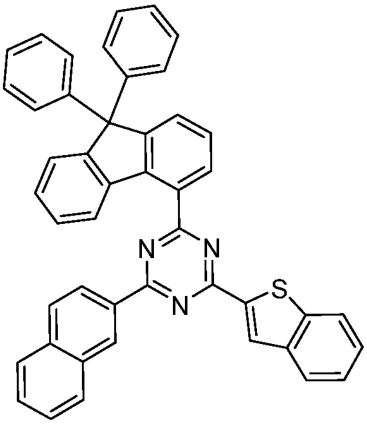 (G6), 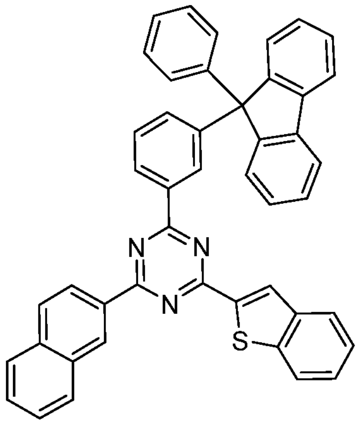

(G7), 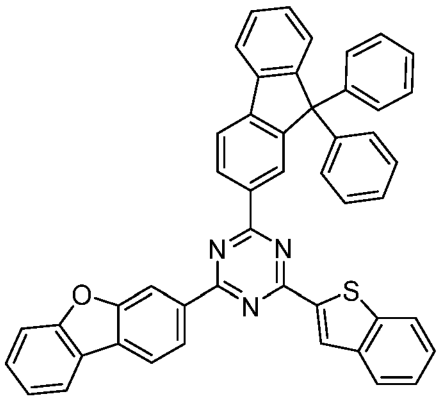 (G8), 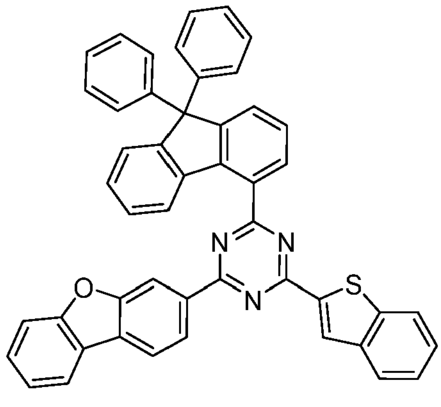 (G9),

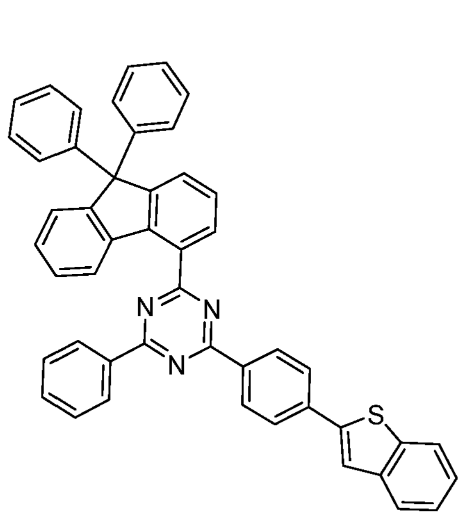

(G10),

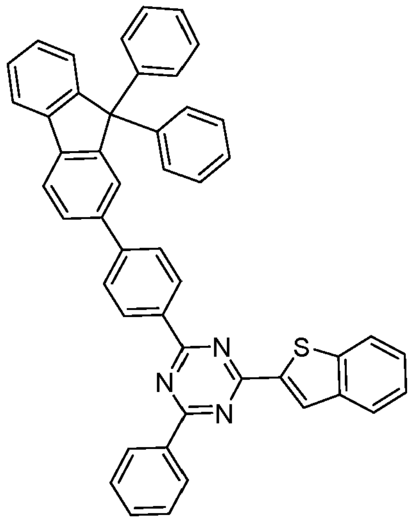

(G11),

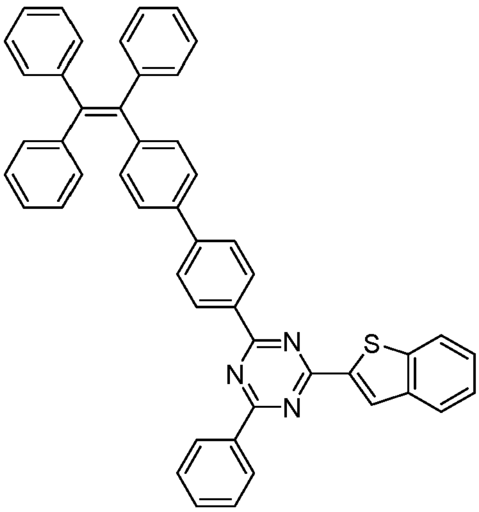

(G12),

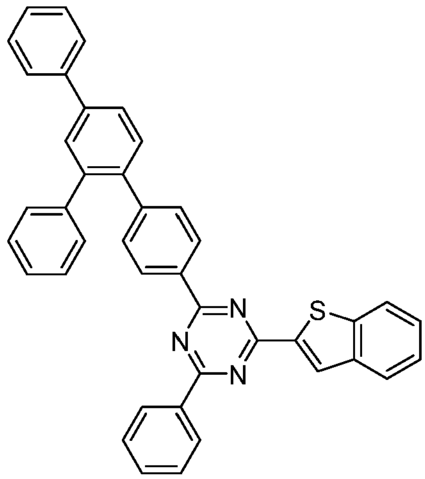

(G13),

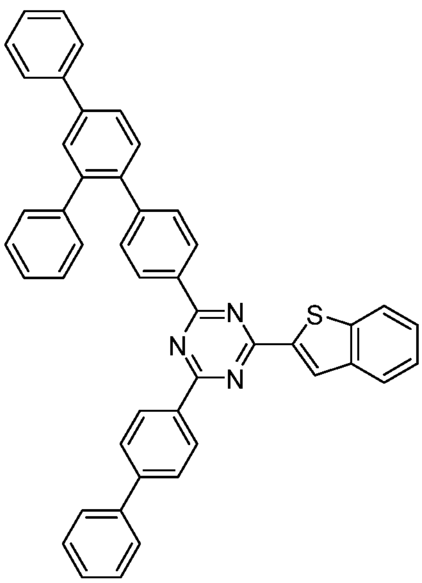

(G14),

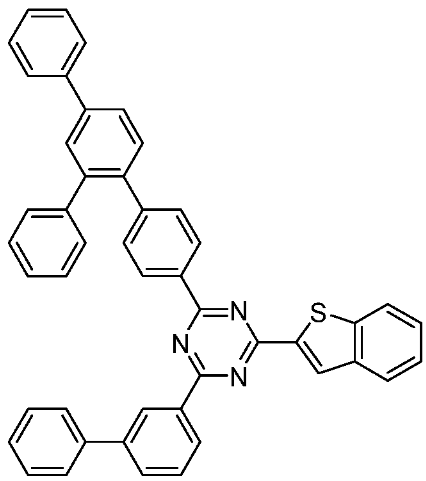

(G15),

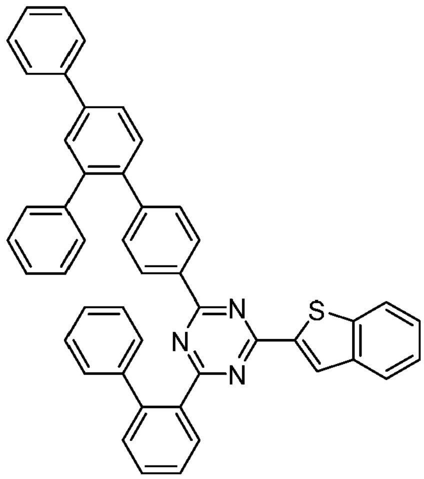

(G16),

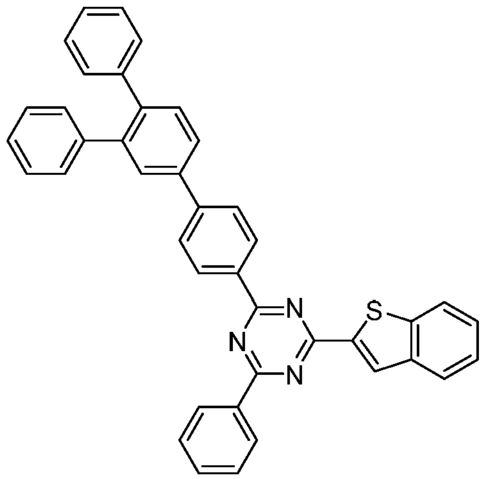

(G17),

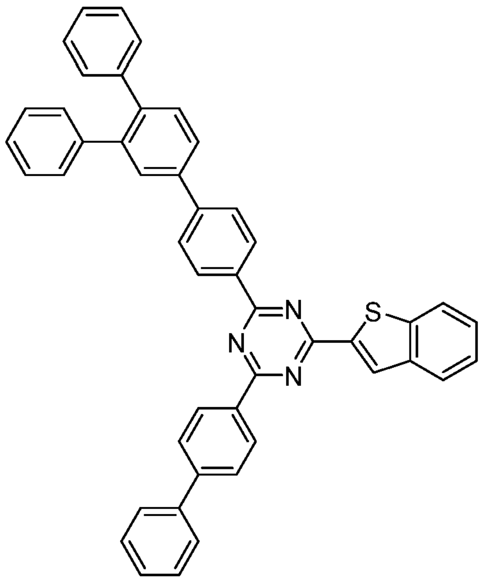

(G18),

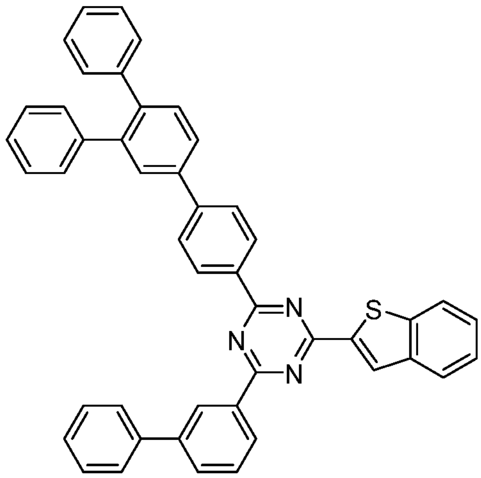

(G19),

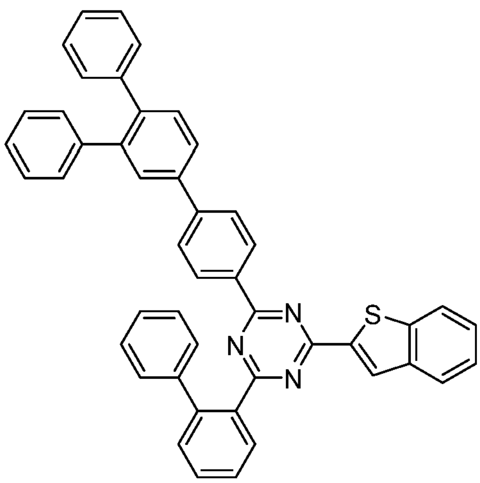

(G20),

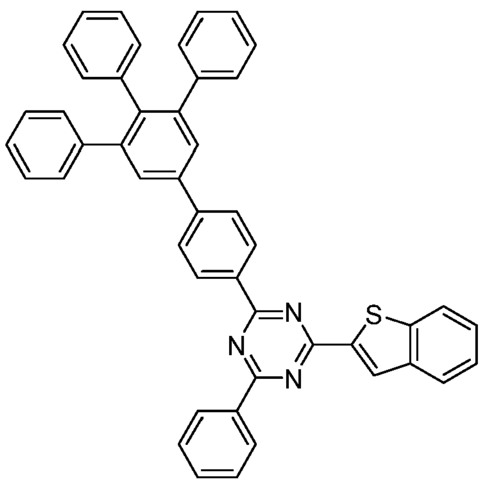

(G21),

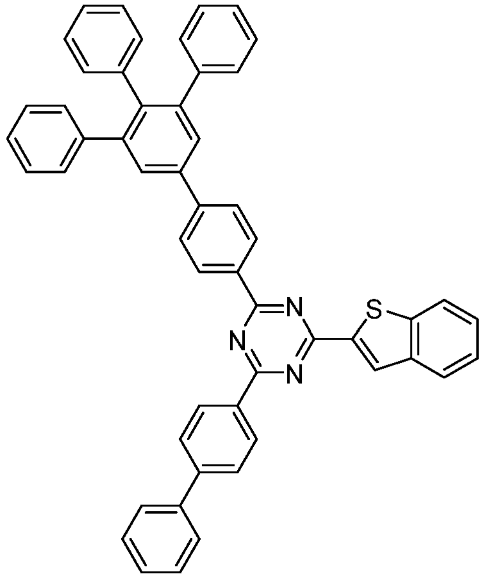

(G22),

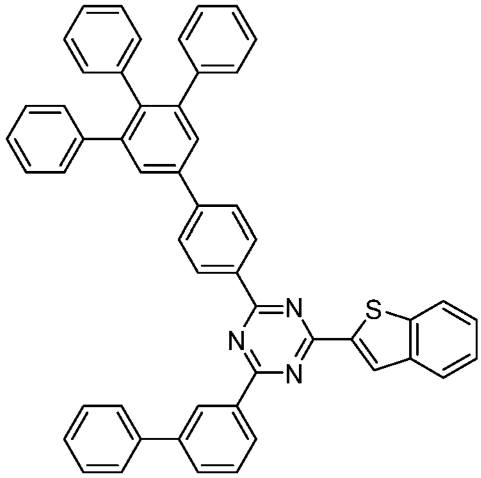

(G23),

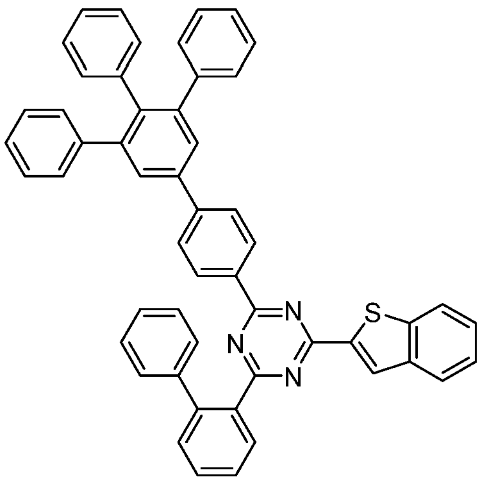

(G24),

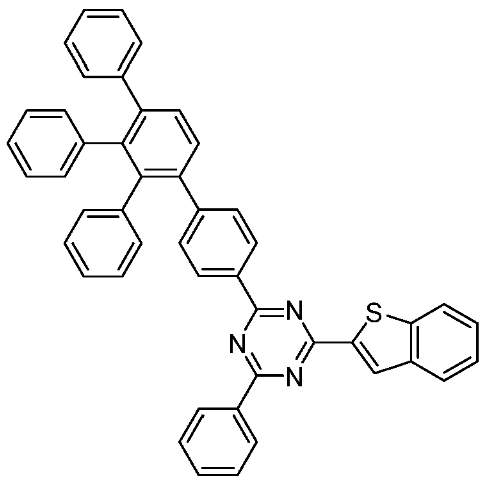

(G25),

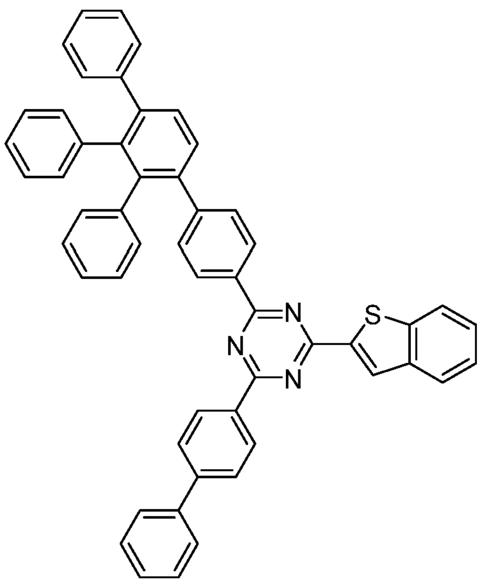

(G26),

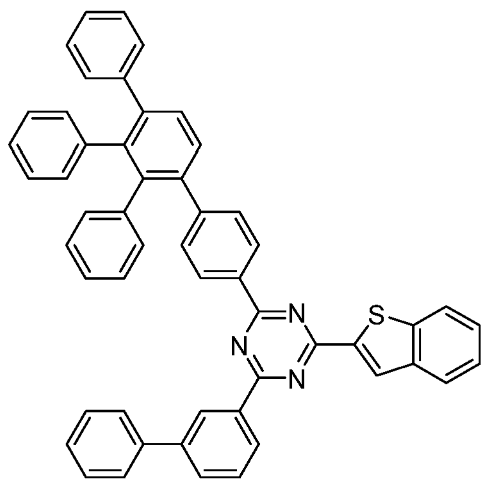

(G27),

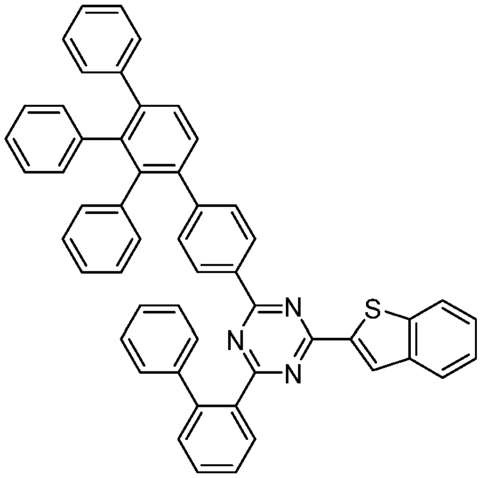

(G28),

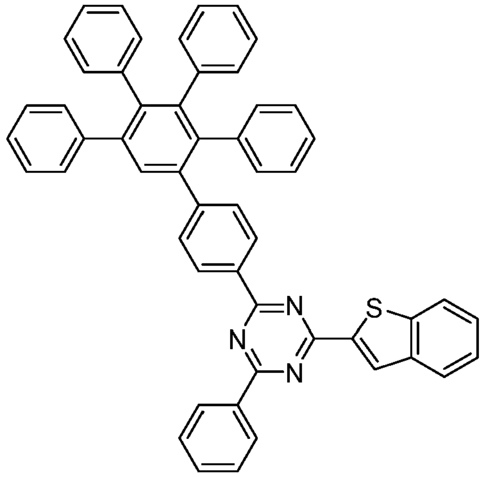

(G29),

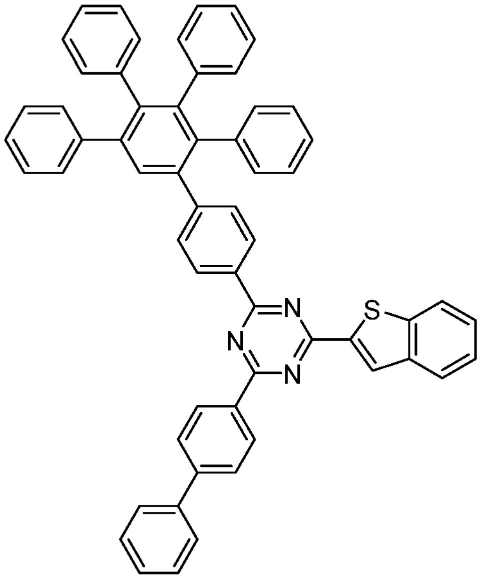

(G30),

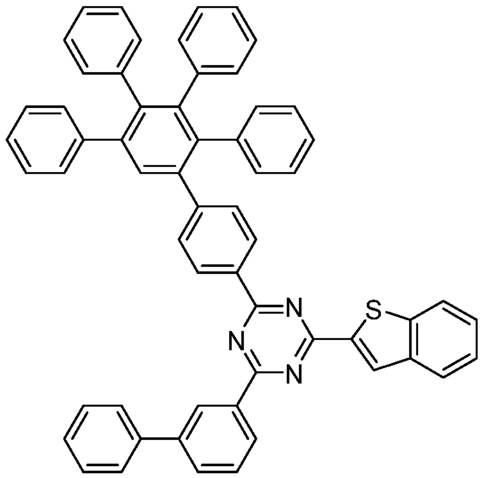

(G31),

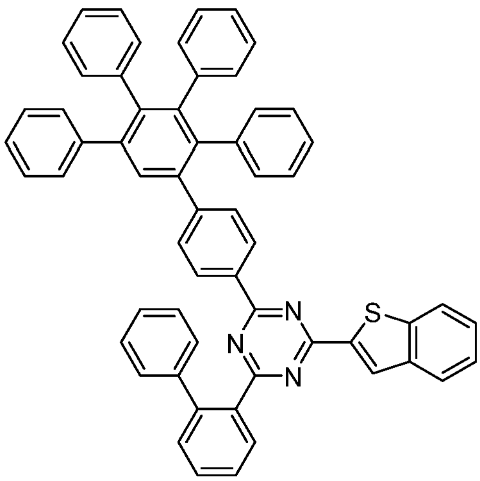

(G32),

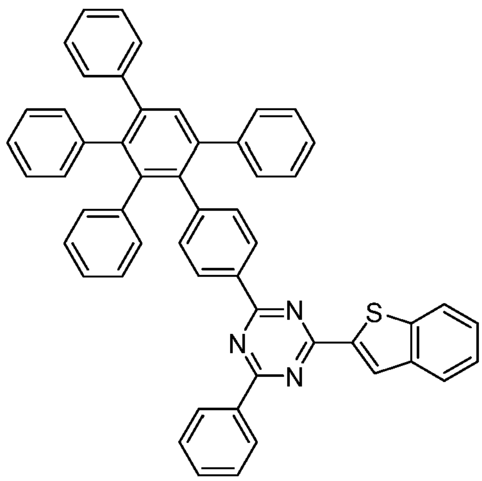

(G33),

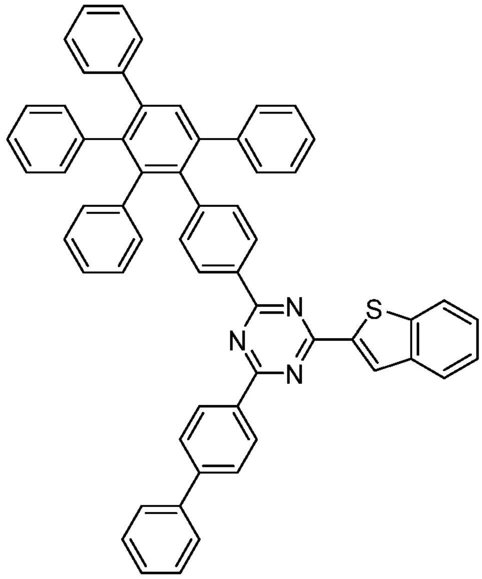

(G34),

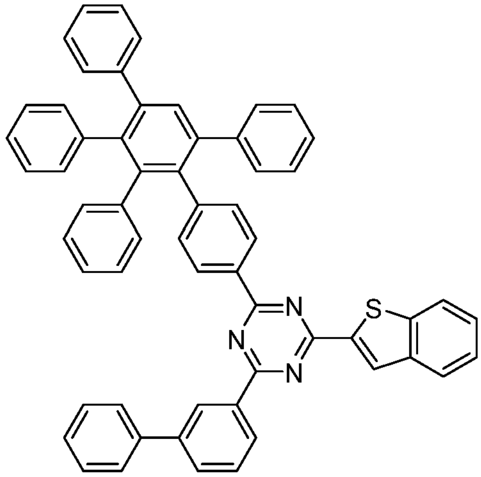

(G35),

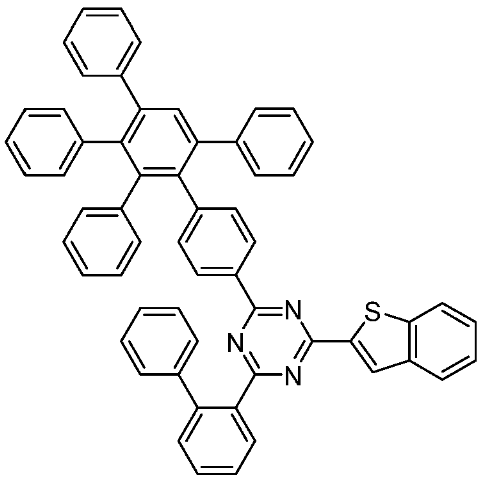

(G36),

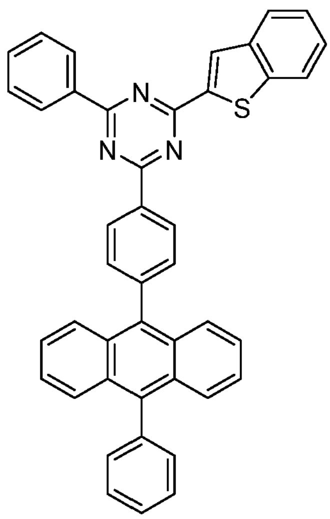

(G37),

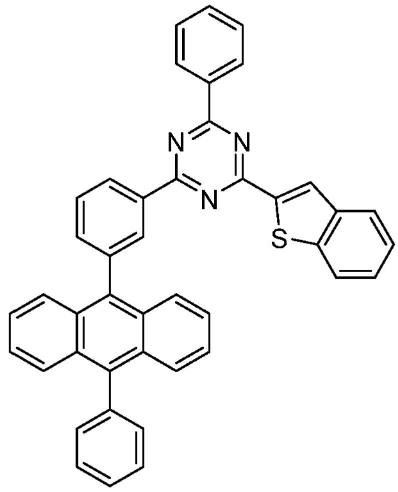

(G38),

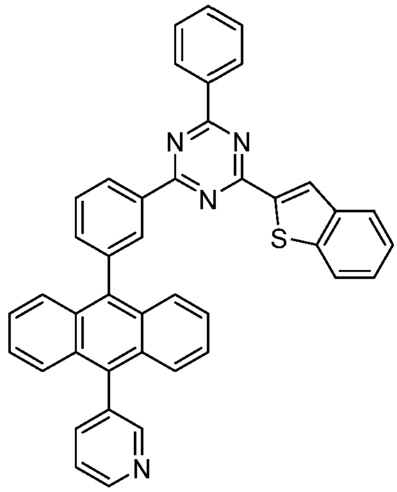

(G39),

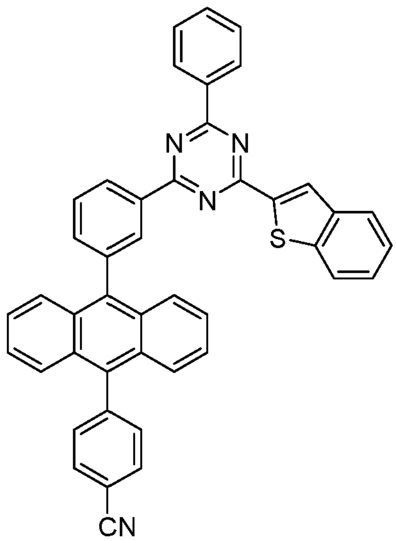
(G40),

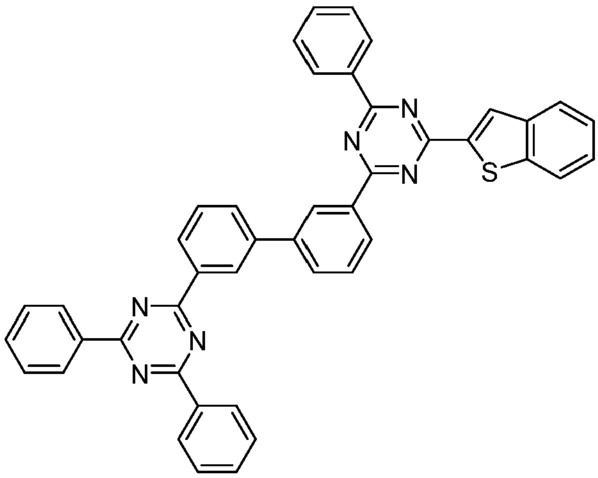
(G41),

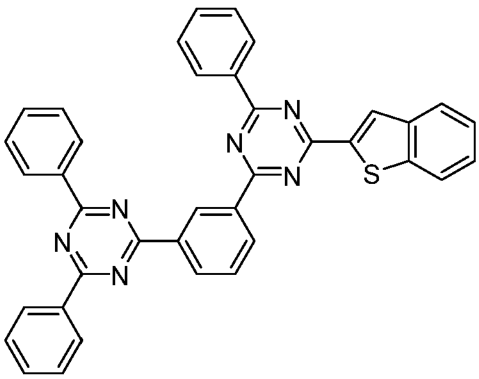
(G42),

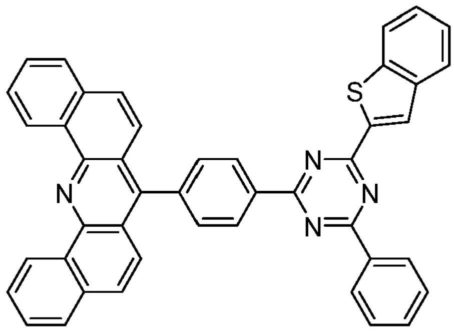
(G43),

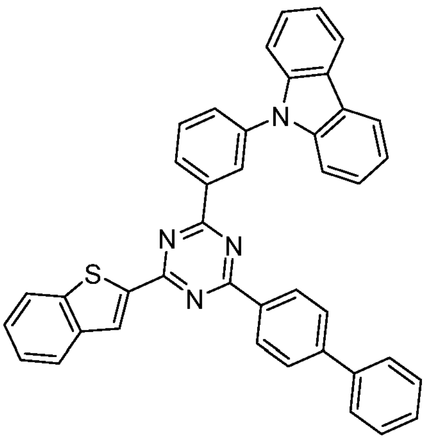
(G44),

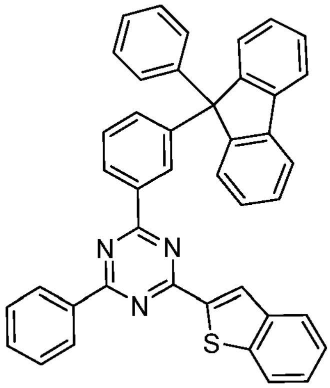
(G45),

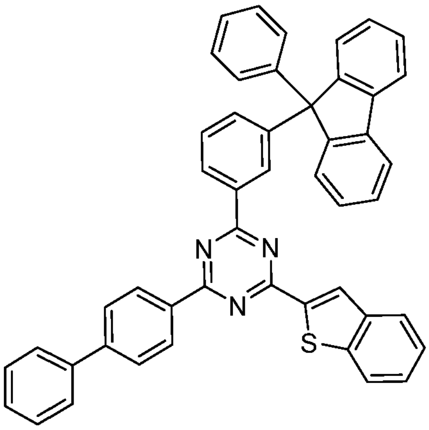
(G46),
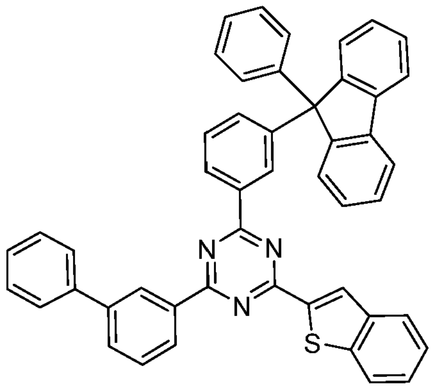
(G47),

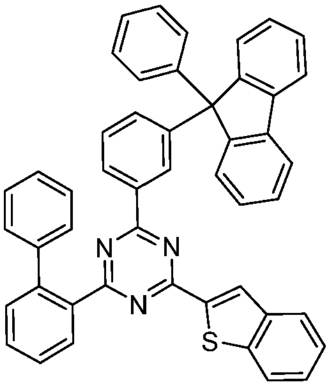
(G48),
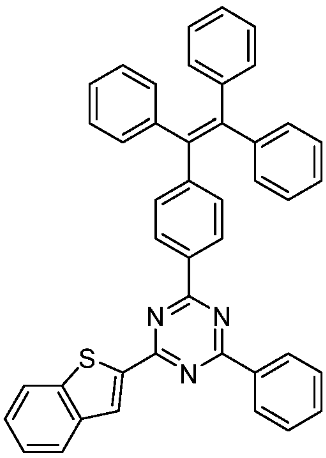
(G49),
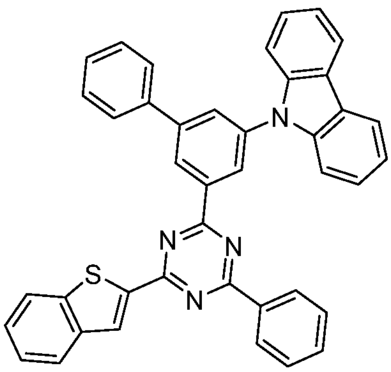

(G50),
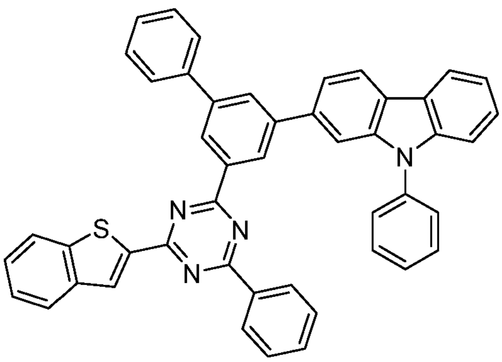
(G51),
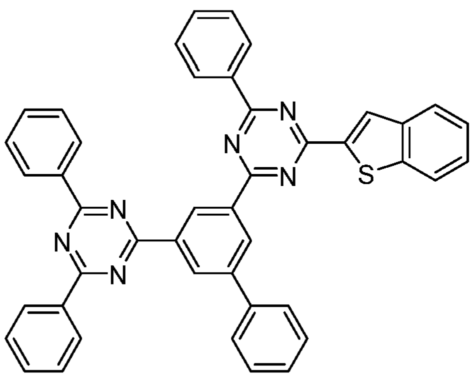
(G52),

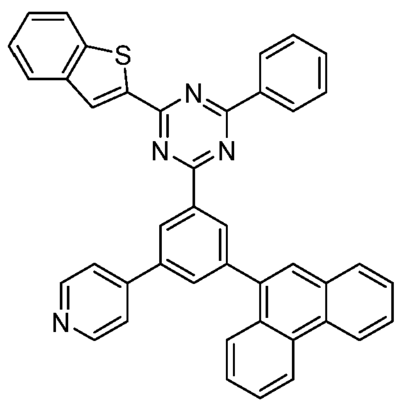 (G53), 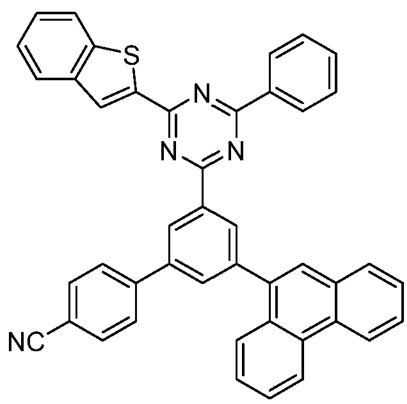 (G54),

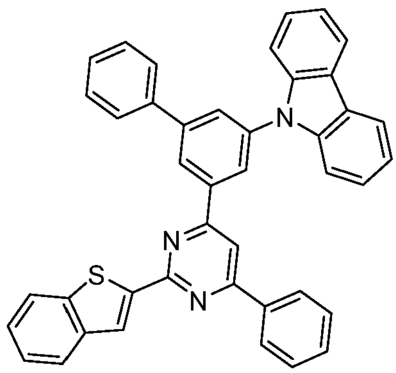 (G55), 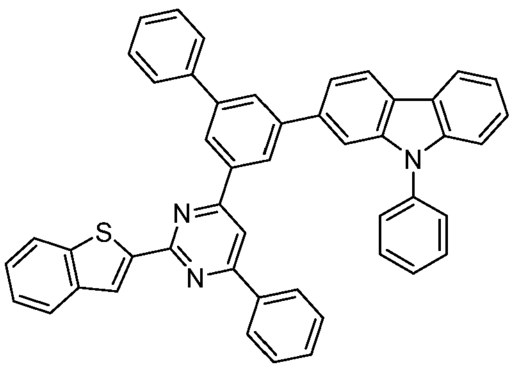 (G57),

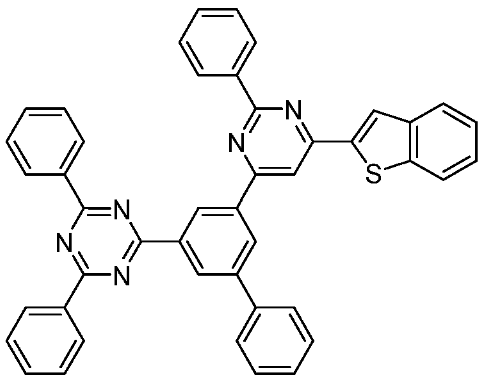 (G58), 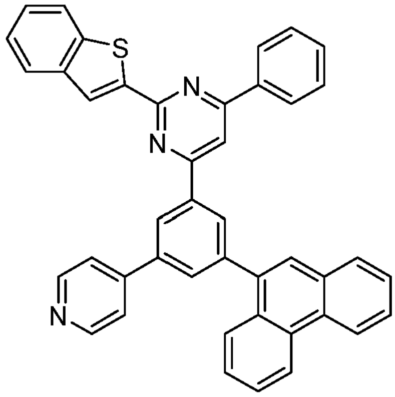 (G59),

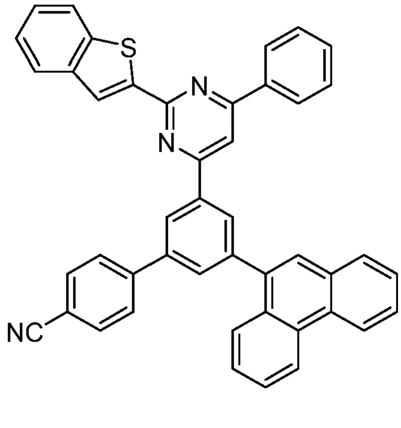 (G60), 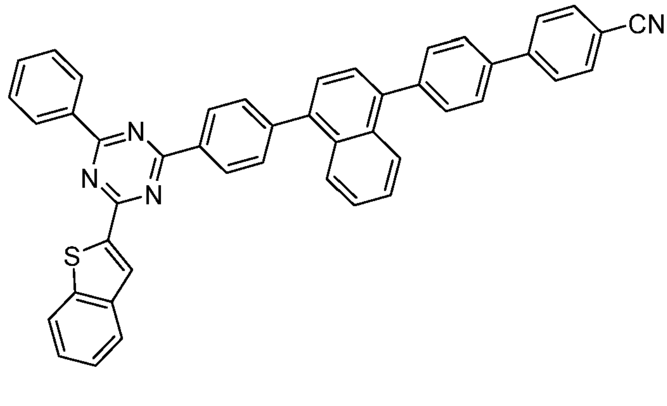 (G61),

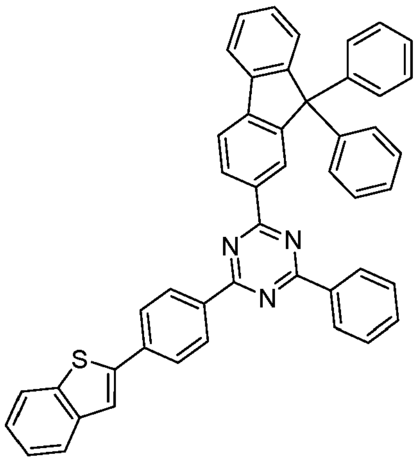
(G62),
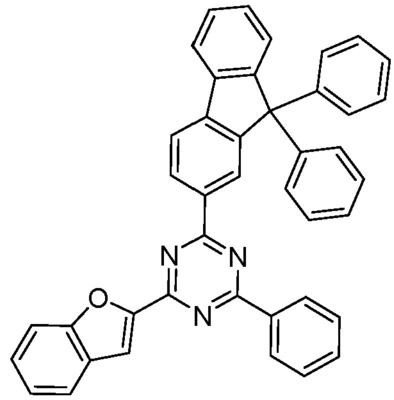
(G63),

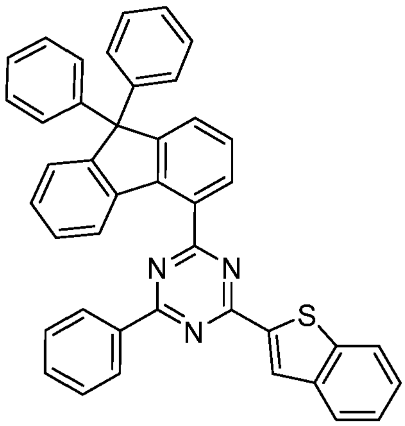
(G64),
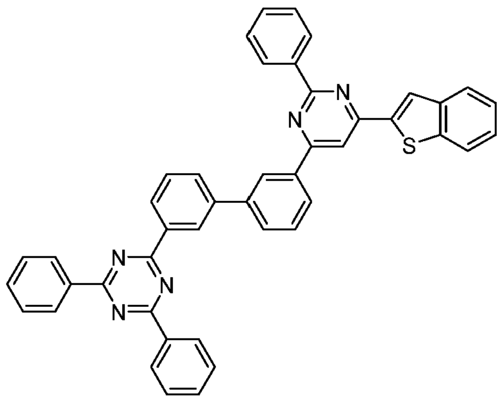
(G65).

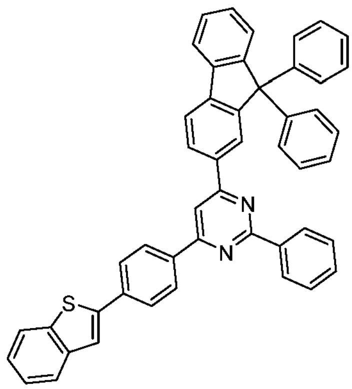
(G66),
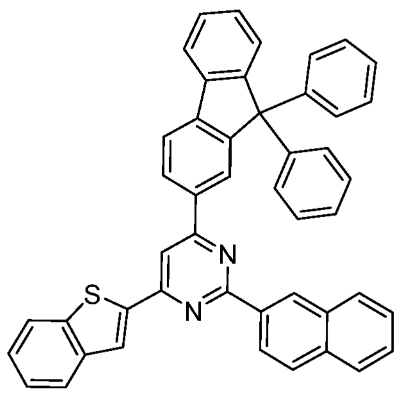
(G67),

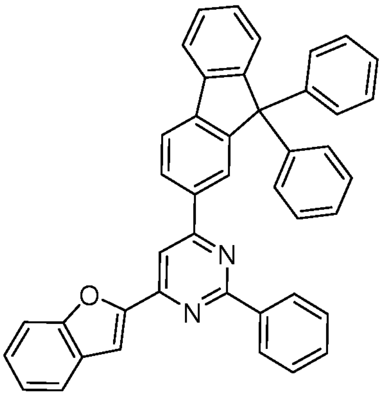 (G68), 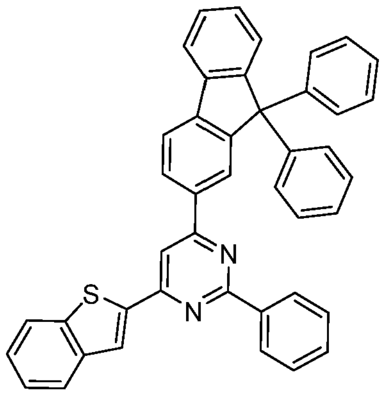 (G69),

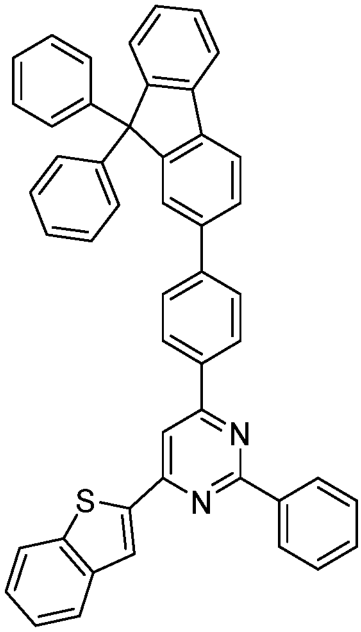 (G70).

11. An organic semiconductor layer comprising at least one compound of formula 1 according to any of the preceding claims 1 to 10.

12. The organic semiconductor layer according to claim 11, wherein the organic semiconductor layer further comprises a metal, metal salt or organic metal complex, preferably an organic monovalent or divalent metal complex, more preferably LiQ or alkali borate.

13. An organic electronic device comprising at least one organic semiconductor layer according to any of the preceding claim 11 or 12, wherein the at least one organic semiconductor layer comprises a compound of formula 1 according to any of the preceding claims 1 to 10.

14. The organic electronic device according to claim 13, wherein the electronic device is a light emitting device, thin film transistor, a battery, a display device or a photovoltaic cell, and preferably a light emitting device.

**Patentansprüche**

1. Verbindung gemäß Formel 1:

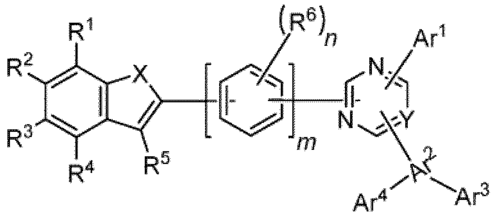

(1),

wobei

Alkylgruppe als eine lineare, cyclische oder verzweigte Alkylgruppe definiert ist;
X für O oder S steht;
$R^1$, $R^2$, $R^3$, $R^4$, $R^5$, $R^6$ unabhängig aus H, substituiertem oder unsubstituiertem $C_6$- bis $C_{38}$-Aryl, substituiertem oder unsubstituiertem $C_3$- bis $C_{36}$-Heteroaryl oder einer $C_1$- bis $C_{16}$-Alkylgruppe ausgewählt sind, wobei
die Substituenten aus $C_6$- bis $C_{18}$-Aryl, $C_3$- bis $C_{25}$-Heteroaryl, -PO $(R')_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis $C_{16}$-Alkoxy ausgewählt sind;
m für eine ganze Zahl steht, die aus 0 oder 1 ausgewählt ist;
n für eine ganze Zahl steht, die aus 0 ausgewählt ist;
Y für CR" oder N steht, wobei R" für H, substituiertes oder unsubstituiertes $C_6$- bis $C_{38}$-Aryl, substituiertes oder unsubstituiertes $C_3$- bis $C_{36}$-Heteroaryl oder eine $C_1$- bis $C_{16}$-Alkylgruppe steht, wobei
die Substituenten aus $C_6$- bis $C_{18}$-Aryl, $C_3$- bis $C_{25}$-Heteroaryl, -PO $(R')_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis $C_{16}$-Alkoxy ausgewählt sind;
$Ar^1$ aus substituiertem oder unsubstituiertem $C_6$- bis $C_{38}$-Aryl oder substituiertem oder unsubstituiertem $C_3$- bis $C_{36}$-Heteroaryl ausgewählt ist, wobei
die Substituenten aus $C_6$- bis $C_{18}$-Aryl, $C_3$- bis $C_{25}$-Heteroaryl, -PO $(R')_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis $C_{16}$-Alkoxy ausgewählt sind;
$Ar^2$ aus substituiertem oder unsubstituiertem $C_6$- bis $C_{38}$-Arylen oder Formel 2,

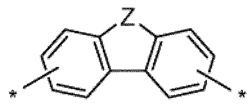

(2)

ausgewählt ist, wobei
die Substituenten aus $C_6$- bis $C_{18}$-Aryl, $C_3$- bis $C_{25}$-Heteroaryl, -PO $(R')_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis $C_{16}$-Alkoxy ausgewählt sind;
Z aus O, S, Se, CR''' oder NR''' ausgewählt ist, wobei R''' aus $C_1$- bis $C_{16}$-Alkyl oder $C_6$- bis $C_{18}$-Aryl ausgewählt ist;
$Ar^3$ aus H, substituiertem oder unsubstituiertem Aryl, substituiertem oder unsubstituiertem Heteroaryl, das aus einer Gruppe bestehend aus Pyridin, Pyrimidin, Triazin, Chinolin, Chinoxalin, Benzoacridin, Dibenzoacridin, Phenanthrolin, Carbazol, Dibenzofuran, Dibenzothiophen ausgewählt ist, oder

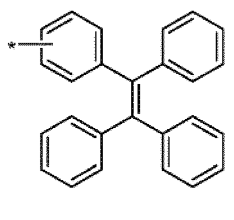

ausgewählt ist, wobei

die Substituenten aus $C_6$- bis $C_{18}$-Aryl, $C_3$- bis $C_{25}$-Heteroaryl, -PO (R')$_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis $C_{16}$-Alkoxy ausgewählt sind;

$Ar^4$ aus H, substituiertem oder unsubstituiertem Aryl, substituiertem oder unsubstituiertem Heteroaryl, das aus einer Gruppe bestehend aus Pyridin, Pyrimidin, Triazin, Chinolin, Chinoxalin, Benzoacridin, Dibenzoacridin, Phenanthrolin, Carbazol, Dibenzofuran, Dibenzothiophen ausgewählt ist, wobei

die Substituenten aus $C_6$- bis $C_{18}$-Aryl, $C_3$- bis $C_{25}$-Heteroaryl, -PO (R')$_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis $C_{16}$-Alkoxy ausgewählt sind;

und wobei $Ar^4$ für H steht, wenn $Ar^2$ aus Formel 2 ausgewählt ist.

**2.** Verbindung der Formel 1 nach Anspruch 1, wobei X für O steht;

$R^1$, $R^2$, $R^3$, $R^4$, $R^5$, $R^6$ unabhängig aus H, substituiertem oder unsubstituiertem $C_6$- bis $C_{38}$-Aryl, substituiertem oder unsubstituiertem $C_3$- bis $C_{36}$-Heteroaryl oder einer $C_1$- bis $C_{16}$-Alkylgruppe ausgewählt sind, wobei

die Substituenten aus $C_6$- bis $C_{18}$-Aryl, $C_3$- bis $C_{25}$-Heteroaryl, -PO (R')$_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis $C_{16}$-Alkoxy ausgewählt sind;

m für eine ganze Zahl steht, die aus 0 oder 1 ausgewählt ist;

n für eine ganze Zahl steht, die aus 0 ausgewählt ist;

Y für N oder CH steht;

$Ar^1$ aus substituiertem oder unsubstituiertem $C_6$- bis $C_{38}$-Aryl oder substituiertem oder unsubstituiertem $C_3$- bis $C_{36}$-Heteroaryl ausgewählt ist, wobei

die Substituenten aus $C_6$- bis $C_{18}$-Aryl, $C_3$- bis $C_{25}$-Heteroaryl, -PO (R')$_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis $C_{16}$-Alkoxy ausgewählt sind;

$Ar^2$ aus substituiertem oder unsubstituiertem $C_6$- bis $C_{38}$-Arylen oder Formel 2,

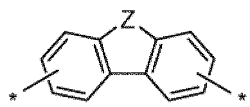

(2)

ausgewählt ist, wobei

die Substituenten aus $C_6$- bis $C_{18}$-Aryl, $C_3$- bis $C_{25}$-Heteroaryl, -PO (R')$_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis $C_{16}$-Alkoxy ausgewählt sind;

Z aus O, S, Se, CR''' oder NR''' ausgewählt ist, wobei R''' aus $C_1$- bis $C_{16}$-Alkyl oder $C_6$- bis $C_{18}$-Aryl ausgewählt ist;

$Ar^3$ aus H, substituiertem oder unsubstituiertem Aryl, substituiertem oder unsubstituiertem Heteroaryl, das aus einer Gruppe bestehend aus Pyridin, Pyrimidin, Triazin, Chinolin, Chinoxalin, Benzoacridin, Dibenzoacridin, Phenanthrolin, Carbazol, Dibenzofuran, Dibenzothiophen ausgewählt ist, ausgewählt ist, wobei

die Substituenten aus $C_6$- bis $C_{18}$-Aryl, $C_3$- bis $C_{25}$-Heteroaryl, -PO (R')$_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis $C_{16}$-Alkoxy ausgewählt sind;

$Ar^4$ aus H, substituiertem oder unsubstituiertem Aryl, substituiertem oder unsubstituiertem Heteroaryl, das aus einer Gruppe bestehend aus Pyridin, Pyrimidin, Triazin, Chinolin, Chinoxalin, Benzoacridin, Dibenzoacridin, Phenanthrolin, Carbazol, Dibenzofuran, Dibenzothiophen ausgewählt ist, wobei

die Substituenten aus $C_6$- bis $C_{18}$-Aryl, $C_3$- bis $C_{25}$-Heteroaryl, -PO (R')$_2$, wobei R' für Alkyl, Aryl oder Heteroaryl steht, D, F oder CN, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$- bis $C_{16}$-Alkoxy, teil- oder perfluoriertem $C_1$- bis $C_{16}$-Alkoxy, teil- oder perdeuteriertem $C_1$- bis

$C_{16}$-Alkoxy ausgewählt sind.

3. Verbindung der Formel 1 nach Anspruch 1 oder 2, wobei die Verbindung der Formel 1 durch Formel 1a und/oder 1b wiedergegeben wird:

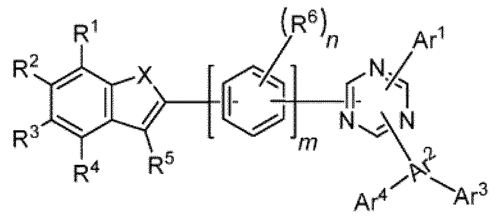

(1a);

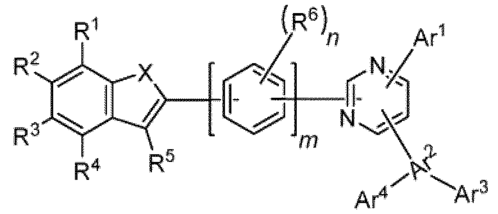

(1b).

4. Verbindung der Formel 1 nach den Ansprüchen 1 bis 3, wobei

X für O oder S steht;
$R^1$, $R^2$, $R^3$, $R^4$, $R^5$, $R^6$ unabhängig aus H, Phenyl, Naphthyl oder Biphenyl ausgewählt sind;
m für 0 steht;
n für 0 steht;
Y für N oder CH, vorzugsweise N, steht;
$Ar^1$ aus einer Phenyl-, Naphthyl-, Biphenyl-, Phenanthryl-, Triphenyl- oder Anthrylgruppe ausgewählt ist;
$Ar^2$ aus einer zweiwertigen Phenylen-, Naphthylen-, Biphenylen-, Phenanthrylen-, Triphenylen- oder Anthrylengruppe ausgewählt ist;
Z aus O, S, Se, CR‴ oder NR‴ ausgewählt ist, wobei R‴ aus $C_1$- bis $C_{16}$-Alkyl oder $C_6$- bis $C_{18}$-Aryl ausgewählt ist;
$Ar^3$ aus H, substituiertem oder unsubstituiertem Aryl, substituiertem oder unsubstituiertem Heteroaryl, das aus einer Gruppe bestehend aus Pyridin, Pyrimidin, Triazin, Chinolin, Chinoxalin, Benzoacridin, Dibenzoacridin, Phenanthrolin, Carbazol, Dibenzofuran, Dibenzothiophen ausgewählt ist, ausgewählt ist;
$Ar^4$ aus H, substituiertem oder unsubstituiertem Aryl, substituiertem oder unsubstituiertem Heteroaryl, das aus einer Gruppe bestehend aus Pyridin, Chinolin, Chinoxalin, Phenanthrolin, Carbazol, Dibenzofuran, Dibenzothiophen ausgewählt ist, ausgewählt ist.

5. Verbindung der Formel 1 nach den Ansprüchen 1 bis 4, wobei

$Ar^2$ aus Formel 2 ausgewählt ist und
Z aus O, S, Se, CR‴ oder NR‴, wobei R‴ aus $C_1$-bis $C_{16}$-Alkyl oder $C_6$- bis $C_{18}$-Aryl ausgewählt ist, und weiter bevorzugt CR‴, wobei R‴ aus $C_1$- bis $C_{16}$-Alkyl oder $C_6$- bis $C_{18}$-Aryl ausgewählt ist, ausgewählt ist.

6. Verbindung der Formel 1 nach einem der Ansprüche 1 bis 4, wobei die Verbindung der Formel 1 mindestens einen Nicht-Hetero-Phenylring bis zwölf Nicht-Hetero-Phenylringe, vorzugsweise mindestens zwei Phenylringe bis sieben Phenylringe, umfasst.

7. Verbindung der Formel 1 nach einem der Ansprüche 1 bis 6, wobei die Verbindung der Formel 1 mindestens einen bis vier, vorzugsweise einen bis drei, weiter bevorzugt einen oder zwei und weiter bevorzugt einen heteroaromatischen 6-gliedrigen Ring und mindestens einen bis zwei, vorzugsweise einen, heteroaromatischen 5-gliedrigen Ring umfasst.

8. Verbindung der Formel 1 nach einem der Ansprüche 1 bis 7, wobei $Ar^1$ und $Ar^3$ und/oder $Ar^4$ unabhängig aus substituiertem oder unsubstituiertem $C_6$- bis $C_{18}$-Aryl oder substituiertem oder unsubstituiertem $C_5$-bis $C_{18}$-Hete-

roaryl ausgewählt sind und $Ar^2$ unabhängig aus substituiertem oder unsubstituiertem $C_6$- bis $C_{18}$-Arylen oder substituiertem oder unsubstituiertem $C_5$-bis $C_{18}$-Heteroarylen ausgewählt sind, wobei
die Substituenten des substituierten $C_6$- bis $C_{18}$-Aryls, des substituierten $C_5$- bis $C_{18}$-Heteroaryls, des substituierten $C_6$- bis $C_{18}$-Arylens oder des substituierten $C_5$- bis $C_{18}$-Heteroarylens unabhängig aus $C_1$- bis $C_{12}$-Alkyl, $C_1$- bis $C_{12}$-Alkoxy, teil- oder perfluoriertem $C_1$-bis $C_{12}$-Alkyl, teil- oder perfluoriertem $C_1$- bis $C_{16}$-Alkoxy, teil- oder perdeuteriertem $C_1$- bis $C_{12}$-Alkyl, teil- oder perdeuteriertem $C_1$- bis $C_{16}$-Alkoxy, $C_6$- bis $C_{18}$-Aryl, $C_3$- bis $C_{25}$-Heteroaryl, D, F oder CN, vorzugsweise aus $C_1$- bis $C_{12}$-Alkyl, ausgewählt sind.

**9.** Verbindung der Formel 1 nach einem der Ansprüche 1 bis 8, wobei

- $Ar^1$ aus Phenyl, Biphenyl, Terphenyl, Naphthyl, Phenanthryl, Triphenyl, Anthracenyl und vorzugsweise aus C1 bis C9 ausgewählt ist:

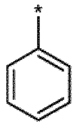 (C1), 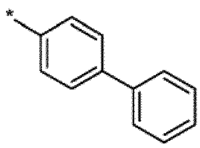 (C2), 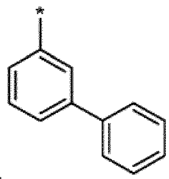 (C3), 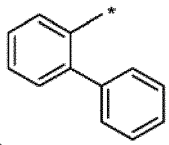 (C4),

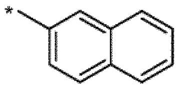 (C5), 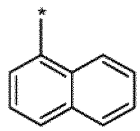 (C6), 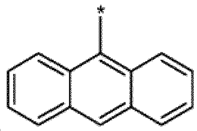 (C7), 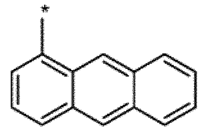 (C8),

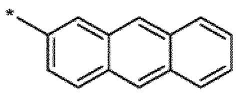 (C9);

und/oder
- $Ar^2$ aus Phenylen, Biphenylen, Terphenylen, Naphthylen, Phenanthrylen, Triphenylen, Anthracenylen und vorzugsweise aus D1 bis D13 ausgewählt ist:

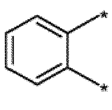 (D1), 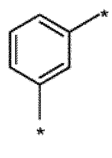 (D2), 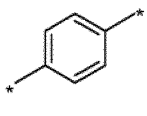 (D3), 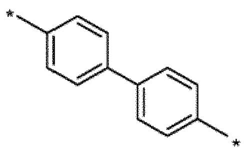 (D4),

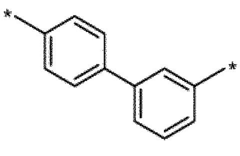 (D5), 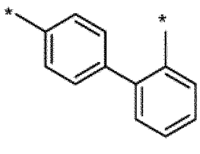 (D6), 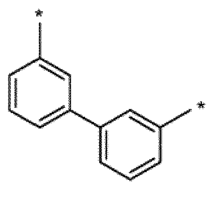 (D7),

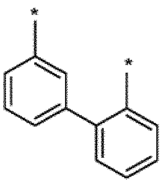 (D8), 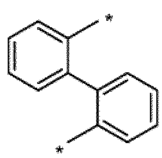 (D9), 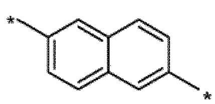 (D10), 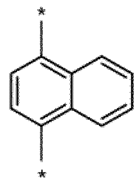 (D11),

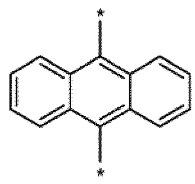 (D12), 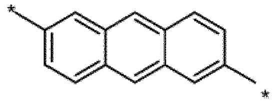 (D13);

- $Ar^3$ unabhängig aus E1 bis E28 ausgewählt ist:

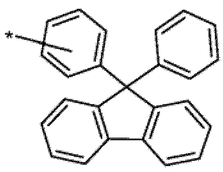 (E1), 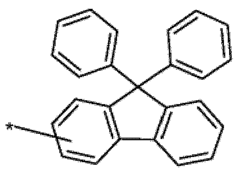 (E2), 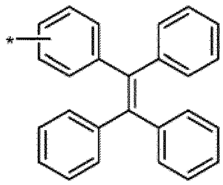 (E3),

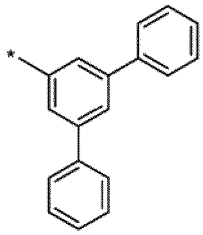 (E4), 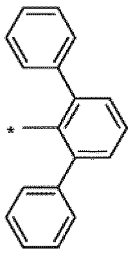 (E5), 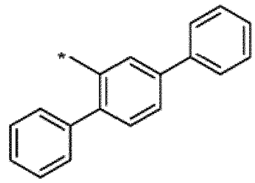 (E6),

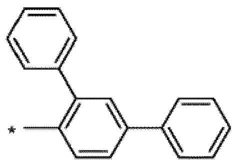 (E7), 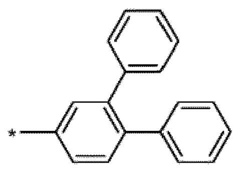 (E8), 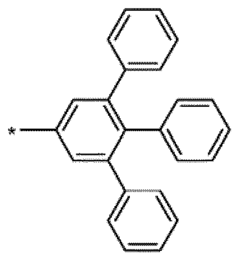 (E9),

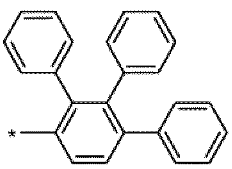 (E10), 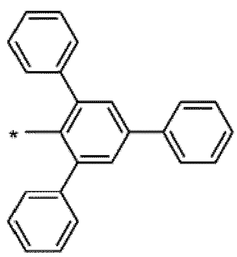 (E11),

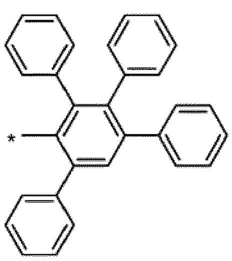 (E12), 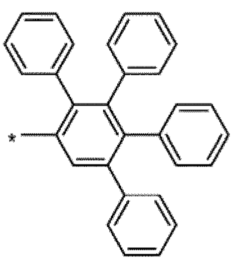 (E13), 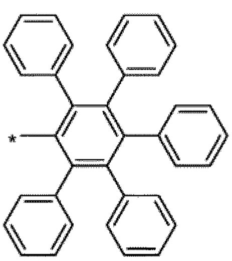 (E14),

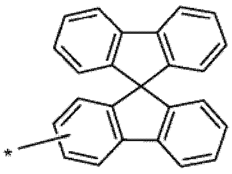 (E15), 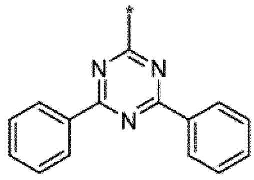 (E16), 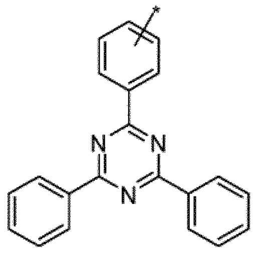 (E17),

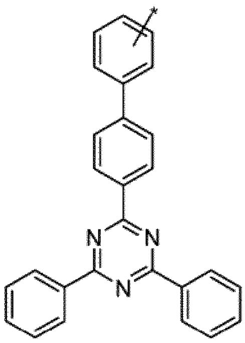 (E18), 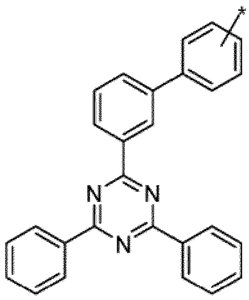 (E19), 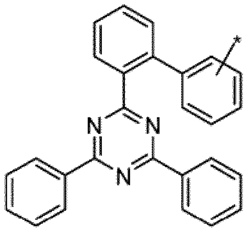 (E20),

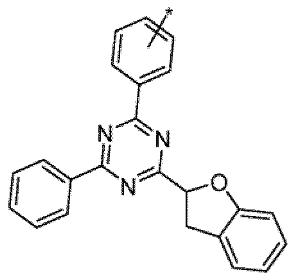 (E21),

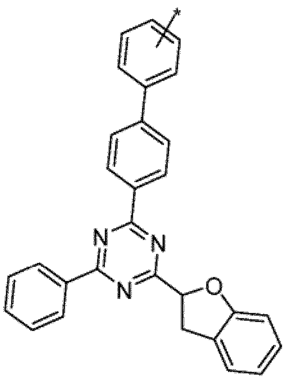 (E22), 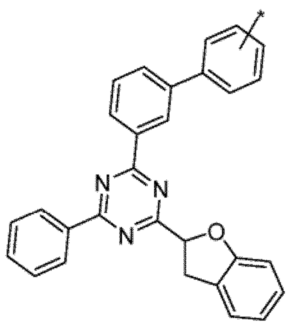 (E23),

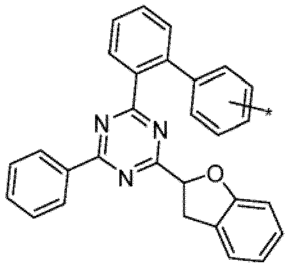 (E24), 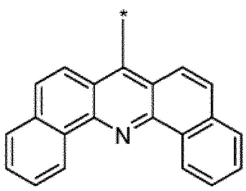 (E25); 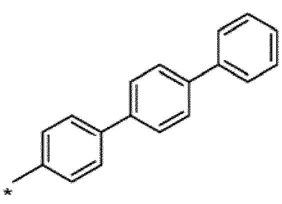 (E26),

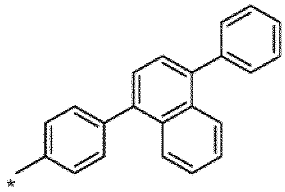 (E27), 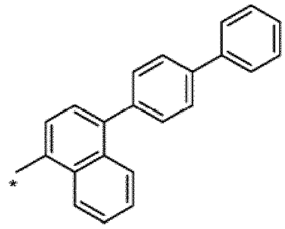 (E28),

- $Ar^4$ unabhängig aus F1 bis F9 ausgewählt ist:

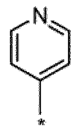 (F1), 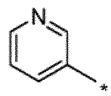 (F2), 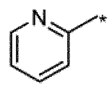 (F3), 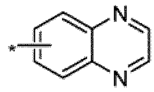 (F4), 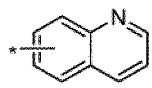 (F5), 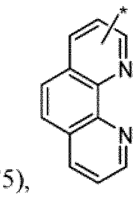 (F6),

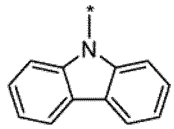 (F7), 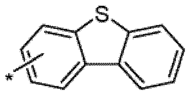 (F8), 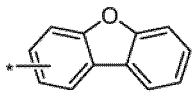 (F9).

**10.** Verbindung der Formel 1 nach einem der Ansprüche 1 bis 9, wobei die Verbindung der Formel 1 aus G1 bis G70 ausgewählt ist:

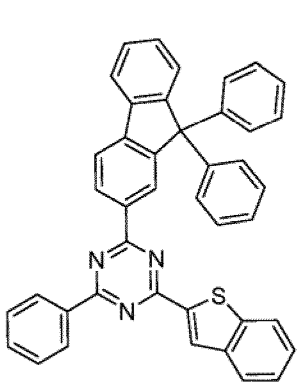 (G1), 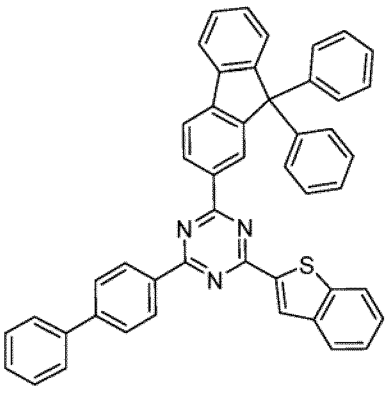 (G2),

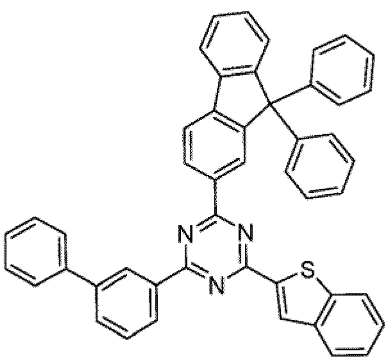 (G3), 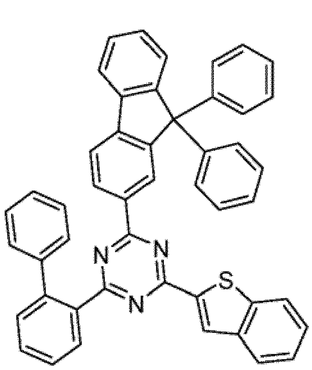 (G4),

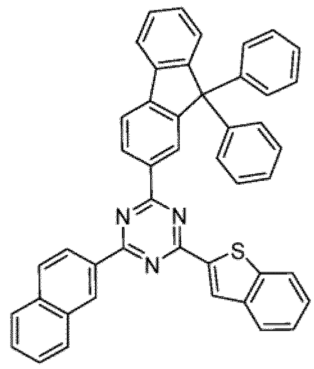

(G5),

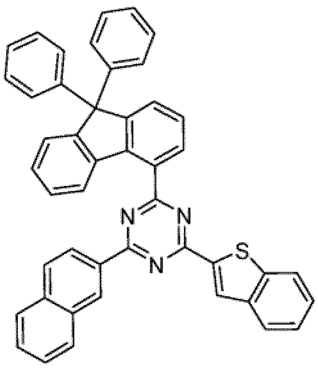

(G6),

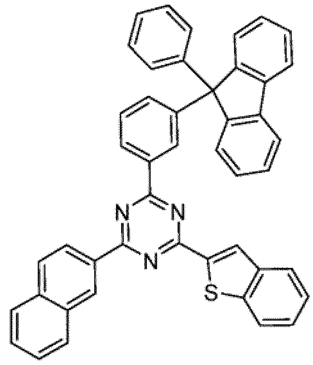

(G7),

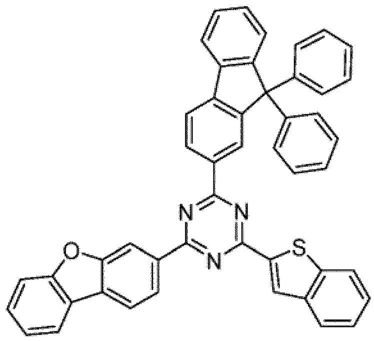

(G8),

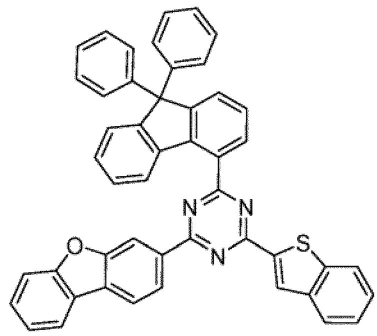

(G9),

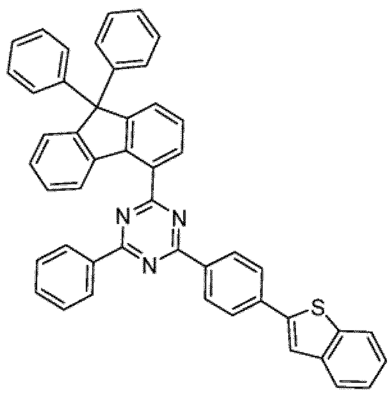

(G10),

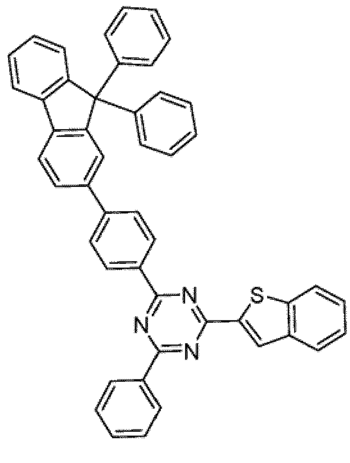

(G11),

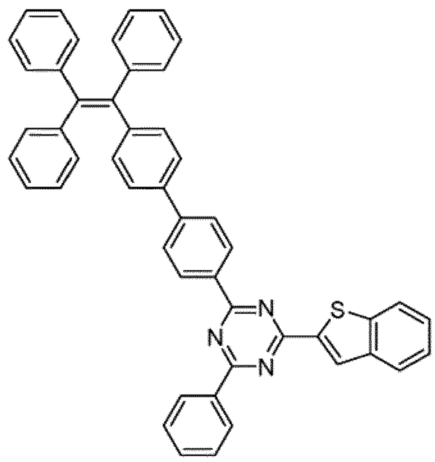

(G12),

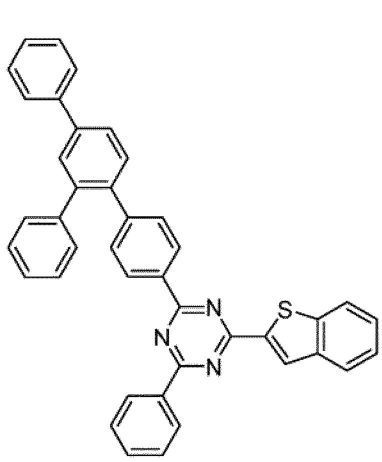

(G13),

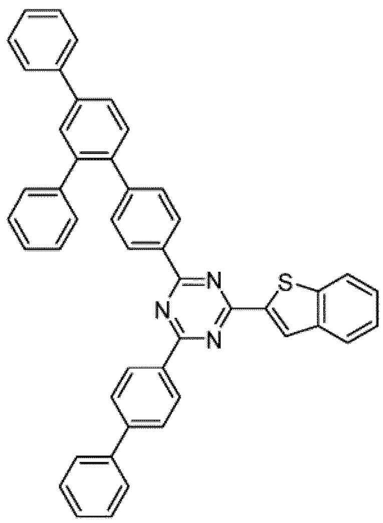
(G14),
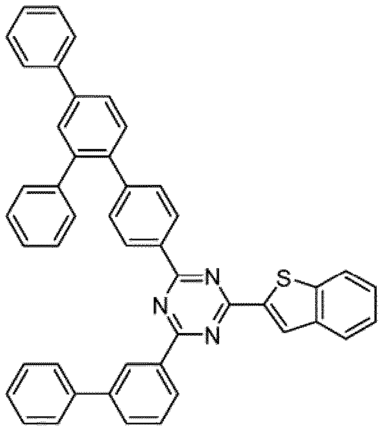
(G15),

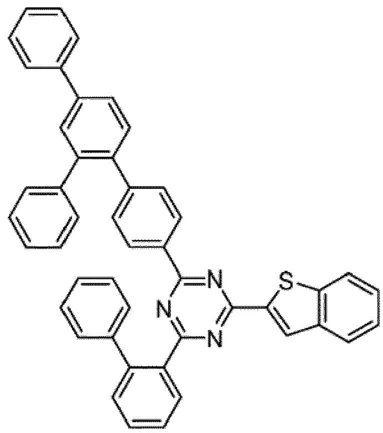
(G16),
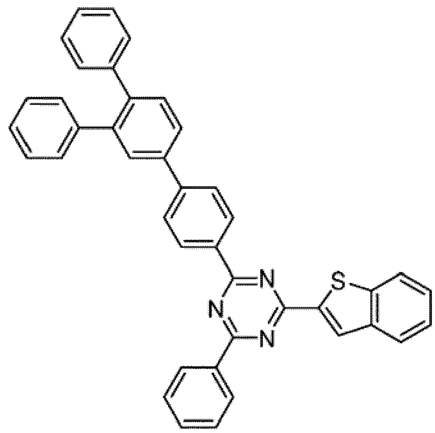
(G17),

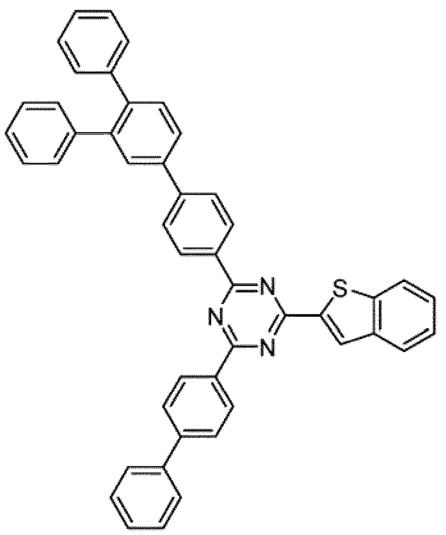
(G18),
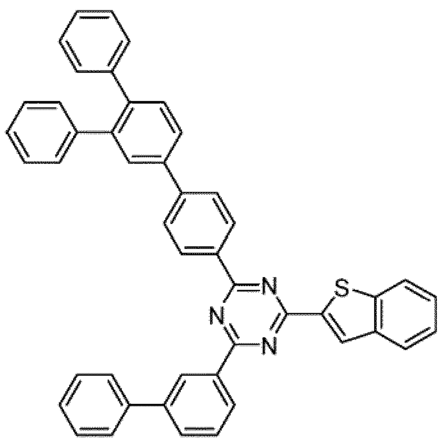
(G19),

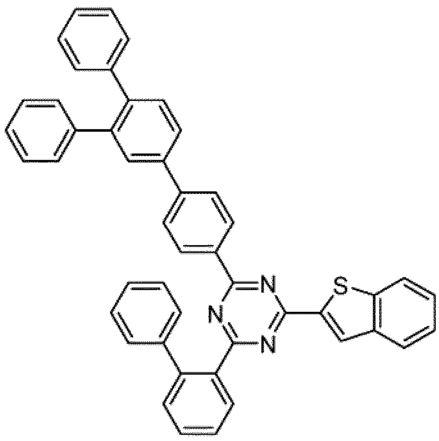
(G20),

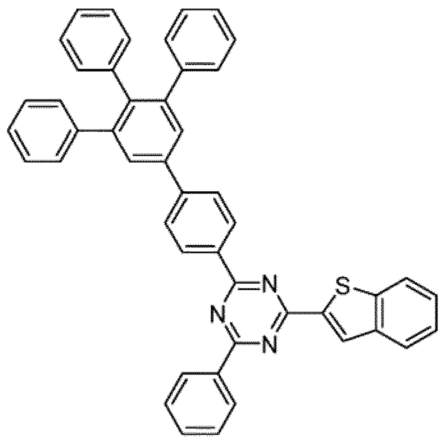
(G21),

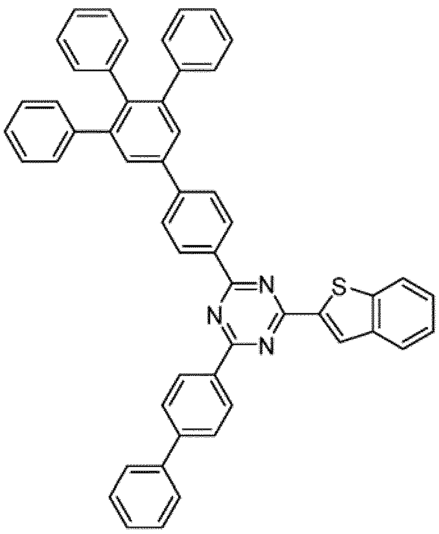
(G22),

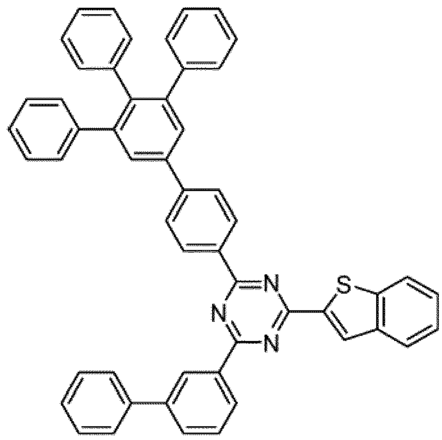
(G23),

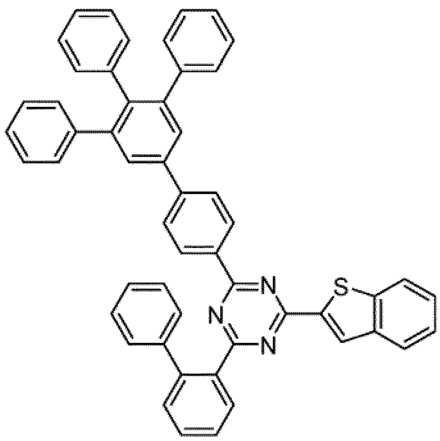
(G24),

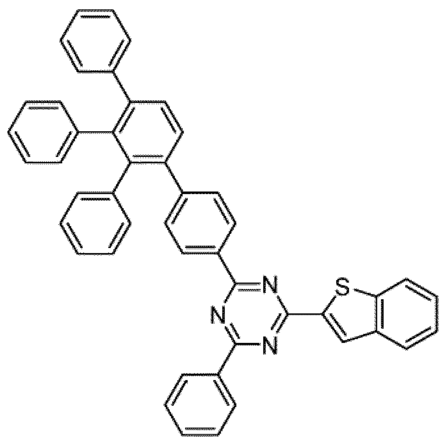
(G25),

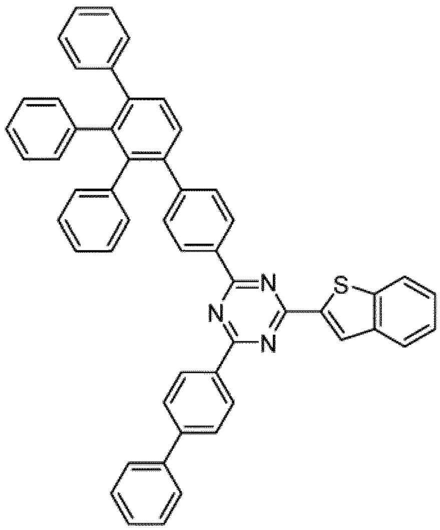

(G26),

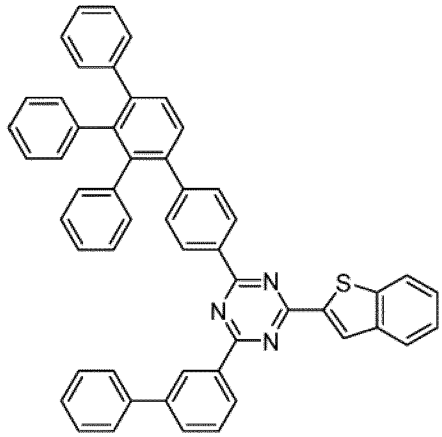

(G27),

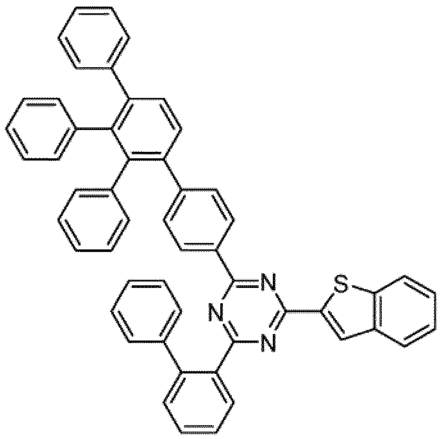

(G28),

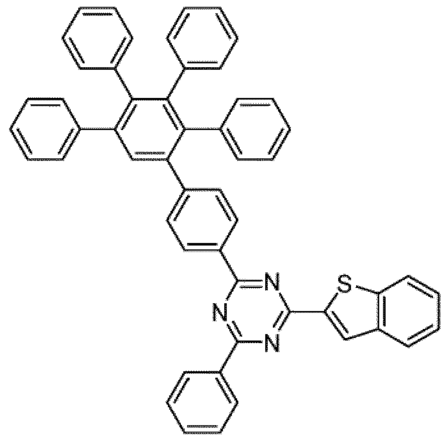

(G29),

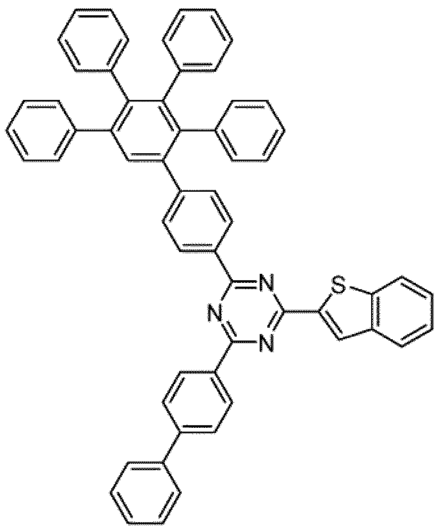

(G30),

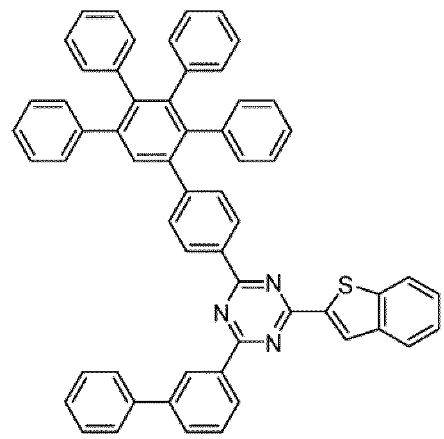

(G31),

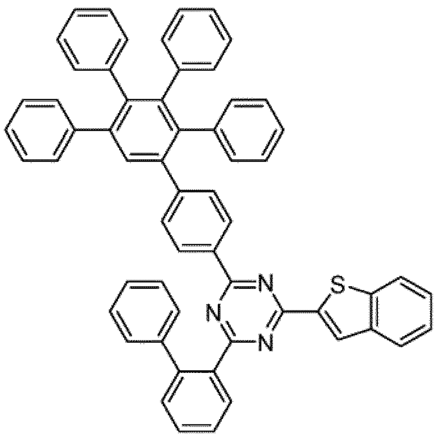
(G32),

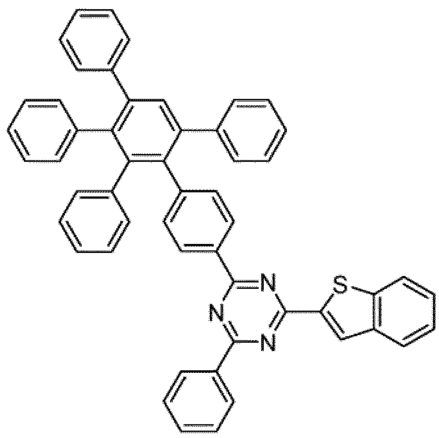
(G33),

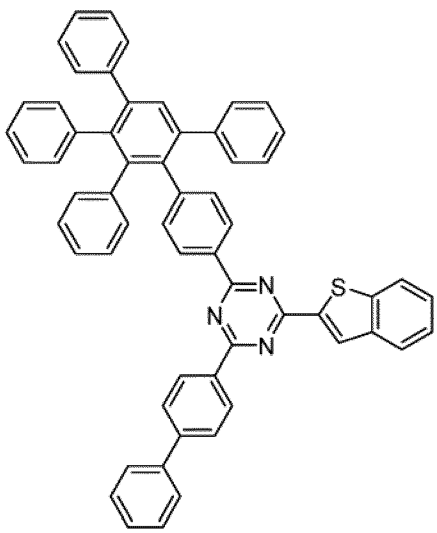
(G34),

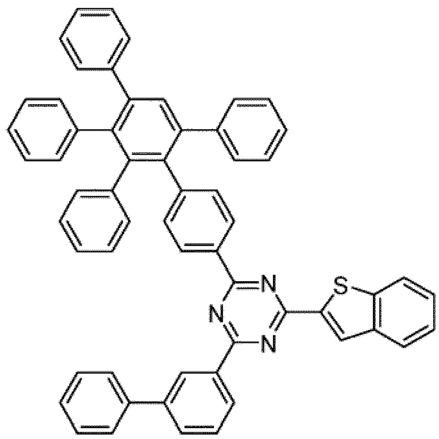
(G35),

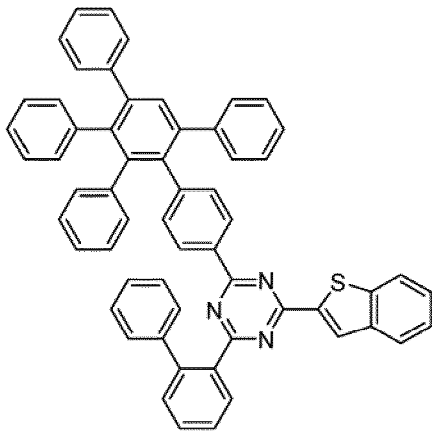
(G36),

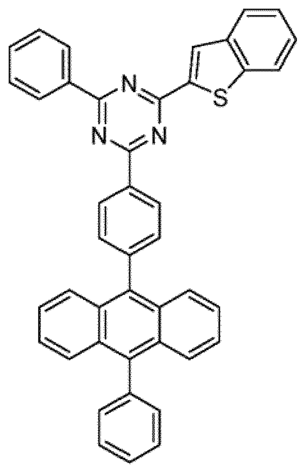
(G37),

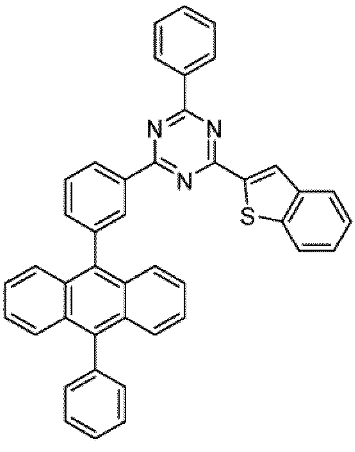 (G38), 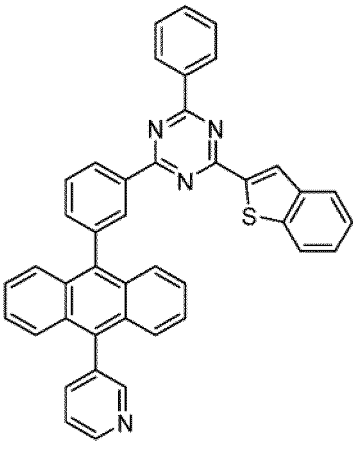 (G39),

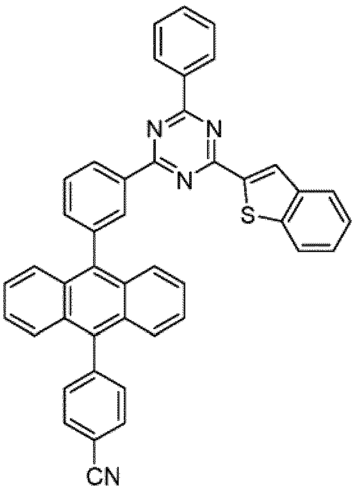 (G40), 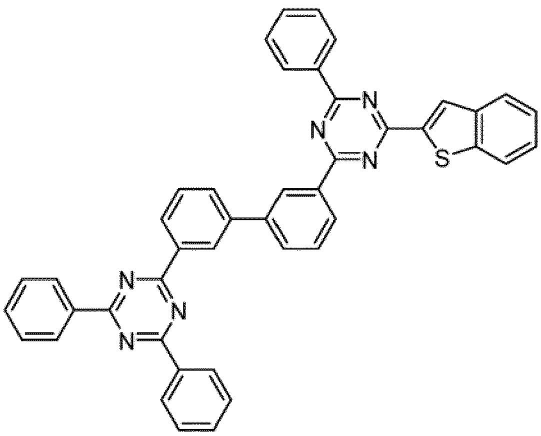 (G41),

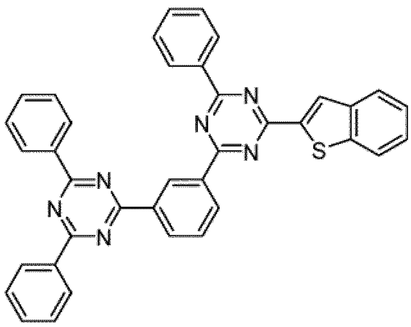 (G42), 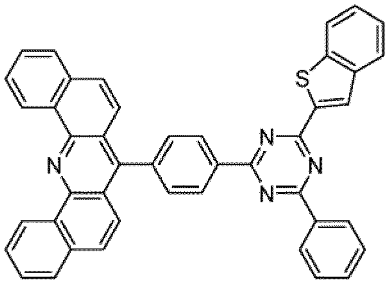 (G43),

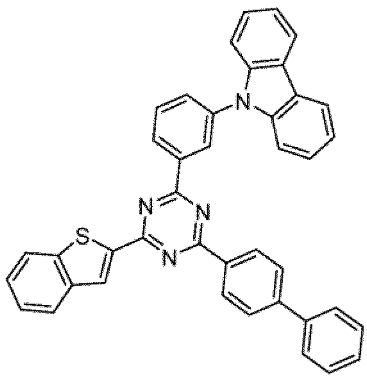 (G44), 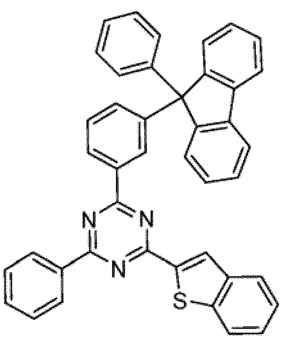 (G45),

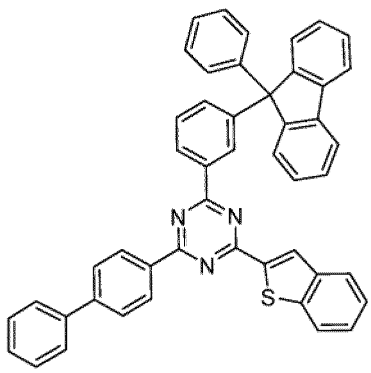

(G46),

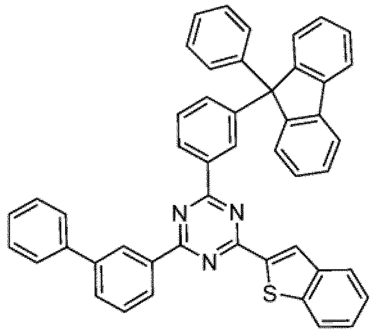

(G47),

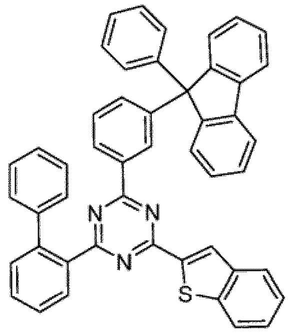

(G48),

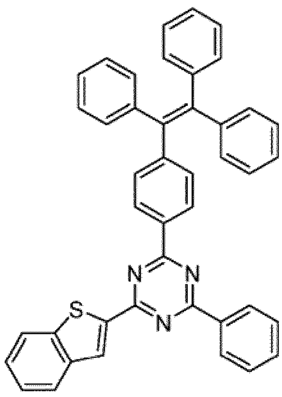

(G49),

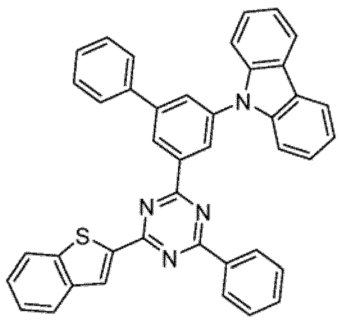

(G50),

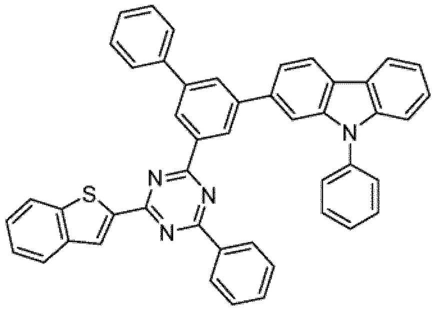

(G51),

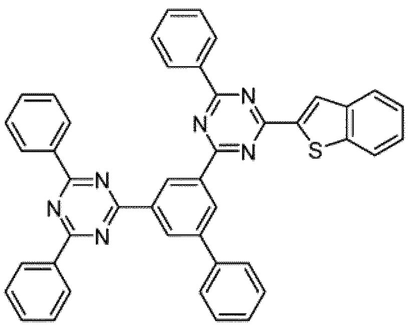

(G52),

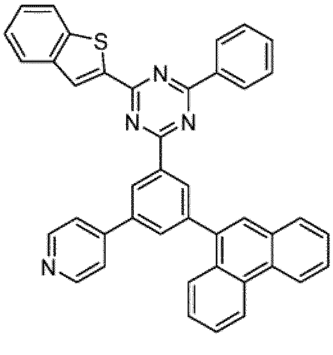

(G53),

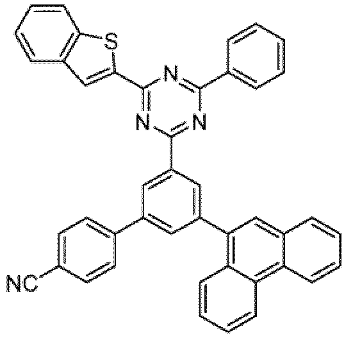

(G54),

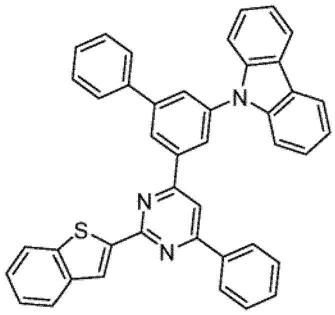
(G55),

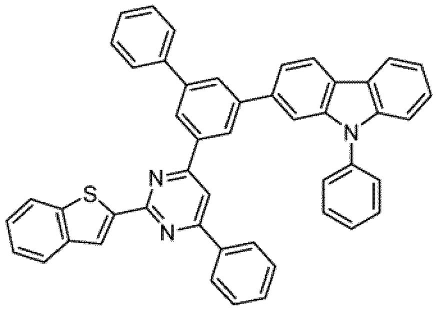
(G57),

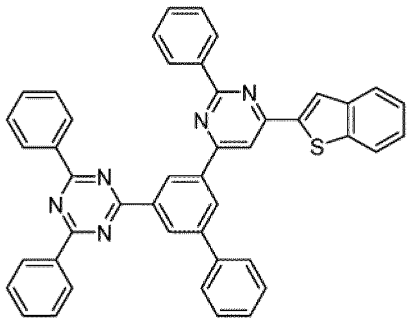
(G58),

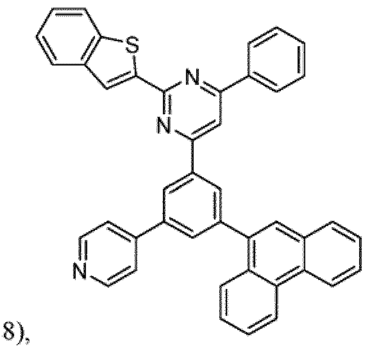
(G59),

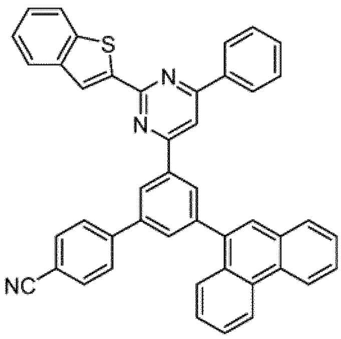
(G60),

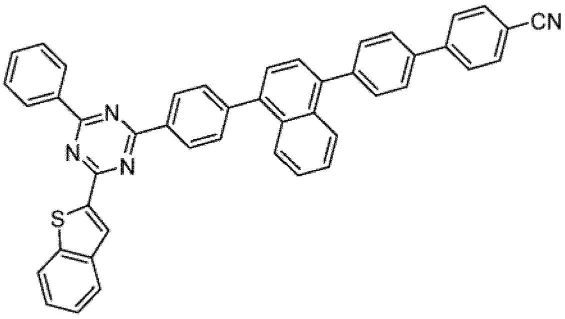
(G61),

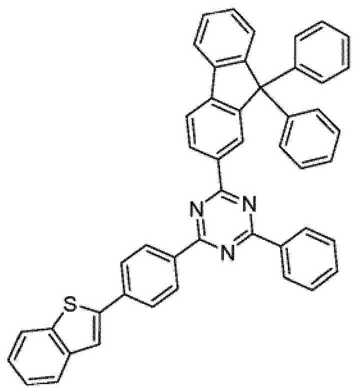
(G62),

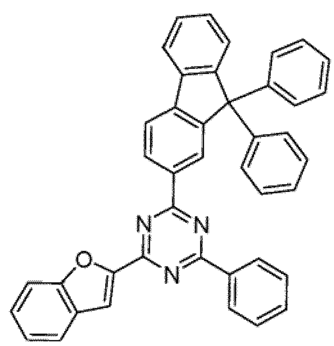
(G63),

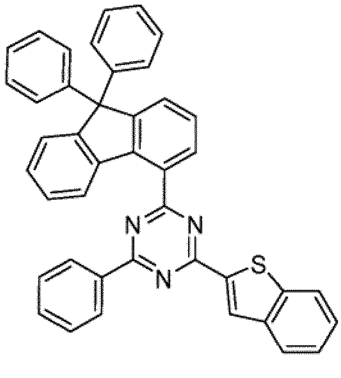 (G64), 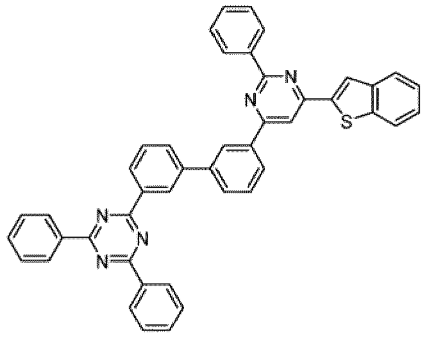 (G65).

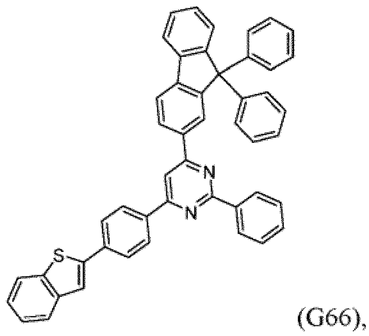 (G66), 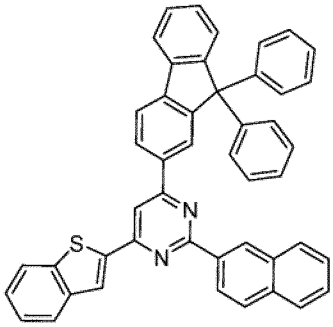 (G67),

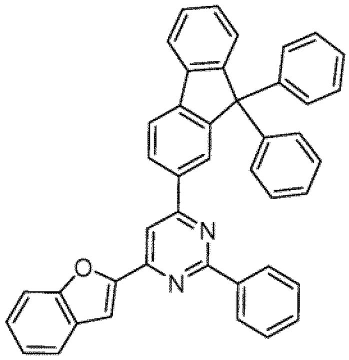 (G68), 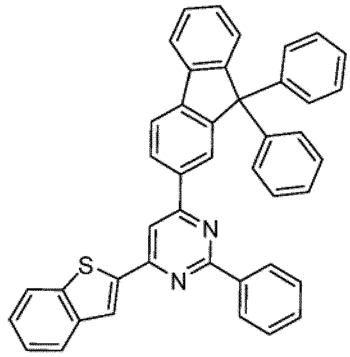 (G69),

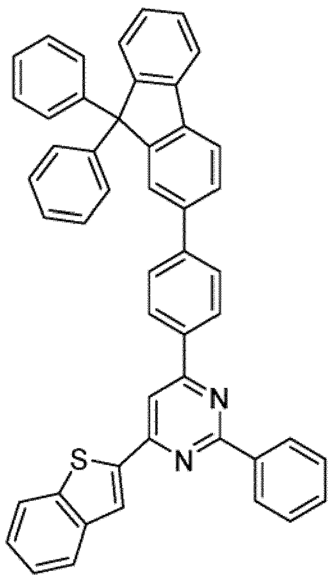 (G70).

**11.** Organische Halbleiterschicht, umfassend mindestens eine Verbindung der Formel 1 nach einem der vorhergehenden Ansprüche 1 bis 10.

**12.** Organische Halbleiterschicht nach Anspruch 11, wobei die organische Halbleiterschicht ferner ein Metall, ein Metallsalz oder einen organischen Metallkomplex, vorzugsweise einen organischen einwertigen oder zweiwertigen Metallkomplex, weiter bevorzugt LiQ oder Alkaliborat, umfasst.

**13.** Organische elektronische Vorrichtung, umfassend mindestens eine organische Halbleiterschicht nach einem der vorhergehenden Ansprüche 11 oder 12, wobei die mindestens eine organische Halbleiterschicht eine Verbindung der Formel 1 nach einem der vorhergehenden Ansprüche 1 bis 10 umfasst.

**14.** Organische elektronische Vorrichtung nach Anspruch 13, wobei es sich bei der elektronischen Vorrichtung um eine lichtemittierende Vorrichtung, einen Dünnschichttransistor, eine Batterie, eine Anzeigevorrichtung oder eine Photovoltaikzelle und vorzugsweise eine lichtemittierende Vorrichtung handelt.

## Revendications

**1.** Composé selon la formule 1 :

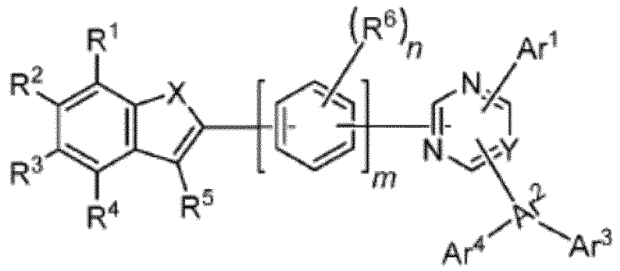
(1),

un groupe alkyle étant défini comme un groupe alkyle linéaire, cyclique ou ramifié ;
X étant O ou S ;
$R^1$, $R^2$, $R^3$, $R^4$, $R^5$, $R^6$ étant indépendamment choisis parmi H, un groupe $C_6$ à $C_{38}$ aryle substitué ou non substitué, Ce à $C_{36}$ hétéroaryle substitué ou non substitué ou $C_1$ à $C_{16}$ alkyle,
les substituants étant choisis parmi $C_6$ à $C_{18}$ aryle, $C_3$ à $C_{25}$ hétéroaryle, -PO(R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré ;
m étant un entier choisi parmi 0 ou 1 ;
n étant un entier choisi parmi 0 ;
Y étant CR" ou N, R" étant H, un groupe $C_6$ à $C_{38}$ aryle substitué ou non substitué, $C_3$ à $C_{36}$ hétéroaryle substitué ou non substitué ou $C_1$ à $C_{16}$ alkyle,
les substituants étant choisis parmi $C_6$ à $C_{18}$ aryle, $C_3$ à $C_{25}$ hétéroaryle, -PO (R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré ;
$Ar^1$ étant choisi parmi $C_6$ à $C_{38}$ aryle substitué ou non substitué ou $C_3$ à $C_{36}$ hétéroaryle substitué ou non substitué, les substituants étant choisis parmi $C_6$ à $C_{18}$ aryle, $C_3$ à $C_{25}$ hétéroaryle, -PO(R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré ;
$Ar^2$ étant choisi parmi $C_6$ à $C_{38}$ arylène substitué ou non substitué ou la formule 2,

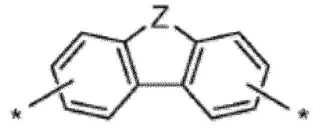

(2),

les substituants étant choisis parmi $C_6$ à $C_{18}$ aryle, $C_3$ à $C_{25}$ hétéroaryle, -PO (R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré ;

Z étant choisi parmi O, S, Se, CR''' ou NR''', R''' étant choisi parmi $C_1$ à $C_{16}$ alkyle ou $C_6$ à $C_{18}$ aryle ; $Ar^3$ étant choisi parmi H, aryle substitué ou non substitué, hétéroaryle substitué ou non substitué choisi dans le groupe composé de pyridine, pyrimidine, triazine, quinoléine, quinoxaline, benzoacridine, dibenzoacridine, phénanthroline, carbazole, dibenzofuranne, dibenzothiophène, ou

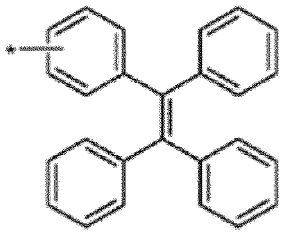

,

les substituants étant choisis parmi $C_6$ à $C_{18}$ aryle, $C_3$ à $C_{25}$ hétéroaryle, -PO (R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré ;

$Ar^4$ étant choisi parmi H, aryle substitué ou non substitué, hétéroaryle substitué ou non substitué choisi dans le groupe composé de pyridine, pyrimidine, triazine, quinoléine, quinoxaline, benzoacridine, dibenzoacridine, phénanthroline, carbazole, dibenzofuranne, dibenzothiophène,

les substituants étant choisis parmi $C_6$ à $C_{18}$ aryle, $C_3$ à $C_{25}$ hétéroaryle, -PO(R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré ;

et $Ar^4$ étant H lorsque $Ar^2$ est choisi parmi la formule 2.

**2.** Composé de formule 1 selon la revendication 1, X étant O ;

$R^1$, $R^2$, $R^3$, $R^4$, $R^5$, $R^6$ étant indépendamment choisis parmi H, un groupe $C_6$ à $C_{38}$ aryle substitué ou non substitué, $C_3$ à $C_{36}$ hétéroaryle substitué ou non substitué ou $C_1$ à $C_{16}$ alkyle,

les substituants étant choisis parmi $C_6$ à $C_{18}$ aryle, $C_3$ à $C_{25}$ hétéroaryle, -PO (R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré ;

m étant un entier choisi parmi 0 ou 1 ;

n étant un entier choisi parmi 0 ;

Y étant N ou CH ;

$Ar^1$ étant choisi parmi $C_6$ à $C_{38}$ aryle substitué ou non substitué ou $C_3$ à $C_{36}$ hétéroaryle substitué ou non substitué, les substituants étant choisis parmi $C_6$ à $C_{18}$ aryle, $C_3$ à $C_{25}$ hétéroaryle, -PO(R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré ;

$Ar^2$ étant choisi parmi $C_6$ à $C_{38}$ arylène substitué ou non substitué ou la formule 2,

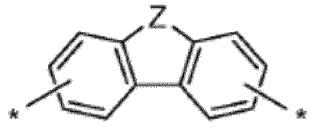

(2),

les substituants étant choisis parmi $C_6$ à $C_{18}$ aryle, $C_3$ à $C_{25}$ hétéroaryle, -PO (R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré ;

Z étant choisi parmi O, S, Se, CR''' ou NR''', R''' étant choisi parmi $C_1$ à $C_{16}$ alkyle ou $C_6$ à $C_{18}$ aryle ; $Ar^3$ étant choisi parmi H, aryle substitué ou non substitué, hétéroaryle substitué ou non substitué choisi dans le groupe composé de pyridine, pyrimidine, triazine, quinoléine, quinoxaline, benzoacridine, dibenzoacridine, phénanthroline, carbazole, dibenzofuranne, dibenzothiophène,

les substituants étant choisis parmi $C_6$ à $C_{18}$ aryle, $C_3$ à $C_{25}$ hétéroaryle, -PO(R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré ;

$Ar^4$ étant choisi parmi H, aryle substitué ou non substitué, hétéroaryle substitué ou non substitué choisi dans le groupe constitué par pyridine, pyrimidine, triazine, quinoléine, quinoxaline, benzoacridine, dibenzoacridine, phénanthroline, carbazole, dibenzofuranne, dibenzothiophène,

les substituants étant choisis parmi $C_6$ à $C_{18}$ aryle, $C_3$ à $C_{25}$ hétéroaryle, -PO(R')$_2$, R' étant alkyle, aryle ou hétéroaryle, D, F ou CN, $C_1$ à $C_{16}$ alkyle, $C_1$ à $C_{16}$ alkyle partiellement fluoré ou perfluoré, $C_1$ à $C_{16}$ alkyle partiellement deutéré ou perdeutéré, $C_1$ à $C_{16}$ alcoxy, $C_1$ à $C_{16}$ alcoxy partiellement fluoré ou perfluoré, $C_1$ à $C_{16}$ alcoxy partiellement deutéré ou perdeutéré.

**3.** Composé de formule 1 selon la revendication 1 ou 2, le composé de formule 1 étant représenté par la formule 1a et/ou 1b :

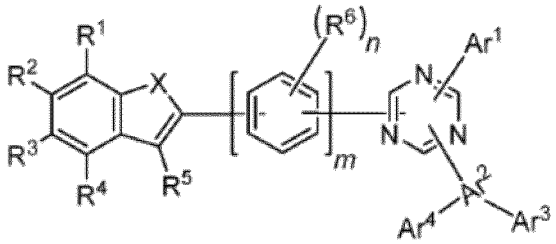

(1a);

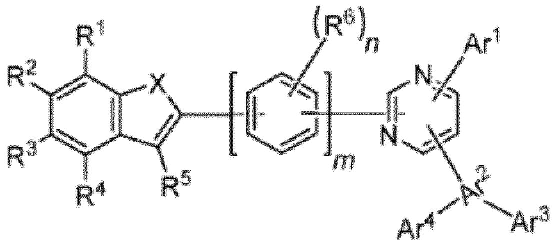

(1b).

**4.** Composé de formule 1 selon les revendications 1 à 3,

X étant O ou S ;
$R^1$, $R^2$, $R^3$, $R^4$, $R^5$, $R^6$ étant indépendamment choisis parmi H, phényle, naphtyle ou biphényle ;
m étant 0 ;
n étant 0 ;
Y étant N ou CH, préférablement N ;
$Ar^1$ étant choisi parmi un groupe phényle, naphtyle, biphényle, phénanthryle, triphényle ou anthryle ; $Ar^2$ étant

choisi parmi un groupe divalent phénylène, naphtylène, biphénylène, phénanthrylène, triphénylène ou anthrylène ;

Z étant choisi parmi O, S, Se, CR‴ ou NR‴, R‴ étant choisi parmi $C_1$ à $C_{16}$ alkyle ou $C_6$ à $C_{18}$ aryle ; $Ar^3$ étant choisi parmi H, aryle substitué ou non substitué, hétéroaryle substitué ou non substitué choisi dans un groupe constitué par pyridine, pyrimidine, triazine, quinoléine, quinoxaline, benzoacridine, dibenzoacridine, phénanthroline, carbazole, dibenzofuranne, dibenzothiophène ;

$Ar^4$ étant choisi parmi H, aryle substitué ou non substitué, hétéroaryle substitué ou non substitué choisi dans un groupe constitué par pyridine, quinoléine, quinoxaline, phénanthroline, carbazole, dibenzofuranne, dibenzothiophène.

**5.** Composé de formule 1 selon les revendications 1 à 4,

$Ar^2$ étant choisi parmi la formule 2 ; et
Z étant choisi parmi O, S, Se, CR‴ ou NR‴, R‴ étant choisi parmi $C_1$ à $C_{16}$ alkyle ou $C_6$ à $C_{18}$ aryle, et de manière plus préférée CR‴, R‴ étant choisi parmi $C_1$ à $C_{16}$ alkyle ou $C_6$ à $C_{18}$ aryle.

**6.** Composé de formule 1 selon l'une quelconque des revendications 1 à 4, le composé de formule 1 comprenant au moins un cycle non-hétéro phényle à douze cycles non-hétéro phényle, préférablement au moins deux cycles phényle à sept cycles phényle.

**7.** Composé de formule 1 selon l'une quelconque des revendications 1 à 6, le composé de formule 1 comprenant au moins un à quatre, préférablement un à trois, en outre de manière préférée un ou deux et de manière plus préférée un cycle hétéroaromatique à 6 chaînons, et au moins un à deux, préférablement un cycle hétéroaromatique à 5 chaînons.

**8.** Composé de formule 1 selon l'une quelconque des revendications 1 à 7, $Ar^1$ et $Ar^3$ et/ou $Ar^4$ étant indépendamment choisis parmi $C_6$ à $C_{18}$ aryle substitué ou non substitué, ou $C_5$ à $C_{18}$ hétéroaryle substitué ou non substitué, et $Ar^2$ étant indépendamment choisi parmi $C_6$ à $C_{18}$ arylène substitué ou non substitué, ou $C_5$ à $C_{18}$ hétéroarylène substitué ou non substitué,
les substituants du $C_6$ à $C_{18}$ aryle substitué, $C_5$ à $C_{18}$ hétéroaryle substitué, $C_6$ à $C_{18}$ arylène substitué ou $C_5$ à $C_{18}$ hétéroarylène substitué étant indépendamment choisis parmi $C_1$ à $C_{12}$ alkyle, $C_1$ à $C_{12}$ alcoxy, $C_1$ à $C_{12}$ alkyle partiellement fluoré ou perfluoré, $C_1$ à $C_{16}$ alcoxy partiellement fluoré ou perfluoré, $C_1$ à $C_{12}$ alkyle partiellement deutéré ou perdeutéré, $C_1$ à $C_{16}$ alcoxy partiellement deutéré ou perdeutéré, $C_6$ à $C_{18}$ aryle, $C_3$ à $C_{25}$ hétéroaryle, D, F ou CN, préférablement $C_1$ à $C_{12}$ alkyle.

**9.** Composé de formule 1 selon l'une quelconque des revendications 1 à 8,

- $Ar^1$ étant choisi parmi phényle, biphényle, terphényle, naphtyle, phénanthryle, triphényle, anthracényle, et préférablement parmi C1 à C9 :

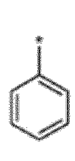 (C1), 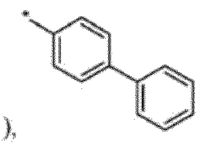 (C2), 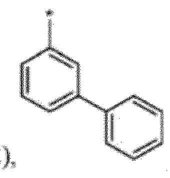 (C3), 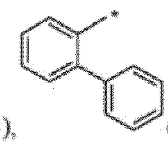 (C4),

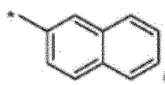 (C5), 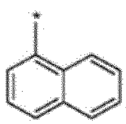 (C6), 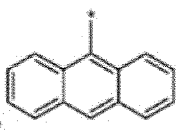 (C7), 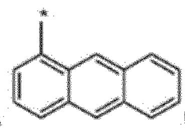 (C8),

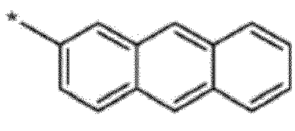 (C9);

et/ou

- $Ar^2$ étant choisi parmi phénylène, biphénylène, terphénylène, naphtylène, phénanthrylène, triphénylène, anthracénylène, et préférablement parmi D1 à D13 :

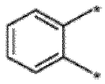 (D1), 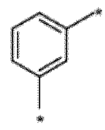 (D2), 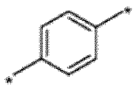 (D3), 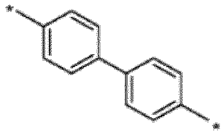 (D4),

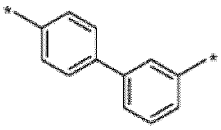 (D5), 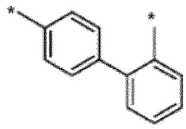 (D6), 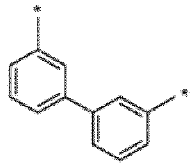 (D7),

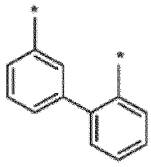 (D8), 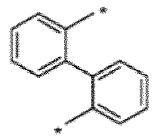 (D9), 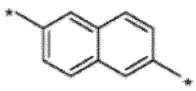 (D10), 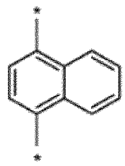 (D11),

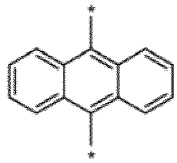 (D12), 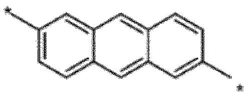 (D13);

- $Ar^3$ étant indépendamment choisi parmi E1 à E28 :

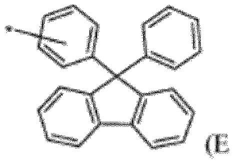 (E1), 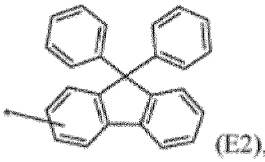 (E2), 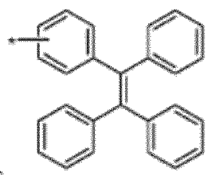 (E3),

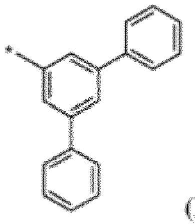 (E4), 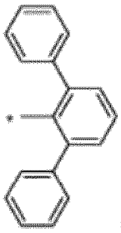 (E5), 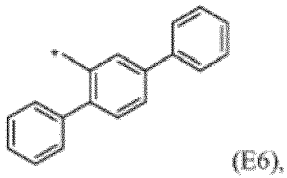 (E6),

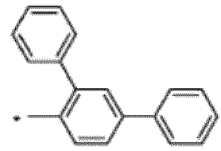 (E7), 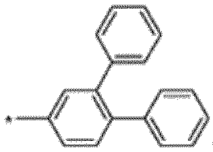 (E8), 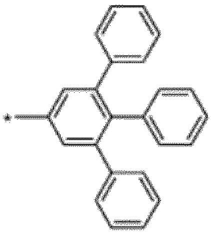 (E9),

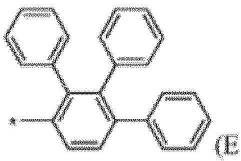 (E10), 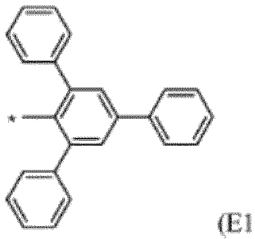 (E11),

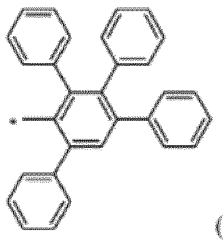 (E12), 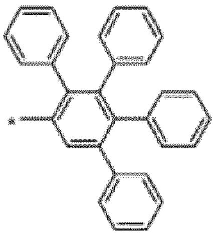 (E13), 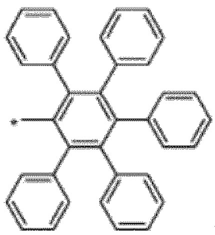 (E14),

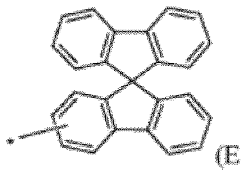 (E15), 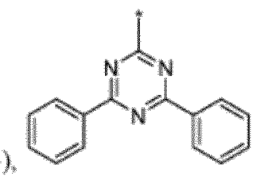 (E16), 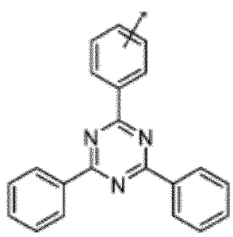 (E17),

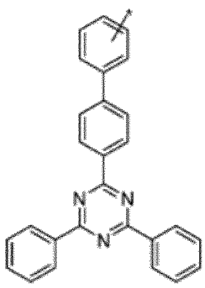 (E18), 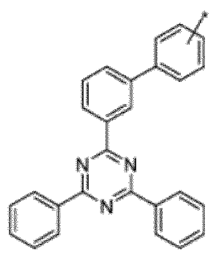 (E19), 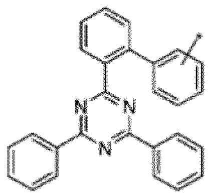 (E20),

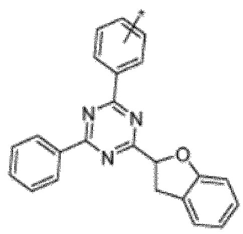 (E21),

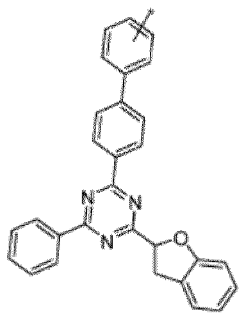 (E22), 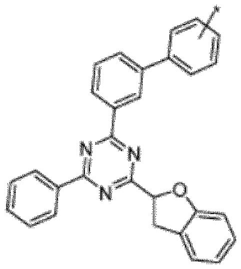 (E23),

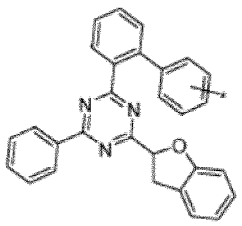 (E24), 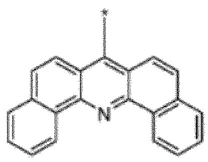 (E25); 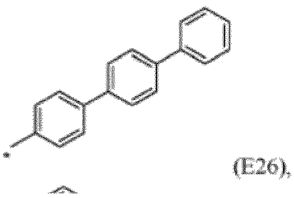 (E26),

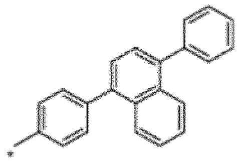 (E27), 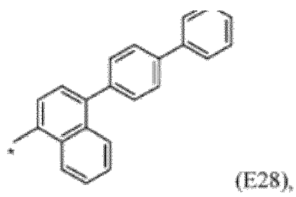 (E28),

- $Ar^4$ étant indépendamment choisi parmi F1 à F9 :

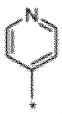 (F1), 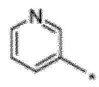 (F2), 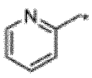 (F3), 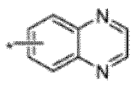 (F4), 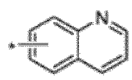 (F5), 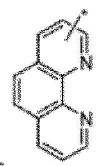 (F6),

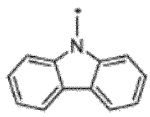 (F7), 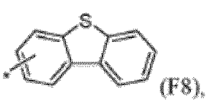 (F8), 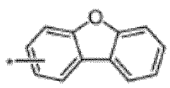 (F9).

**10.** Composé de formule 1 selon l'une quelconque des revendications 1 à 9, le composé de formule 1 étant choisi parmi G1 à G70 :

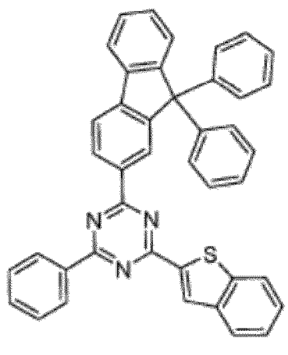 (G1), 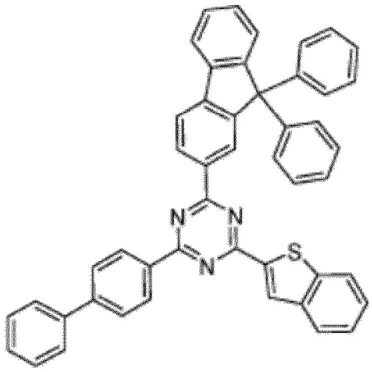 (G2),

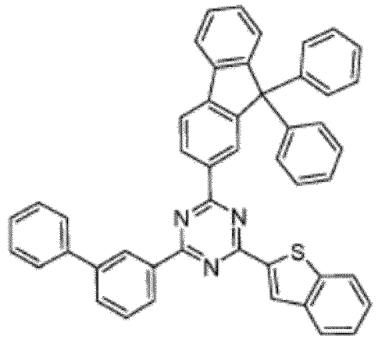 (G3), 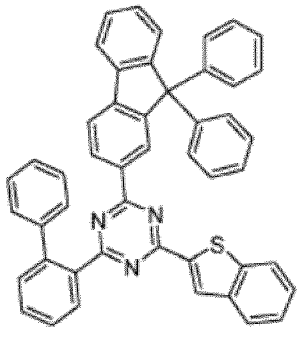 (G4),

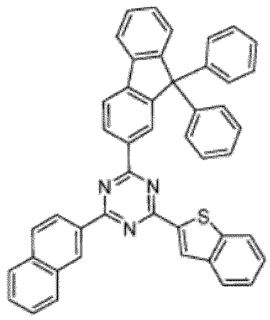 (G5), 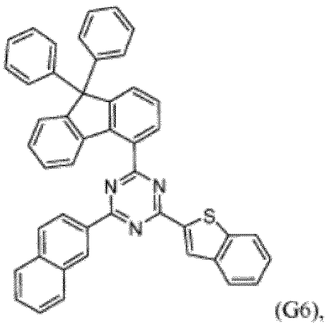 (G6), 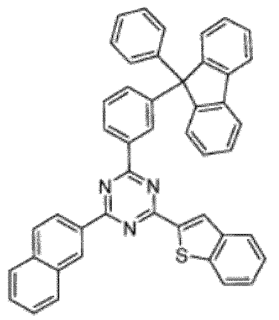

(G7), 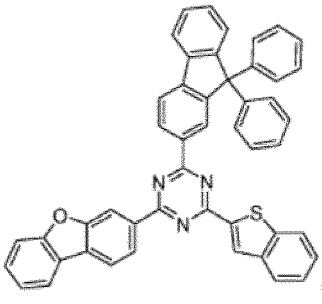 (G8), 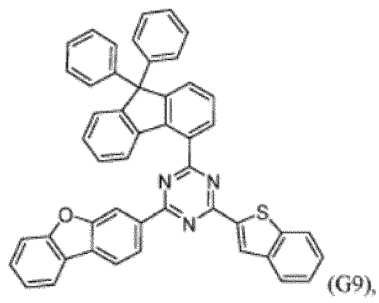 (G9),

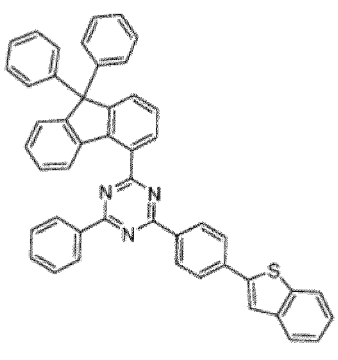 (G10), 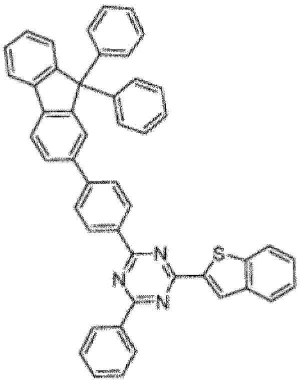 (G11),

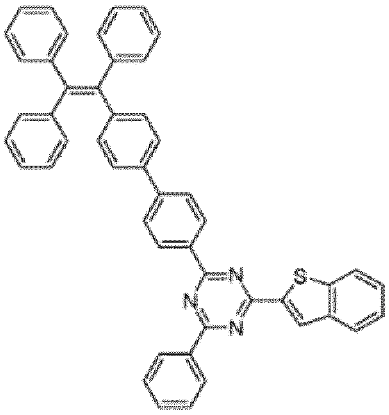 (G12), 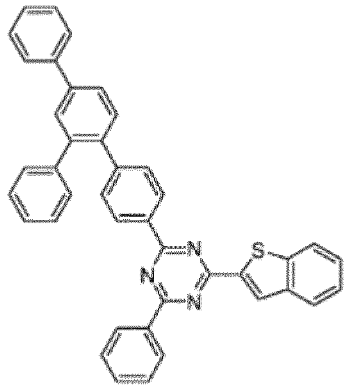 (G13),

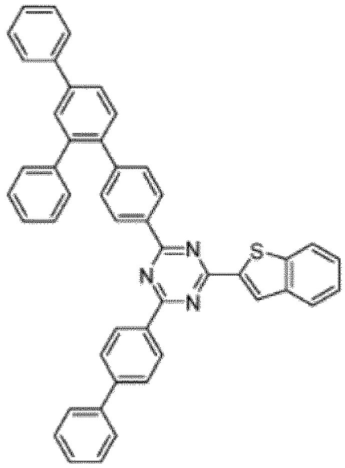 (G14), 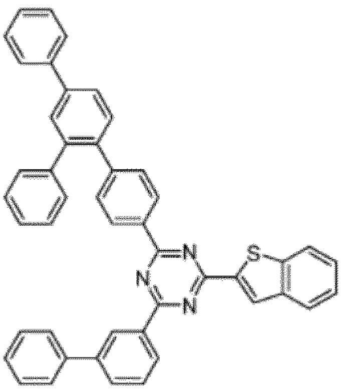 (G15),

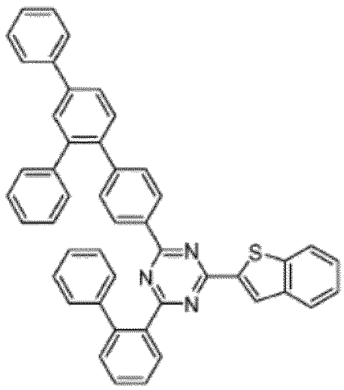

(G16),

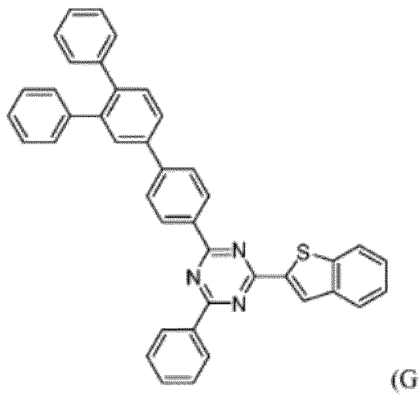

(G17),

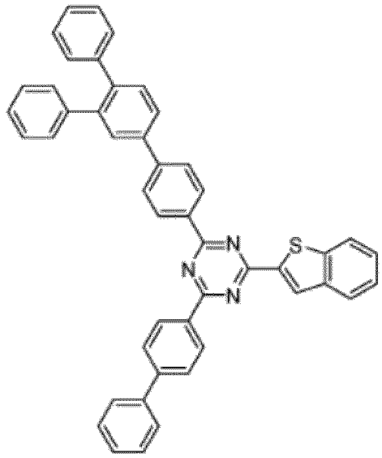

(G18),

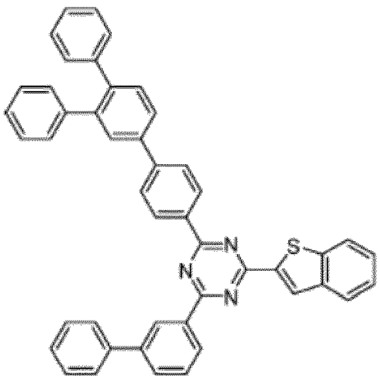

(G19),

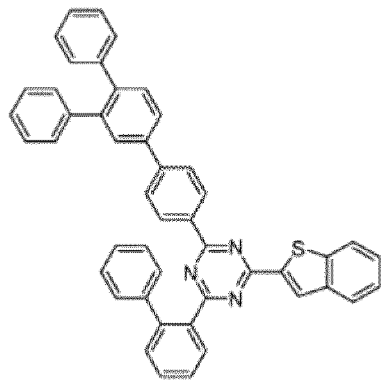

(G20),

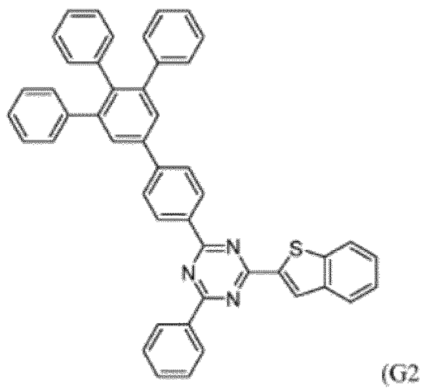

(G21),

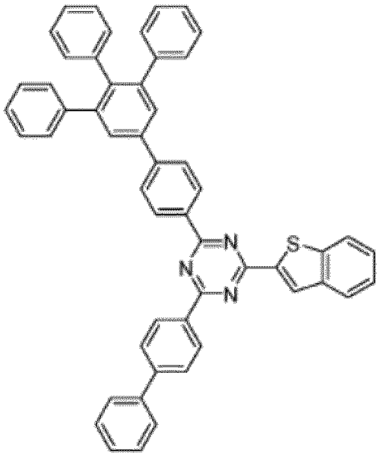
(G22),

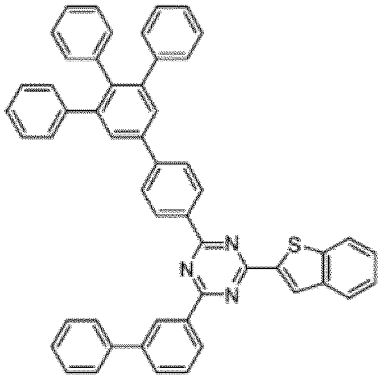
(G23),

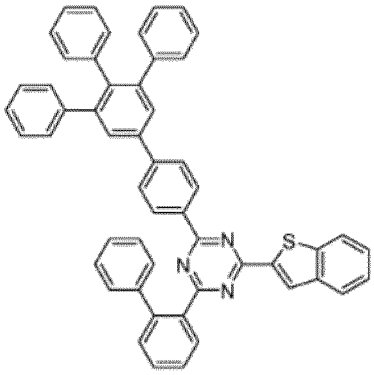
(G24),

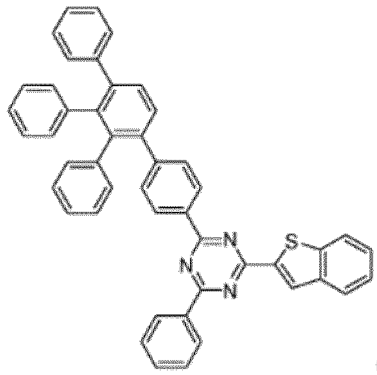
(G25),

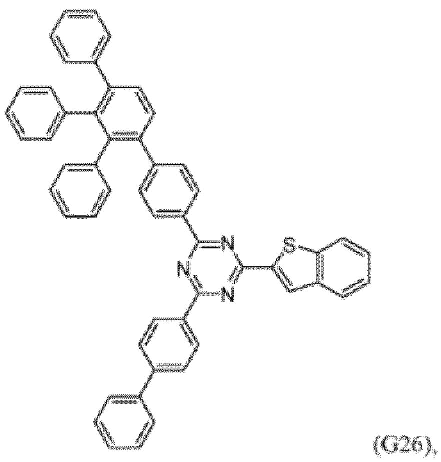
(G26),

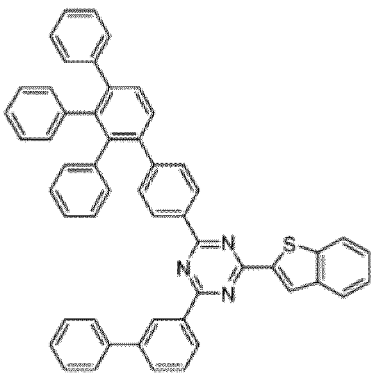
(G27),

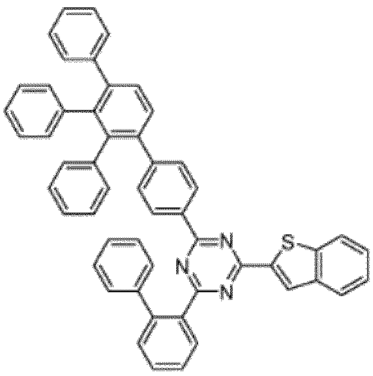

(G28),

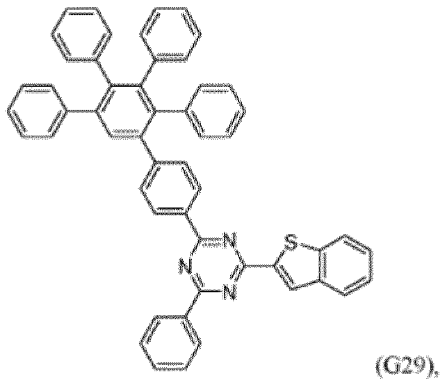

(G29),

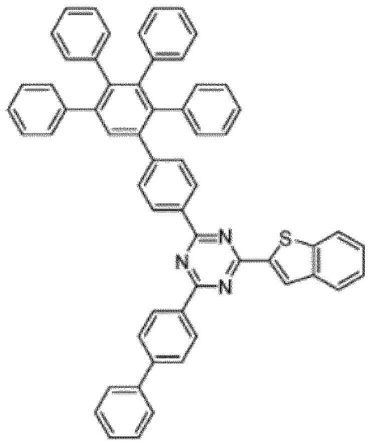

(G30),

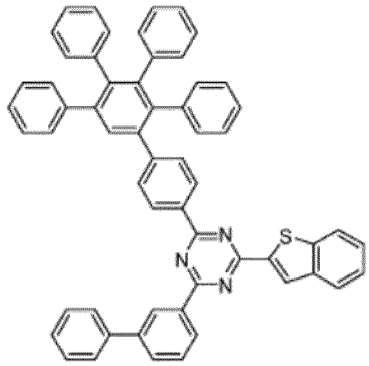

(G31),

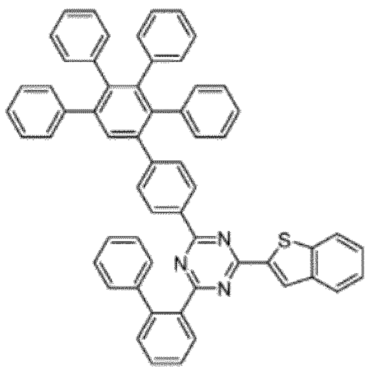

(G32),

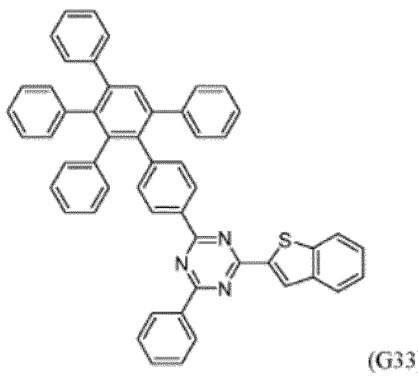

(G33),

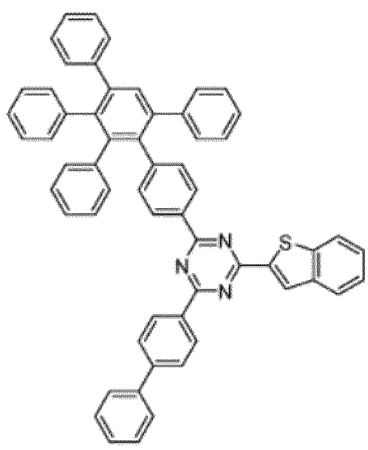

(G34),

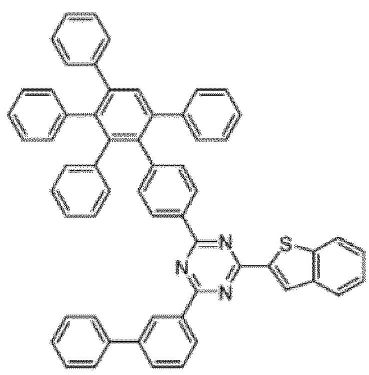

(G35),

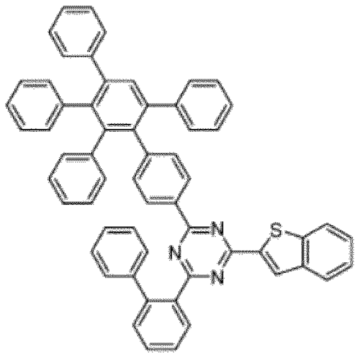
(G36),

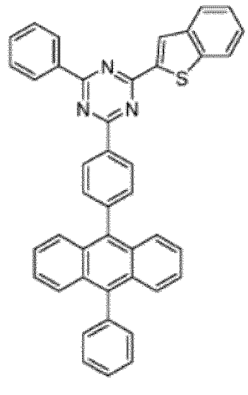
(G37),

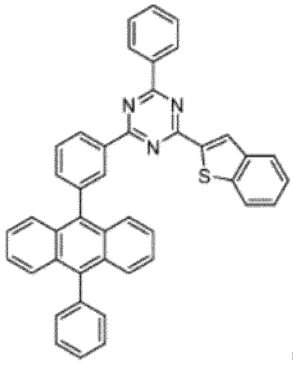
(G38),

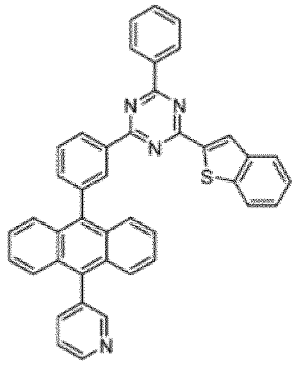
(G39),

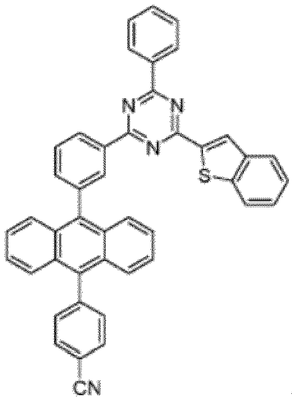
(G40),

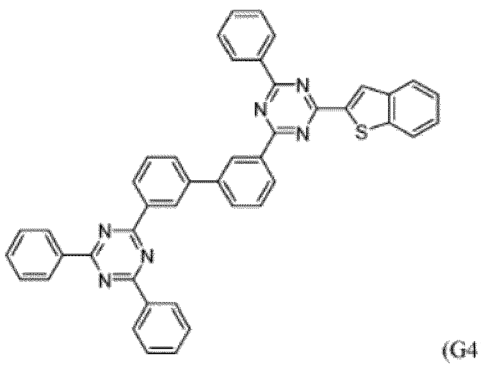
(G41),

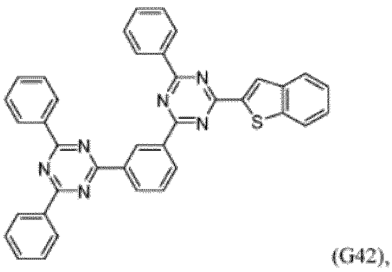
(G42),

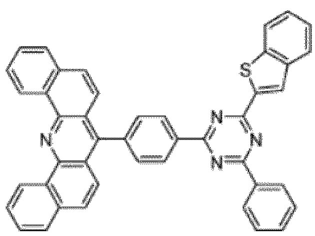
(G43),

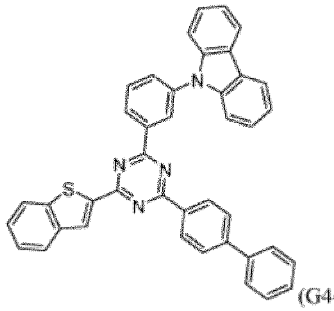 (G44), 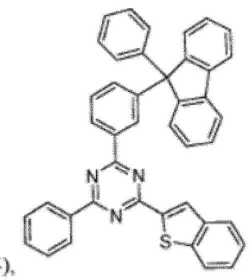 (G45),

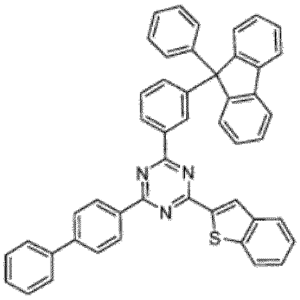 (G46), 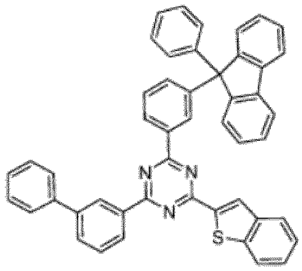 (G47),

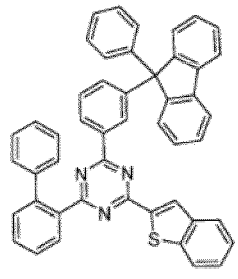 (G48), 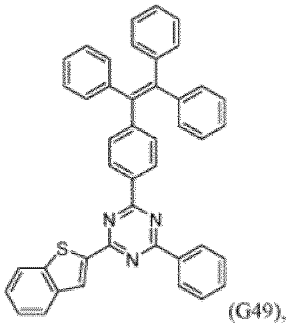 (G49), 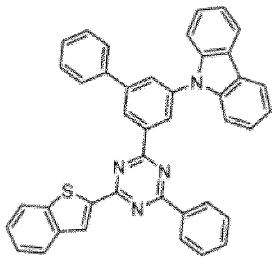

(G50), 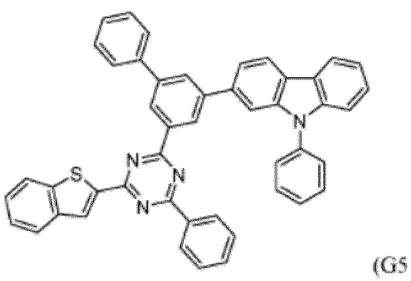 (G51), 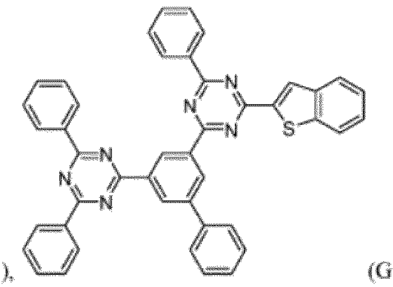 (G52),

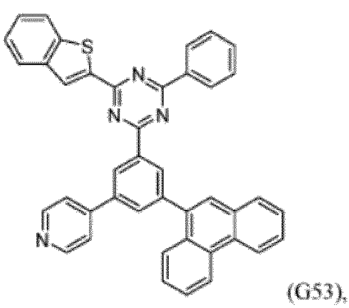 (G53), 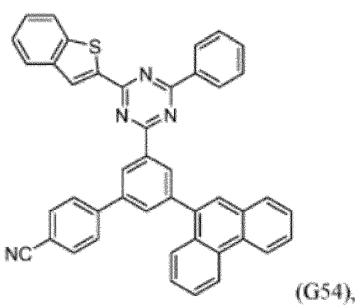 (G54),

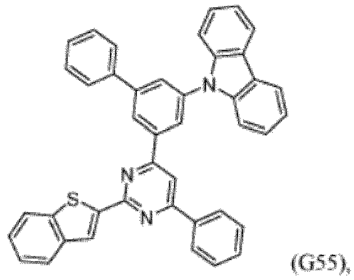
(G55),
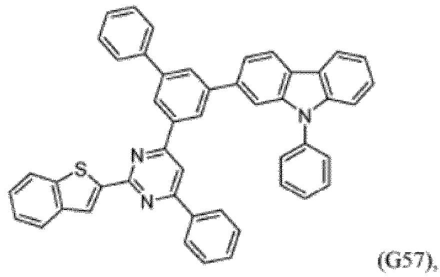
(G57),

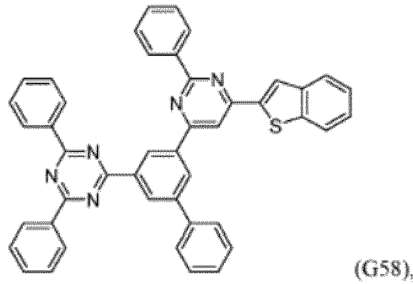
(G58),
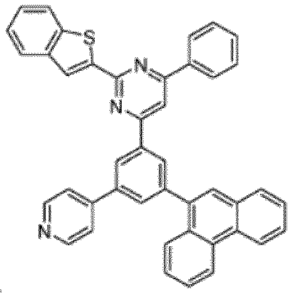
(G59),

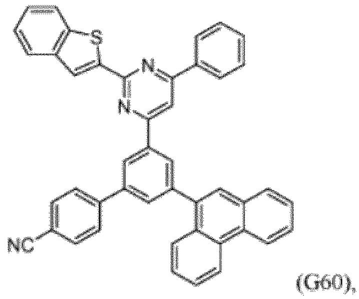
(G60),
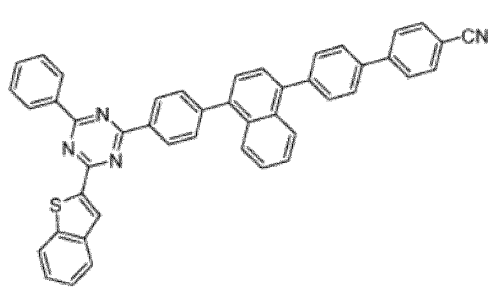
(G61),

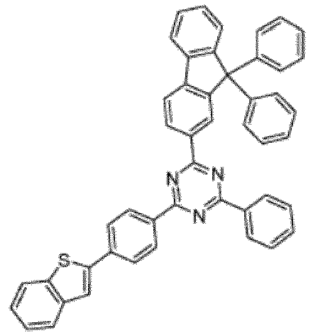
(G62),
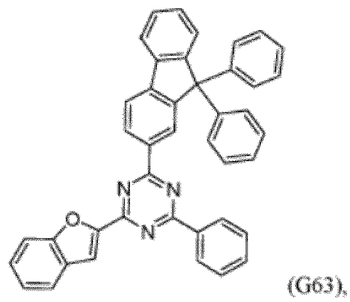
(G63),

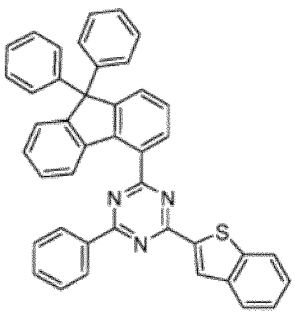
(G64),
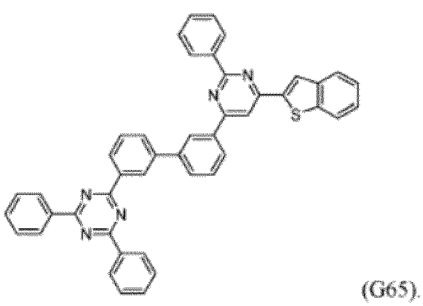
(G65).

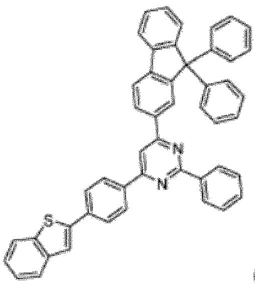 (G66), 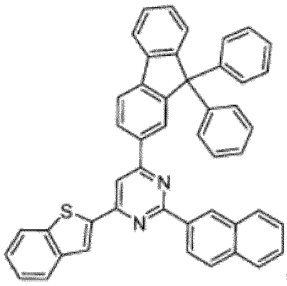 (G67),

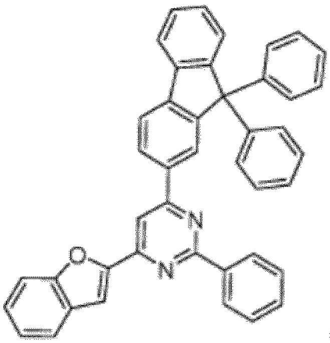 (G68), 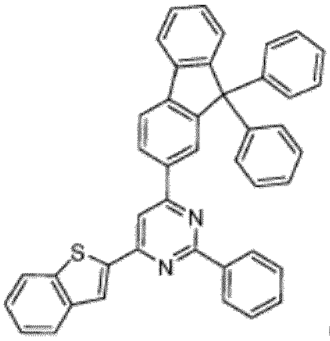 (G69),

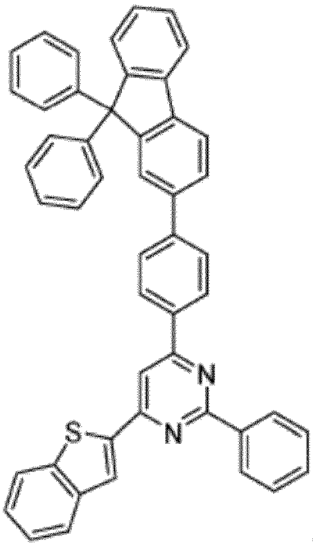 (G70).

**11.** Couche de semi-conducteur organique comprenant au moins un composé de formule 1 selon l'une quelconque des revendications précédentes 1 à 10.

**12.** Couche de semi-conducteur organique selon la revendication 11, la couche de semi-conducteur organique comprenant en outre un métal, un sel métallique ou un complexe de métal organique, préférablement un complexe de métal monovalent ou divalent organique, plus préférablement LiQ ou un borate alcalin.

**13.** Dispositif électronique organique comprenant au moins une couche de semi-conducteur organique selon l'une quelconque des revendications précédentes 11 et 12, l'au moins une couche de semi-conducteur organique comprenant un composé de formule 1 selon l'une quelconque des revendications précédentes 1 à 10.

**14.** Dispositif électronique organique selon la revendication 13, le dispositif électronique étant un dispositif émetteur de lumière, un transistor en film mince, une batterie, un dispositif d'affichage ou une cellule photovoltaïque, et préférablement un dispositif émetteur de lumière.

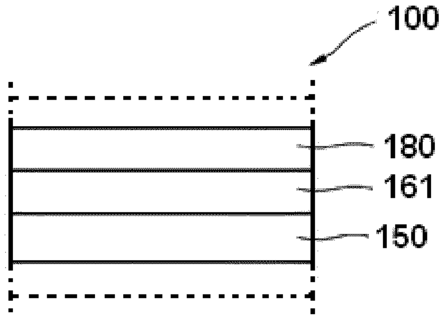


FIG. 1

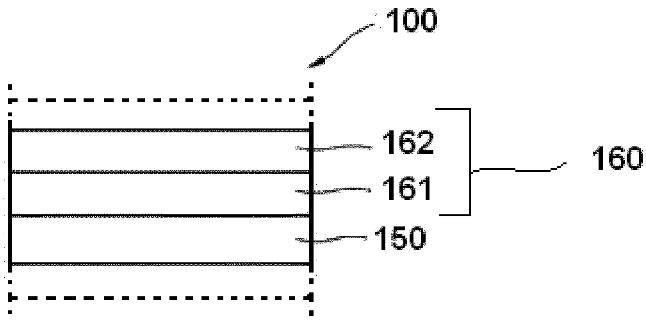


FIG. 2

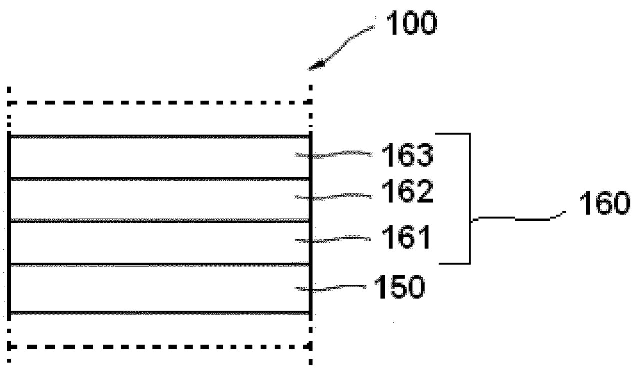


FIG. 3

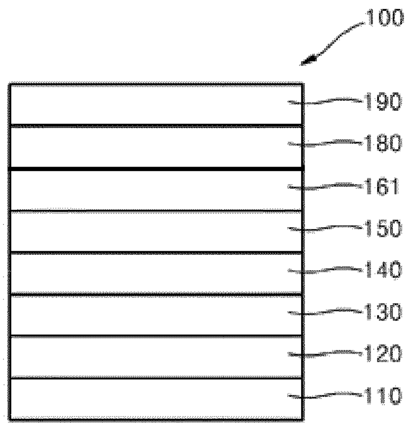


FIG. 4

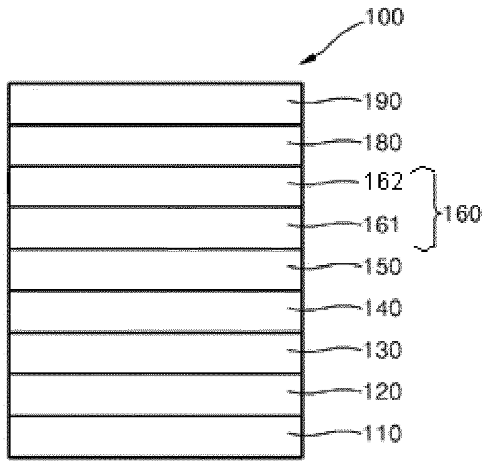


FIG. 5

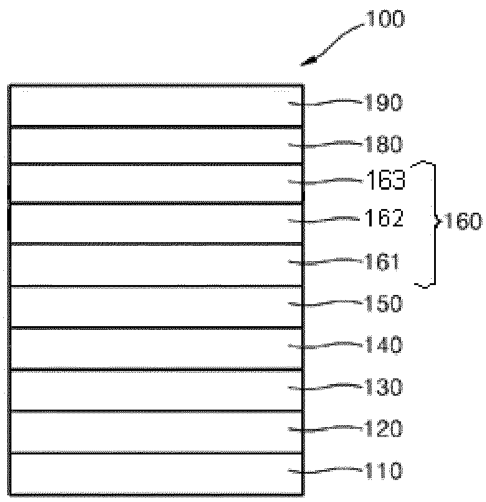


FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014208755 A1
- EP 1962354 A1
- WO 2017135510 A1
- KR 20170116944 A
- US 6140763 A
- US 6614176 B
- US 2016248022 A
- WO 2013079217 A1
- WO 2013079676 A1
- WO 2013079678 A1
- JP 2008195623 B
- JP 2001291593 B
- US 20030165711 A
- WO 2016171358 A
- WO 2017135510 A


### Non-patent literature cited in the description


- *CHEMICAL ABSTRACTS,* 1242056-42-3
- *CHEMICAL ABSTRACTS,* 1198399-61-9